# EUROPEAN PATENT SPECIFICATION

(11) **EP 3 606 510 B1**
(45) Date of publication and mention of the grant of the patent: **10.12.2025**
(21) Application number: 17718301.9
(22) Date of filing: 06.04.2017
(51) Int. Cl.: A61K 9/28, A61K 31/44

(54) **SUSTAINED RELEASE COMPOSITIONS OF 4-AMINOPYRIDINE**
ZUSAMMENSETZUNGEN MIT VERLÄNGERTER FREISETZUNG VON 4-AMINOPYRIDINE
COMPOSITIONS À LIBÉRATION PROLONGÉE DE 4-AMINOPYRIDINE

(43) Date of publication of application: 12.02.2020
(73) Proprietor: Merz Pharmaceuticals, LLC, Raleigh, NC 27615 (US)
(72) Inventor: COBB, Joseph, E., Jr., Greenville, NC 27834 (US); GOLD, Thomas, B., Greenville, NC 27834 (US); D'SOUZA, Rohini, Pomona, NY 10970 (US); WAY, Susan, L., Danbury, CT 06811 (US)
(74) Representative: Carpmaels & Ransford LLP
(86) International application number: PCT/US2017/026360
(87) International publication number: WO 2018/186866

(56) References cited:
- WO-A1-2004/082684
- RU-C1- 2 536 269
- US-A1- 2005 276 851
- US-A1- 2017 071 923

## Description

### CROSS-REFERENCE TO RELATED APPLICATIONS

This application is a continuation-in-part of International Patent Application No. PCT/US2016/054109, filed September 28, 2016, which claims the benefit of U.S. Provisional Application No. 62/234,455, filed on September 29, 2015.

### 1 FIELD OF THE INVENTION

The present invention generally relates to sustained release 4-aminopyridine tablets, which include a core and a coating. The sustained release tablets of the invention are generally suitable for once daily oral administration for the treatment of neurological disorders.

### 2 BACKGROUND OF THE INVENTION

4-Aminopyridine is a potassium (K+) channel blocker approved by the U.S. Food and Drug Administration as a treatment for patients with multiple sclerosis to improve walking. Dalfampridine is the United States Adopted Name (USAN) for the chemical 4-aminopyridine, which has a molecular formula of C₅H₆N₂ and molecular weight of 94.1; the former USAN name for this compound was fampridine (which remains the International Nonproprietary Name). The terms "dalfampridine", "fampridine" and "4-aminopyridine" will be used throughout this specification to refer to the active drug substance.

Studies of 4-aminopyridine (dalfampridine, fampridine) have been conducted using intravenous (i.v.) administration and immediate-release (IR) oral capsule formulations in addition to controlled-release or sustained-release formulations. Administration of IR capsules resulted in rapid and short-lasting peaks of 4-aminopyridine in the plasma. Early pharmacokinetic studies were conducted using an immediate release (IR) formulation for oral administration, which consisted of 4-aminopyridine powder in a gelatin-based capsule or oral solution. Administration resulted in rapidly changing 4-aminopyridine plasma levels that were not well tolerated. A sustained-release matrix tablet (known as Fampridine-SR or AMPYRA^{®}, Acorda Therapeutics, Ardsley, NY) was then developed. The sustained release matrix tablet showed improved stability and an appropriate pharmacokinetic profile for twice-daily dosing. Sustained release compositions of 4-aminopyridine and related use of such compositions are set forth, e.g., in US Patent 5,370,879; US Patent 5,540,938; US Patent 8,007,826; US Patent 8,354,437; US Patent 8,663,655; International Publication WO 2012/10347, and International Publication WO 2014/093475. For example, suitable formulations, methods of manufacture, pharmacokinetic characteristics of sustained release aminopyridine compositions and methods of treating various neurological disorders are further described in U.S. Patent No. 8,007,826 entitled "Sustained Release Aminopyridine Composition" issued on August 30, 2011; and U.S. Patent No. 8,354,437 entitled "Methods of Using Sustained Release Aminopyridine Compositions" issued on January 15, 2013.

Provided herein are once daily sustained release 4-aminopyridine tablets that deliver a therapeutically effective amount of the active agent over 24 hours for the treatment of neurological disorders.
US 2005/276851 A1 describes a sustained release tablet of 4-aminopyridine wherein the tablet is a non-coated tablet with the active agent dispersed in an HPMC-matrix.

### 3 SUMMARY OF THE INVENTION

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core, said amount of the ethylcellulose coat being in the range of about 5% w/w to about 10% w/w of the compressed core;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core, said amount of the ethylcellulose coat being in the range of about 5% w/w to about 10% w/w of the compressed core;
wherein, the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core, said amount of the ethylcellulose coat being in the range of about 5% w/w to about 7% w/w of the compressed core;
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a sustained release tablet comprising:
(a) a compressed core, wherein said compressed core comprises: (i) 4-aminopyridine, wherein the amount of 4-aminopyridine is in the range of about 4% w/w to about 6% w/w of the compressed core; (ii) a polyethylene oxide with a molecular weight between about 1,000,000 and 10,000,000, wherein the amount of the polyethylene oxide is in the range of about 10% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core, and
(b) an amount of an ethylcellulose coat surrounding said compressed core, said amount of the ethylcellulose coat being in the range of about 5% w/w to about 10% w/w of the compressed core;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a sustained release tablet comprising:
(a) a compressed core, wherein said compressed core comprises: (i) 4-aminopyridine, wherein the amount of 4-aminopyridine is in the range of about 4% w/w to about 6% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 9% w/w of the compressed core;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a sustained release tablet comprising:
(a) a compressed core, wherein said compressed core comprises: (i) 4-aminopyridine, wherein the amount of 4-aminopyridine is in the range of about 3% w/w to about 5% w/w of the compressed core; (ii) a polyethylene oxide with a molecular weight between about 1,000,000 and 10,000,000, wherein the amount of the polyethylene oxide is in the range of about 10% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core, said amount of the ethylcellulose coat being in the range of about 5% w/w to about 7% w/w of the compressed core;
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a sustained release tablet comprising:
(a) a compressed core, wherein said compressed core comprises: (i) 4-aminopyridine, wherein the amount of 4-aminopyridine is in the range of about 3% w/w to about 5% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 6% w/w of the compressed core;
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core;
wherein the ratio of the amount of the ethylcellulose coat to the amount of 4-aminopyridine in the compressed core is in the range of about 0.5:1 to about 3:1, wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core; the amount of 4-aminopyridine in the sustained release tablet is about 22 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core;
wherein the ratio of the amount of the ethylcellulose coat to the amount of 4-aminopyridine in the compressed core is in the range of about 0.5:1 to about 3:1, wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core; the amount of 4-aminopyridine in the sustained release tablet is about 16.5 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core;
wherein the ratio of the amount of the ethylcellulose coat to the amount of 4-aminopyridine in the compressed core is in the range of about 0.1:1 to about 0.7:1, wherein for calculating said ratio, the amount of the ethylcellulose coat is the weight percentage of the ethylcellulose coat by weight of the compressed core, and the amount of 4-aminopyridine is the weight in milligrams of 4-aminopyridine; the amount of 4-aminopyridine in the sustained release tablet is about 22 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core;
wherein the ratio of the amount of the ethylcellulose coat to the amount of 4-aminopyridine in the compressed core is in the range of about 0.1:1 to about 0.7:1, wherein for calculating said ratio, the amount of the ethylcellulose coat is the weight percentage of the ethylcellulose coat by weight of the compressed core, and the amount of 4-aminopyridine is the weight in milligrams of 4-aminopyridine; the amount of 4-aminopyridine in the sustained release tablet is about 16.5 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a method of making a sustained release tablet comprising 4-aminopyridine, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 10% w/w of the compressed core; and
(c) curing the coated compressed core by exposing it to a temperature in the range of 40-70 °C for a period of time of at least 1 hour;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a method of making a sustained release tablet comprising 4-aminopyridine, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 10% w/w of the compressed core; and
(c) curing the coated compressed core by exposing it to a temperature in the range of 40-70 °C for a period of time of at least 1 hour;
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

Provided herein is a method of making a sustained release tablet comprising 4-aminopyridine, which method comprises:
(a) forming a compressed core comprising (i) 4-aminopyridine, wherein the amount of 4-aminopyridine is in the range of about 4% w/w to about 6% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 9% w/w of the compressed core; and
(c) curing the coated compressed core by exposing it to a temperature in the range of 50-60 °C for a period of time of at least 1 hour;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

Provided herein is a method of making a sustained release tablet comprising 4-aminopyridine, which method comprises:
(a) forming a compressed core comprising (i) 4-aminopyridine, wherein the amount of 4-aminopyridine is in the range of about 3% w/w to about 5% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 6% w/w of the compressed core; and
(c) curing the coated compressed core by exposing it to a temperature in the range of 50-60 °C for a period of time of at least 1 hour;
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

In one embodiment, the sustained release tablet provided herein is the product of a process comprising a curing step after coating the compressed core with ethylcellulose, and wherein said curing step comprises heating the coated compressed core to a temperature above 23 °C for a period of time of at least 15 minutes.

In one embodiment of the sustained release tablet provided herein, the curing step comprises exposing the coated compressed core to a temperature in the range of 40-70 °C for a period of time of at least 1 hour.

In one embodiment of the sustained release tablet provided herein, the curing step comprises exposing the coated compressed core to a temperature in the range of 50-60 °C for a period of time of at least 1 hour.

In one embodiment of the sustained release tablet provided herein, the amount of the ethylcellulose coat is in the range of about 8% w/w to about 10% w/w of the tablet,
wherein said tablet is the product of a process comprising a curing step after coating the compressed core with ethylcellulose, and wherein said curing step comprises heating the coated compressed core to a temperature above 23 °C for a period of time of at least 15 minutes.

In one embodiment of the sustained release tablet provided herein, the mixture comprising polyvinyl acetate and polyvinyl pyrrolidone further comprises one or more pharmaceutically acceptable excipients.

In one embodiment of the sustained release tablet provided herein, the mixture comprising polyvinyl acetate and polyvinyl pyrrolidone comprises 70-90% polyvinyl acetate and 15-20% polyvinyl pyrrolidone.

In one embodiment of the sustained release tablet provided herein, the mixture comprising polyvinyl acetate and polyvinyl pyrrolidone further comprises a surfactant.

In one embodiment of the sustained release tablet provided herein, the mixture comprising polyvinyl acetate and polyvinyl pyrrolidone further comprises silica.

In one embodiment of the sustained release tablet provided herein, the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% silica.

In one embodiment of the sustained release tablet provided herein, the compressed core comprising 4-aminopyridine further comprises a filler and a lubricant. In one embodiment, the filler is dibasic calcium phosphate dihydrate, and the lubricant is magnesium stearate.

In one aspect, provided herein is a sustained release tablet according to the invention comprising:
(a) a compressed core, wherein said compressed core comprises: (i) 4-aminopyridine, (ii) a polyethylene oxide with a molecular weight between about 1,000,000 and 10,000,000, (iii) a mixture consisting of about 80% polyvinyl acetate, about 19% polyvinyl pyrrolidone, about 0.8% sodium lauryl sulfate, and about 0.2% of silica, (iv) dibasic calcium phosphate dihydrate, and (v) magnesium stearate; and
(b) an amount of an ethylcellulose coat surrounding said compressed core, said amount of the ethylcellulose coat being in the range of about 5% w/w to about 10% w/w of the compressed core,
wherein the amount of 4-aminopyridine is in the range of about 4% w/w to about 6% w/w of the compressed core.

In one embodiment of the sustained release tablet provided herein, the amount of the ethylcellulose coat surrounding the compressed core is about 9% w/w of the compressed core.

In one aspect, provided herein is a sustained release tablet according to the invention comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and
(b) an amount of an ethylcellulose coat surrounding said compressed core, said amount of the ethylcellulose coat being in the range of about 5% w/w to about 7% w/w of the compressed core.

In one embodiment of the sustained release tablet provided herein, the amount of 4-aminopyridine is in the range of about 3% w/w to about 5% w/w of the compressed core and the amount of the ethylcellulose coat surrounding the compressed core is about 6% w/w of the compressed core.

In one aspect, provided herein is a sustained release tablet according to the invention comprising:
(a) a compressed core, wherein said compressed core comprises: (i) 4-aminopyridine, (ii) a polyethylene oxide with a molecular weight between about 1,000,000 and 10,000,000, (iii) a mixture consisting of about 80% polyvinyl acetate, about 19% polyvinyl pyrrolidone, about 0.8% sodium lauryl sulfate, and about 0.2% of silica, (iv) dibasic calcium phosphate dihydrate, and (v) magnesium stearate; and
(b) an amount of an ethylcellulose coat surrounding said compressed core, said amount of the ethylcellulose coat being in the range of about 8% w/w to about 10% w/w of the tablet,

wherein said sustained release tablet is the product of a process comprising a curing step after coating the compressed core with ethylcellulose, wherein said curing step comprises exposing the coated compressed core to a temperature in the range of 50-60 °C for a period of time of at least 1 hour, and
wherein the amount of 4-aminopyridine is in the range of about 4% w/w to about 6% w/w of the sustained release tablet.

In one embodiment, the polyethylene oxide in the sustained release tablet provided herein has a molecular weight between 4,000,000 and 8,000,000.

In one embodiment, the polyethylene oxide in the sustained release tablet provided herein has a molecular weight of 7,000,000.

In one embodiment, the total amount of the 4-aminopyridine in the tablet provided herein is in the range of about 1% w/w to about 10% w/w of the sustained release tablet.

In one embodiment, the total amount of the 4-aminopyridine in the tablet provided herein is in the range of about 1% w/w to about 10% w/w of the compressed core.

In one embodiment of the sustained release tablet provided herein, the amount of 4-aminopyridine in the compressed core is about 22 mg.

In one embodiment of the sustained release tablet provided herein, the amount of 4-aminopyridine in the compressed core is about 16.5 mg.

In one embodiment of the sustained release tablet provided herein, the amount of 4-aminopyridine in the compressed core is in the range of about 5 mg to about 12 mg.

In one aspect, provided herein is a sustained release tablet according to the invention comprising:
(a) a compressed core, wherein said compressed core comprises: (i) 4-aminopyridine, wherein the amount of 4-aminopyridine is in the range of about 4% w/w to about 6% w/w of the compressed core; (ii) a polyethylene oxide with a molecular weight between about 1,000,000 and 10,000,000, wherein the amount of the polyethylene oxide is in the range of about 10% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core, said amount of the ethylcellulose coat being in the range of about 5% w/w to about 10% w/w of the compressed core.

In one aspect, provided herein is a sustained release tablet according to the invention comprising:
(a) a compressed core, wherein said compressed core comprises: (i) 4-aminopyridine, wherein the amount of 4-aminopyridine is in the range of about 4% w/w to about 6% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 9% w/w of the compressed core.

In one embodiment of the foregoing, the amount of 4-aminopyridine in the compressed core is about 22 mg.

In one aspect, provided herein is a sustained release tablet according to the invention comprising:
(a) a compressed core, wherein said compressed core comprises: (i) 4-aminopyridine, wherein the amount of 4-aminopyridine is in the range of about 3% w/w to about 5% w/w of the compressed core; (ii) a polyethylene oxide with a molecular weight between about 1,000,000 and 10,000,000, wherein the amount of the polyethylene oxide is in the range of about 10% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core, said amount of the ethylcellulose coat being in the range of about 5% w/w to about 7% w/w of the compressed core.

In one aspect, provided herein is a sustained release tablet according to the invention comprising:
(a) a compressed core, wherein said compressed core comprises: (i) 4-aminopyridine, wherein the amount of 4-aminopyridine is in the range of about 3% w/w to about 5% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 6% w/w of the compressed core.

In one embodiment of the foregoing, the amount of 4-aminopyridine in the compressed core is about 16.5 mg.

In one embodiment, the sustained release tablet provided herein does not further comprise an immediate release drug overcoat containing 4-aminopyridine.

In one embodiment, the sustained release tablet provided herein provides a zero-order or near-zero-order release of the 4-aminopyridine. In one embodiment, the release is zero-order.

In one embodiment, the sustained release tablet provided herein is suitable for once daily oral administration.

In one embodiment, the sustained release tablet provided herein comprises an amount of 4-aminopyridine that is therapeutically effective over a period of 24 hours upon oral administration to a human patient.

In one embodiment, the release of the 4-aminopyridine from the sustained release tablet provided herein, upon subjecting the tablet to an in vitro dissolution test employing 50 mM Phosphate Buffer, pH 6.8 as dissolution medium, is as follows:
within the first 2 hours after the start of the test at most 30% w/w of the total amount of the 4-aminopyridine contained in the sustained release tablet is released.

In one embodiment, the release of the 4-aminopyridine from the sustained release tablet provided herein, upon subjecting the tablet to an in vitro dissolution test employing 50 mM Phosphate Buffer, pH 6.8 as dissolution medium, is as follows:
(a) within the first 2 hours after the start of the test at most 30% w/w of the total amount of the 4-aminopyridine contained in the sustained release tablet is released, and
(b) within the first 24 hours after the start of the test at least 80% w/w of the total amount of the 4-aminopyridine contained in the sustained release tablet is released.

In one embodiment, the release of the 4-aminopyridine from the sustained release tablet provided herein, upon subjecting the tablet to an in vitro dissolution test employing 50 mM Phosphate Buffer, pH 6.8 as dissolution medium, is as follows:
within the first 2 hours after the start of the test at most 20% w/w of the total amount of the 4-aminopyridine contained in the sustained release tablet is released.

In one embodiment, the release of the 4-aminopyridine from the sustained release tablet provided herein, upon subjecting the tablet to an in vitro dissolution test employing 50 mM Phosphate Buffer, pH 6.8 as dissolution medium, is as follows:
(a) within the first 2 hours after the start of the test at most 20% w/w of the total amount of the 4-aminopyridine contained in the sustained release tablet is released, and
(b) within the first 24 hours after the start of the test at least 80% w/w of the total amount of the 4-aminopyridine contained in the sustained release tablet is released.

In one embodiment, the sustained release tablet provided herein further comprises an immediate release drug overcoat containing 4-aminopyridine.

Provided herein is a method of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 10% w/w of the compressed core; and
(c) curing the coated compressed core by exposing it to a temperature in the range of 40-70 °C for a period of time of at least 1 hour.

Provided herein is a method of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 8% w/w to about 10% w/w of the sustained release tablet; and
(c) curing the coated compressed core by exposing it to a temperature in the range of 40-70 °C for a period of time of at least 1 hour.

In one embodiment, forming the compressed core in the method of making provided herein comprises:
(a) blending the 4-aminopyridine, polyethylene oxide, the mixture comprising polyvinyl acetate and polyvinyl pyrrolidone, and dibasic calcium phosphate dihydrate to form a blended mixture;
(b) adding magnesium stearate to the blended mixture to form a new blend; and
(c) compressing the new blend to form a compressed core.

In one embodiment of the method of making provided herein, the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% silica.

In one embodiment of the method of making provided herein, the polyethylene oxide has a molecular weight between 4,000,000 and 8,000,000. In one embodiment, the polyethylene oxide has a molecular weight of 7,000,000.

In one embodiment of the method of making provided herein, the coating comprises applying an aqueous ethylcellulose dispersion to the compressed core.

In one embodiment of the method of making provided herein, the total amount of the 4-aminopyridine in the sustained release tablet is in the range of about 1% w/w to about 10% w/w of the compressed core.

In one embodiment of the method of making provided herein, the total amount of the 4-aminopyridine in the sustained release tablet is in the range of about 1% w/w to about 10% w/w of the sustained release tablet.

Provided herein is a method of making a sustained release tablet comprising 4-aminopyridine according to the invention, which method comprises:
(a) forming a compressed core comprising (i) 4-aminopyridine, wherein the amount of 4-aminopyridine is in the range of about 4% w/w to about 6% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 9% w/w of the compressed core; and
(c) curing the coated compressed core by exposing it to a temperature in the range of 50-60 °C for a period of time of at least 1 hour.

In one embodiment of the foregoing method of making, the amount of 4-aminopyridine in the compressed core is about 22 mg.

Provided herein is a method of making a sustained release tablet comprising 4-aminopyridine according to the invention, which method comprises:
(a) forming a compressed core comprising (i) 4-aminopyridine, wherein the amount of 4-aminopyridine is in the range of about 3% w/w to about 5% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 6% w/w of the compressed core; and
(c) curing the coated compressed core by exposing it to a temperature in the range of 50-60 °C for a period of time of at least 1 hour.

In one embodiment of the foregoing method of making, the amount of 4-aminopyridine in the compressed core is about 16.5 mg.

In one embodiment of the method of making provided herein, the sustained release tablet is suitable for once daily oral administration.

In one embodiment of the method of making provided herein, the sustained release tablet comprises an amount of 4-aminopyridine that is therapeutically effective over a period of 24 hours.

Also provided herein is a sustained release tablet according to the invention for use in a method of treating a neurological disorder in a patient in need thereof comprising orally administering to the patient once daily the sustained release tablet provided herein.

Also provided herein is a sustained release tablet according to the invention for use in a method of treating a neurological disorder in a patient in need thereof comprising orally administering to the patient a therapeutically effective amount of the sustained release tablet provided herein, in combination with one or more additional medicaments. In one embodiment of the sustained release tablet for use in a method of treating provided herein, the neurological disorder is multiple sclerosis, and the one or more additional medicaments is a therapeutic agent effective for the treatment of multiple sclerosis. In one embodiment, the one or more therapeutic agents effective for the treatment of multiple sclerosis is selected from the group consisting of interferon beta-1a, interferon beta-1b, glatiramer acetate, mitoxantrone, natalizumab, teriflunomide, fingolimod, alemtuzumab, peginterferon beta-1a, dimethyl fumarate, prednisone, prednisolone, methylprednisolone, betamethasone, and dexamethasone.

In one embodiment of the sustained release tablet for use in a method of treating provided herein, the neurological disorder is multiple sclerosis or stroke.

In one embodiment of the sustained release tablet for use in a method of treating provided herein, the neurological disorder is multiple sclerosis.

In one embodiment of the sustained release tablet for use in a method of treating provided herein, the neurological disorder is a walking impairment associated with multiple sclerosis.

In one embodiment of the sustained release tablet for use in a method of treating provided herein, the neurological disorder is a neurocognitive or neuropsychiatric impairment associated with multiple sclerosis.

In one embodiment of the sustained release tablet for use in a method of treating provided herein, the neurological disorder is stroke.

In one embodiment of the sustained release tablet for use in a method of treating provided herein, the neurological disorder is a sensorimotor impairment associated with stroke.

In one embodiment of the sustained release tablet for use in a method of treating provided herein, the neurological disorder is a walking impairment associated with stroke.

In one embodiment of the sustained release tablet for use in a method of treating provided herein, the patient is a human patient.

### 4 BRIEF DESCRIPTION OF THE FIGURES

**Figure 1** depicts a dissolution profile of the sustained release tablet in Example 1 employing 50 mM Phosphate Buffer, pH 6.8 as a dissolution medium.
**Figure 2** depicts dissolution profiles of the sustained release tablet in Example 1 employing 0.1 N hydrochloric acid as a dissolution medium with and without 40% alcohol (ethanol).
**Figure 3** depicts dissolution profiles of sustained release tablets containing 22 mg 4-aminopyridine, coated to 8%, 9%, and 10% weight gain, employing 50 mM Phosphate Buffer, pH 6.8 as a dissolution medium.
**Figure 4** depicts dissolution profiles of sustained release tablets containing 16.5 mg 4-aminopyridine, coated to 5%, 6%, and 7% weight gain, employing 50 mM Phosphate Buffer, pH 6.8 as a dissolution medium.
**Figure 5** depicts a dissolution profile of the sustained release tablet in Example 4 employing 50 mM Phosphate Buffer, pH 6.8 as a dissolution medium.
**Figure 6** depicts mean plasma levels following administration of a single oral dose of the 22 mg 4-aminopyridine sustained release tablet of Example 1 in human subjects in fed and fasted states.
**Figure 7** depicts the arithmetic mean steady state plasma concentration profiles of Dalfampridine ER tablets on Day 7 described in Example 6.
**Figure 8** depicts the arithmetic mean dalfampridine plasma trough concentration profiles for all treatments described in Example 6.

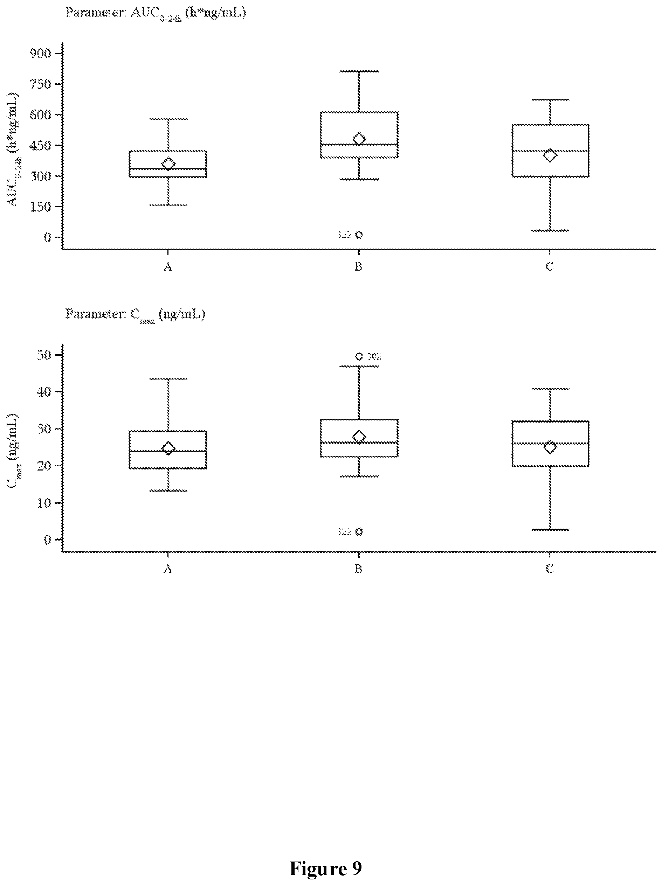

**Figure 9** depicts box plots of steady state pharmacokinetic parameters of Dalfampridine ER tablets described in Example 6. A = Dalfampridine-ER tablet 16.5 mg morning; B = Dalfampridine-ER tablet 22 mg morning; C = Dalfampridine-ER tablet 22 mg evening. The bottom and top edges of the box represents the 25th and 75th percentiles. The diamond in the box interior represents the mean, and the horizontal line in the box interior represents the median. The vertical lines issuing from the box extend to minimum data values greater than or equal to the lower fence and maximum data value less than or equal to the upper fence of the analysis variable. Any values outside ± 1.5 times interquartile range are presented as outliers (empty circle).
**Figure 10** depicts the arithmetic mean single dose plasma concentration profiles of Dalfampridine ER tablets on Day 1 described in Example 6.

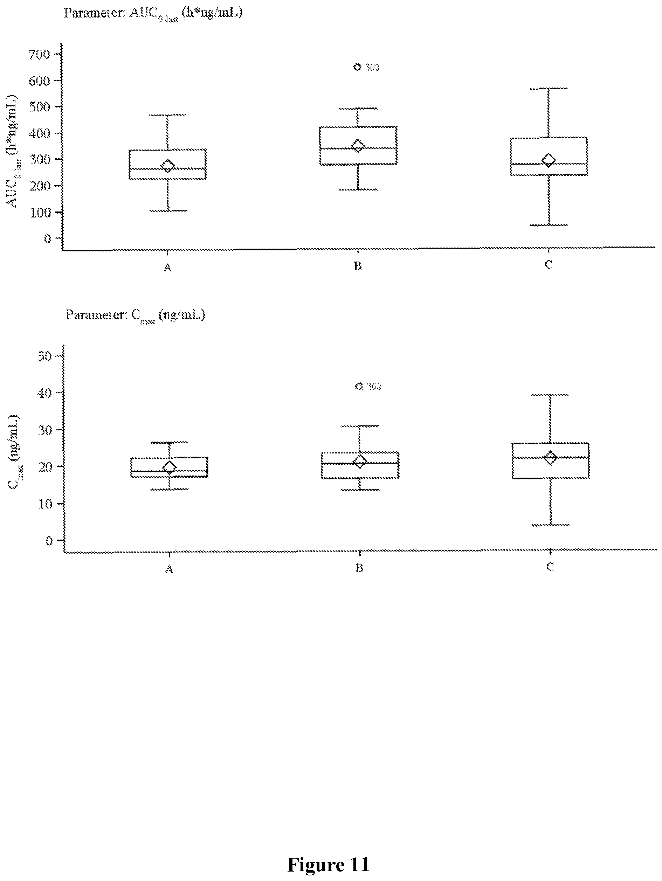

**Figure 11** depicts box plots of single dose pharmacokinetic parameters of Dalfampridine ER tablets described in Example 6. A = Dalfampridine-ER tablet 16.5 mg morning; B = Dalfampridine-ER tablet 22 mg morning; C = Dalfampridine-ER tablet 22 mg evening. The bottom and top edges of the box represents the 25^{th} and 75^{th} percentiles. The diamond in the box interior represents the mean, and the horizontal line in the box interior represents the median. The vertical lines issuing from the box extend to minimum data values greater than or equal to the lower fence and maximum data value less than or equal to the upper fence of the analysis variable. Any values outside ± 1.5 times interquartile range are presented as outliers (empty circle).

### 5 DETAILED DESCRIPTION

### 5.1 Sustained Release Tablets

The invention provides sustained release tablets comprising 4-aminopyridine as the active ingredient, preferably for once daily administration.
Specifically, the invention provides a sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core;
   (b1) wherein said amount of the ethylcellulose coat is in the range of about 5% w/w to about 10% w/w of the compressed core; or
   (b2) wherein the ratio of the amount of the ethylcellulose coat to the amount of 4-aminopyridine in the compressed core is in the range of about 0.5:1 to about 3:1, wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core; or
   (b3) wherein the ratio of the amount of the ethylcellulose coat to the amount of 4-aminopyridine in the compressed core is in the range of about 0.1:1 to about 0.7:1, wherein for calculating said ratio, the amount of the ethylcellulose coat is the weight percentage of the ethylcellulose coat by weight of the compressed core, and the amount of 4-aminopyridine is the weight in milligrams of 4-aminopyridine;
   wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h; or wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

In one embodiment, provided herein are sustained release tablets according to the invention comprising: (a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and (b) an amount of an ethylcellulose coat surrounding said compressed core,
wherein the ratio of the amount of the ethylcellulose coat to the amount of 4-aminopyridine in the compressed core is in the range of about 0.5:1 to about 3:1; wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core. In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes.

Also provided herein are sustained release tablets according to the invention comprising: (a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and (b) an amount of an ethylcellulose coat surrounding said compressed core,
wherein the ratio of the amount of the ethylcellulose coat to the amount of 4-aminopyridine in the compressed core is in the range of about 0.1:1 to about 0.7:1; wherein for calculating said ratio, the amount of the ethylcellulose coat is the weight percentage of the ethylcellulose coat by weight of the compressed core, and the amount of 4-aminopyridine is the weight in milligrams of 4-aminopyridine. In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes.

Also provided herein are sustained release tablets according to the invention comprising: (a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and (b) an amount of an ethylcellulose coat surrounding said compressed core, said amount of the ethylcellulose coat being in the range of about 8% w/w to about 10% w/w of the tablet. In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes.

Also provided herein are sustained release tablets according to the invention comprising: (a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and (b) an amount of an ethylcellulose coat surrounding said compressed core, said amount of the ethylcellulose coat being in the range of about 5% w/w to about 10% w/w of the compressed core. In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes.

Also provided herein are sustained release tablets according to the invention comprising:
(a) a compressed core, wherein said compressed core comprises (i) 4-aminopyridine, wherein the amount of 4-aminopyridine is in the range of about 4% w/w to about 6% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 9% w/w of the compressed core.

In a specific embodiment of the foregoing embodiment, the amount of 4-aminopyridine in the compressed core is about 22 mg. In another specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

Also provided herein are sustained release tablets according to the invention comprising:
(a) a compressed core, wherein said compressed core comprises (i) 4-aminopyridine, wherein the amount of 4-aminopyridine is in the range of about 4% w/w to about 6% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 5% w/w of the compressed core.

In a specific embodiment of the foregoing embodiment, the amount of 4-aminopyridine in the compressed core is about 22 mg. In another specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

Also provided herein are sustained release tablets according to the invention comprising: (a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and (b) an amount of an ethylcellulose coat surrounding said compressed core, said amount of the ethylcellulose coat being in the range of about 5% w/w to about 7% w/w of the compressed core. In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes.

Also provided herein are sustained release tablets according to the invention comprising:
(a) a compressed core, wherein said compressed core comprises (i) 4-aminopyridine, wherein the amount of 4-aminopyridine is in the range of about 3% w/w to about 5% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 6% w/w of the compressed core.

In a specific embodiment of the foregoing embodiment, the amount of 4-aminopyridine in the compressed core is about 16.5 mg. In another specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In specific embodiments, the sustained release tablets comprise, or consist essentially of, or consist of, the components as described herein. In particular embodiments, the compressed cores of the sustained release tablets comprise, or consist essentially of, or consist of, the components as described herein. The sustained release tablets are pharmaceutically acceptable. The terms "sustained release" and "extended release" as used herein are generally synonymous unless the context clearly indicates otherwise.

In the sustained release tablets according to the invention, the compressed core provided herein comprises 4-aminopyridine, a polyethylene oxide with a molecular weight between about 1,000,000 and 10,000,000, and a mixture comprising polyvinyl acetate and polyvinyl pyrrolidone. In a specific embodiment, the compressed core provided herein consists essentially of 4-aminopyridine, a polyethylene oxide with a molecular weight between about 1,000,000 and 10,000,000, and a mixture comprising polyvinyl acetate and polyvinyl pyrrolidone. In a specific embodiment, the mixture consists essentially of polyvinyl acetate and polyvinyl pyrrolidone, and optionally a surfactant and optionally silica.

In a specific embodiment, the amount of 4-aminopyridine in the sustained release tablet is in the range of about 1% w/w to about 10% w/w of the sustained release tablet. In some embodiments, the amount of 4-aminopyridine in the sustained release tablet is in range of about 1% w/w to about 7% w/w, about 1% w/w to about 5% w/w, about 1% w/w to about 3% w/w, about 1% w/w to about 2% w/w, about 2% w/w to about 10% w/w, about 2% w/w to about 7% w/w, about 2% w/w to about 5% w/w, about 2% w/w to about 3% w/w, about 3% w/w to about 10% w/w, about 3% w/w to about 7% w/w, about 3% w/w to about 5% w/w, about 3% w/w to about 4% w/w, about 4% w/w to about 10% w/w, about 4% w/w to about 7% w/w, about 4% w/w to about 6% w/w, about 4% w/w to about 5% w/w, about 5% w/w to about 10% w/w, about 5% w/w to about 7% w/w, about 5% w/w to about 6% w/w, 7% w/w to about 10% w/w, about 7% w/w to about 8% w/w, about 7% w/w to about 9% w/w, about 8% w/w to about 10% w/w, about 8% w/w to about 9% w/w, or about 9% w/w to about 10% w/w of the sustained release tablet.

In a specific embodiment, the amount of 4-aminopyridine in the compressed core is in the range of about 1% w/w to about 10% w/w of the compressed core. In some embodiments, the amount of 4-aminopyridine in the compressed core is in range of about 1% w/w to about 7% w/w, about 1% w/w to about 5% w/w, about 1% w/w to about 3% w/w, about 1% w/w to about 2% w/w, about 2% w/w to about 10% w/w, about 2% w/w to about 7% w/w, about 2% w/w to about 5% w/w, about 2% w/w to about 3% w/w, about 3% w/w to about 10% w/w, about 3% w/w to about 7% w/w, about 3% w/w to about 5% w/w, about 3% w/w to about 4% w/w, about 4% w/w to about 10% w/w, about 4% w/w to about 7% w/w, about 4% w/w to about 6% w/w, about 4% w/w to about 5% w/w, about 5% w/w to about 10% w/w, about 5% w/w to about 7% w/w, about 5% w/w to about 6% w/w, 7% w/w to about 10% w/w, about 7% w/w to about 8% w/w, about 7% w/w to about 9% w/w, about 8% w/w to about 10% w/w, about 8% w/w to about 9% w/w, or about 9% w/w to about 10% w/w of the compressed core. In some embodiments, the amount of 4-aminopyridine in the compressed core is about 2% w/w, about 2.25% w/w, about 2.5% w/w, about 2.75% w/w, about 3% w/w, about 3.25% w/w, about 3.5% w/w, about 3.75% w/w, about 4% w/w, about 4.25% w/w, about 4.5% w/w, about 4.75% w/w, or about 5% w/w of the compressed core. In a specific embodiment, the amount of 4-aminopyridine in the compressed core is in the range of about 3% w/w to about 5% w/w of the compressed core. In another specific embodiment, the amount of 4-aminopyridine in the compressed core is in the range of about 4% w/w to about 6% w/w of the compressed core. In a specific embodiment, the amount of 4-aminopyridine in the compressed core is about 2.5% w/w of the compressed core. In a specific embodiment, the amount of 4-aminopyridine in the compressed core is about 3.75% w/w of the compressed core. In a specific embodiment, the amount of 4-aminopyridine in the compressed core is about 5% w/w of the compressed core.

In some embodiments, the amount of 4-aminopyridine in the compressed core is in the range of about 5 mg to about 20 mg, about 5 mg to about 15 mg, about 5 mg to about 10 mg, about 10 mg to about 20 mg, about 10 mg to about 15 mg, about 15 mg to about 20 mg. In a specific embodiment, the amount of 4-aminopyridine in the compressed core is about 16 mg, about 16.2 mg, about 16.4 mg, about 16.6 mg, about 16.8 mg, about 17 mg, about 17.2 mg, about 17.4 mg, about 17.6 mg, about 17.8 mg, about 18 mg, about 18.2 mg, about 18.4 mg, about 18.6 mg, about 18.8 mg, about 19 mg, about 19.2 mg, about 19.4 mg, about 19.6 mg, about 19.8 mg, about 20 mg, about 20.2 mg, about 20.4 mg, about 20.6 mg, about 20.8 mg, about 21 mg, about 21.2 mg, about 21.4 mg, about 21.6 mg, about 21.8 mg, about 22 mg. In one embodiment, the amount of 4-aminopyridine in the compressed core is in the range of about 12 mg to about 15 mg. In one embodiment, the amount of 4-aminopyridine in the compressed core is in the range of about 5 mg to about 12 mg. In a specific embodiment, the amount of 4-aminopyridine in the compressed core is about 22 mg. In another specific embodiment, the amount of 4-aminopyridine in the compressed core is about 16.5 mg. In another specific embodiment, the amount of 4-aminopyridine in the compressed core is about 11 mg. In a preferred embodiment, 4-aminopyridine is present only in the compressed core (and thus the sustained release tablet does not have an immediate-release drug overcoat containing 4-aminopyridine). In an alternative specific embodiment, 4-aminopyridine is present only in: the compressed core and in an immediate-release drug overcoat of the sustained release tablet.

In a specific embodiment, the amount of 4-aminopyridine in the compressed core is about 5% w/w of the compressed core, said amount being equal to about 22 mg. In another specific embodiment, the amount of 4-aminopyridine in the sustained release tablet is about 3.75% w/w of the compressed core, said amount being equal to about 16.5 mg.

The sustained release tablet provided herein comprises one or more polymers that are capable of modifying the release rate of the 4-aminopyridine from the sustained release tablet. Examples of such release modifying polymers include, but are not limited to, polyethylene oxide, a mixture of polyvinyl acetate and polyvinyl pyrrolidone (e.g., KOLLIDON^{®} SR), hypromellose, ethylcellulose, povidone, and any combination thereof. In one embodiment, the sustained release tablet comprises polyethylene oxide and a mixture of polyvinyl acetate and polyvinyl pyrrolidone (e.g., KOLLIDON^{®} SR).

Polyethylene oxide is a hydrophilic, water soluble polymer with good binding, thickening, lubricity, water retention, and film forming properties. Polyethylene oxide also has beneficial effects in retarding the release rate of a drug from a suitable pharmaceutical composition. In a specific embodiment, the polyethylene oxide present in the sustained release tablet disclosed herein has a molecular weight between 4,000,000 and 8,000,000. In another specific embodiment, the polyethylene oxide has a molecular weight of 7,000,000. In another specific embodiment, the polyethylene oxide is sold under the name POLYOX WSR-303 available from Dow Chemical Co.

In some embodiments, the amount of the polyethylene oxide in the compressed core is in the range of about 5% w/w to about 25% w/w of the compressed core. In some embodiments, the amount of the polyethylene oxide in the compressed core is in the range of about 5% w/w to about 20% w/w, about 5% w/w to about 15% w/w, about 5% w/w to about 10% w/w, about 10% w/w to about 25% w/w, 10% w/w to about 20% w/w, about 10% w/w to about 15% w/w, about 12% w/w to about 25% w/w, about 12% w/w to about 20% w/w, about 12% w/w to about 15% w/w, about 15% w/w to about 25% w/w, 15% w/w to about 20% w/w, or about 20% w/w to about 25% w/w of the compressed core. In a specific embodiment, the amount of the polyethylene oxide in the compressed core is about 10% w/w, about 10.5% w/w, about 11% w/w, about 11.5% w/w, about 12.5% w/w, about 13% w/w, about 13.5% w/w, about 14% w/w, about 14.5% w/w, about 15% w/w, about 15.5% w/w, about 16% w/w, about 16.5% w/w, or about 17% w/w of the compressed core. In a specific embodiment, the amount of the polyethylene oxide in the compressed core is in the range of about 10% w/w to about 20% w/w of the compressed core. In a specific embodiment, the amount of the polyethylene oxide in the compressed core is about 15% w/w of the compressed core. In another specific embodiment, the polyethylene oxide in the compressed core is a polyethylene oxide having a molecular weight of about 7,000,000, which is present in an amount of about 15% w/w of the compressed core.

In a specific embodiment, the mixture of polyvinyl acetate and polyvinyl pyrrolidone present in the sustained release tablet disclosed herein further comprises a surfactant. In a specific embodiment, the mixture further comprises silica. In another specific embodiment, the mixture further comprises a surfactant and silica. The surfactants suitable for use in the mixture provided herein include, but are not limited to, sodium lauryl sulfate, polyethylene stearates, polyethylene sorbitan fatty acid esters, polyoxyethylene castor oil derivatives, polyoxyethylene alkyl ethers, benzyl benzoate, cetrimide, cetyl alcohol, docusate sodium, glyceryl monooleate, glyceryl monostearate, glyceryl palmitostearate, lecithin, medium chain triglycerides, monoethanolamine, oleic acid, poloxamers, polyvinyl alcohol and sorbitan fatty acid esters. In a specific embodiment, the surfactant is sodium lauryl sulfate.

In specific embodiments, the mixture of polyvinyl acetate and polyvinyl pyrrolidone comprises 70-90% polyvinyl acetate and 15-20% polyvinyl pyrrolidone. In a specific embodiment, the mixture consists of about 80% polyvinyl acetate, about 19% polyvinyl pyrrolidone, about 0.8% sodium lauryl sulfate, and about 0.2% of silica. In one embodiment, the mixture comprising polyvinyl acetate and polyvinyl pyrrolidone is KOLLIDON^{®} SR polyvinyl acetate/polyvinyl pyrrolidone (BASF); KOLLIDON^{®} SR is a blend of 80% polyvinyl acetate and 19% povidone (also called polyvinyl pyrrolidone) along with small quantities of sodium lauryl sulfate and silica used as stabilizers (see Signet Chemical Corporation's website page for KOLLIDON^{®} SR).

In some embodiments, the amount of the mixture comprising polyvinyl acetate and polyvinyl pyrrolidone in the compressed core is in the range of about 10% w/w to about 40% w/w of the compressed core.

In some embodiments, the amount of the mixture comprising polyvinyl acetate and polyvinyl pyrrolidone in the compressed core is in the range of about 10% w/w to about 35% w/w, about 10% w/w to about 30% w/w, about 10% w/w to about 25% w/w, about 10% w/w to about 20% w/w, about 10% w/w to about 15% w/w, about 15% w/w to about 40% w/w, about 15% w/w to about 35% w/w, about 15% w/w to about 30% w/w, about 15% w/w to about 25% w/w, about 15% w/w to about 20% w/w, about 20% w/w to about 40% w/w, about 20% w/w to about 35% w/w, about 20% w/w to about 30% w/w, about 20% w/w to about 25% w/w, about 25% w/w to about 40% w/w, about 25% w/w to about 35% w/w, about 25% w/w to about 30% w/w, about 30% w/w to about 40% w/w, about 30% w/w to about 35% w/w, or about 35% w/w to about 40% w/w of the compressed core. In a specific embodiment, the amount of the mixture comprising polyvinyl acetate and polyvinyl pyrrolidone in the compressed core is in the range of about 20% w/w to about 30% w/w. In some embodiments, the amount of the mixture comprising polyvinyl acetate and polyvinyl pyrrolidone in the compressed core is about 20% w/w, about 20.5% w/w, about 21% w/w, about 21.5% w/w, about 22% w/w, about 22.5% w/w, about 23% w/w, about 23.5% w/w, about 24% w/w, about 24.5% w/w, about 25% w/w, about 25.5% w/w, about 26% w/w, about 26.5% w/w, about 27% w/w, about 27.5% w/w, about 28% w/w, about 28.5% w/w, about 29% w/w, about 29.5% w/w, or about 30% w/w of the compressed core. In a specific embodiment, the amount of the mixture comprising polyvinyl acetate and polyvinyl pyrrolidone in the compressed core is about 25% w/w of the compressed core.

In addition to the polymers described herein, one or more fillers, binders, lubricants and the like may be used in the compressed core of the invention.

Suitable fillers include, as described in US Patent 8,858,993, but are not limited to, inorganic compounds such as the chloride, sulfate, and phosphate salts of potassium, sodium and magnesium as well as calcium citrate, phosphate, lactate, gluconate and succinate salts. In a specific embodiment, the filler is dibasic calcium phosphate dihydrate.

The concentration for the filler can be in the range of about 40% w/w to about 60% w/w of the sustained release tablet. In a specific embodiment, the amount of the filler in the compressed core is in the range of about 47% w/w to about 53% w/w of the sustained release tablet.

In some embodiments, the amount of the filler in the compressed core is in the range of about 40% w/w to about 65% w/w of the compressed core. In some embodiments, the amount of the filler in the compressed core is in the range of about 40% w/w to about 60% w/w about 40% w/w to about 55% w/w, about 40% w/w to about 50% w/w, about 40% w/w to about 45% w/w, about 45% w/w to about 65% w/w, about 45% w/w to about 60% w/w, about 45% w/w to about 55% w/w, about 45% w/w to about 50% w/w, about 50% w/w to about 65% w/w, about 50% w/w to about 60% w/w, about 50% w/w to about 55% w/w, about 55% w/w to about 65% w/w, about 55% w/w to about 60% w/w, or about 60% w/w to about 65% w/w of the compressed core. In a specific embodiment, the amount of the filler in the compressed core is in the range of about 50% w/w to about 60% w/w of the compressed core. In some embodiments, the amount of the filler in the compressed core is about 45% w/w, about 46% w/w, about 47% w/w, about 48% w/w, about 49% w/w, about 50% w/w, about 51% w/w, about 52% w/w, about 53% w/w, about 54% w/w, about 55% w/w, about 56% w/w, about 57% w/w, about 58% w/w, about 58% w/w, or about 60% w/w of the compressed core. In a specific embodiment, the amount of the filler in the compressed core is about 54% w/w of the compressed core. In another specific embodiment, the amount of the filler in the compressed core is about 55% w/w of the compressed core. In a specific embodiment, the filler is dibasic calcium phosphate dihydrate in an amount of about 54% w/w of the compressed core. In another specific embodiment, the filler is dibasic calcium phosphate dihydrate in an amount of about 55% w/w of the compressed core.

Pharmaceutically acceptable binders suitable for use in the compressed core of the present invention include, as described in US Patent 8,858,993, but are not limited to, sucrose, gelatin, acacia, tragacanth, cellulose derivatives, povidone, and other binders known to those familiar with pharmaceutical formulations.

The compressed core provided herein may further comprise one or more lubricants. Examples of lubricants include, but are not limited to, stearate salts; e.g., calcium stearate, magnesium stearate, zinc stearate, and sodium stearyl fumarate; stearic acid; mineral oil; vegetable oil derivatives; talc; and the like. In a specific embodiment, the lubricant is magnesium stearate.

The concentration ranges for lubricants can be up to about 5% by weight of the sustained release tablet. In general, lubricants are present at a concentration of 0.5-5% by weight of the sustained release tablet. In a specific embodiment, the amount of lubricant in the compressed core is in the range of about 0.7% w/w to about 1.3% w/w of the sustained release tablet. In some embodiments, the compressed core contains no lubricant.

In some embodiments, the amount of the lubricant in the compressed core is up to about 5% w/w of the compressed core. In some embodiments, the amount of lubricant in the compressed core is in the range of about 0.5% w/w to about 5% w/w of the compressed core. In a specific embodiment, the amount of lubricant in the compressed core is in the range of about 0.7% w/w to about 1.3% w/w of the compressed core. In a specific embodiment, the amount of the lubricant in the compressed core is about 1% w/w of the compressed core. In a specific embodiment, the lubricant is magnesium stearate in an amount of about 1% w/w of the compressed core.

In specific embodiments, the core comprising the 4-aminopyridine, polyethylene oxide polymer, mixture of polyvinyl acetate and polyvinyl pyrrolidone, and optionally one or more fillers, binders and lubricants is compressed to form intermediate tablets by methods well known in the art. The compressed core as described herein refers to such intermediate tablets. The compressed core (i.e., the intermediate tablets) is coated with a coating agent, and the resulting coated tablets are subjected to a curing step by exposing the coated tablets to a thermal treatment to yield the final sustained release tablets.

The release profile of 4-aminopyridine from the tablet can be modulated by coating the compressed core (i.e., the intermediate tablet) with a suitable coating agent. In specific embodiments, the compressed core is coated with a solution of ethyl cellulose or other cellulosic materials. In specific embodiments, the compressed core is coated with a colloidal suspension or a dispersion of ethyl cellulose, optionally with additional components such as light silicic acid anhydrides. Such light silicic anhydrides are described in The Pharmacopoeia of Japan XII and are commercially available under the trade name of, for example, AEROSIL-200 (produced by Nippon Aerosil Co., Ltd.). In some embodiments, an ethyl ether of cellulose having an ethoxy group content of 46 to 51 % is employed as a coating agent. This type of ethyl cellulose is commercially available under the trade name, for example, of ETHOCEL STANDARD (produced by Dow Chemical Co., Ltd.) and the like. In certain embodiments, the coating agent comprises a solution of ethyl cellulose in ethanol.

In a specific embodiment, the coating agent comprises pH independent ethylcellulose. A "pH independent" ethylcellulose provides for a pH independent release of the 4-aminopyridine from the tablet. In a specific embodiment, the coating agent is an aqueous ethylcellulose dispersion. In a specific embodiment, the coating agent is SURELEASE^{®}, commercially available from Colorcon. SURELEASE^{®} is a pH independent ethylcellulose provided as an aqueous ethylcellulose dispersion. Another aqueous ethylcellulose dispersion that can be used as the coating agent is AQUACOAT^{®} ECD Ethylcellulose Aqueous Dispersion, commercially available from FMC BioPolymer. As will be clear, the ethylcellulose coat can contain other compounds in addition to ethylcellulose, but ethylcellulose will be the predominant component by weight of the ethylcellulose coat. In a specific embodiment, the amount of the ethylcellulose coat surrounding the compressed core is 8-10% w/w of the final sustained release tablet.

In one embodiment, the amount of the ethylcellulose coat surrounding the compressed core is in the range of about 1% w/w to about 20% w/w of the compressed core. In a specific embodiment, the amount of the ethylcellulose coat surrounding the compressed core is in the range of about 5% w/w to about 10% w/w of the compressed core. In some embodiments, the amount of the ethylcellulose coat surrounding the compressed core is above 8% w/w and up to about 20% w/w of the compressed core. In a specific embodiment, the amount of the ethylcellulose coat surrounding the compressed core is above 8% w/w and up to about 12% w/w or above 8% w/w and up to about 10% w/w of the compressed core. In another specific embodiment, the amount of the ethylcellulose coat surrounding the compressed core is in the range of about 8% w/w to about 10% w/w of the compressed core. In some embodiments, the amount of the ethylcellulose coat surrounding the compressed core is in the range of about 8% w/w to about 9.5% w/w, about 8% w/w to about 9% w/w, 8% w/w to about 8.5% w/w, about 9% w/w to about 10% w/w, about 9% w/w to about 9.5% w/w, or about 9.5% w/w to about 10% w/w of the compressed core. In some embodiments, the amount of the ethylcellulose coat surrounding the compressed core is about 8% w/w, about 8.1% w/w, about 8.2% w/w, 8.3% w/w, about 8.4% w/w, about 8.5% w/w, about 8.6% w/w, about 8.7% w/w, about 8.8% w/w, about 8.9% w/w, about 9% w/w, about 9.1% w/w, about 9.2% w/w, about 9.3% w/w, about 9.4% w/w, about 9.5% w/w, about 9.6% w/w, about 9.7% w/w, about 9.8% w/w, about 9.9% w/w, or about 10% w/w of the compressed core. In a specific embodiment, the amount of the ethylcellulose coat surrounding the compressed core is about 8% w/w of the compressed core. In a specific embodiment, the amount of the ethylcellulose coat surrounding the compressed core is about 9% w/w of the compressed core. In a specific embodiment, the amount of the ethylcellulose coat surrounding the compressed core is about 10% w/w of the compressed core.

In some embodiments, the amount of the ethylcellulose coat surrounding the compressed core is in the range of about 5% w/w to about 8% w/w of the compressed core. In some embodiments, the amount of the ethylcellulose coat surrounding the compressed core is in the range of about 5% w/w to about 7% w/w of the compressed core. In some embodiments, the amount of the ethylcellulose coat surrounding the compressed core is in the range of about 5% w/w to about 7.5% w/w, about 5% w/w to about 7% w/w, about 5% w/w to about 6.5% w/w, about 5% w/w to about 6% w/w, 5% w/w to about 5.5% w/w, about 5.5% w/w to about 8% w/w, about 5.5% w/w to about 7.5% w/w, about 5.5% w/w to about 7% w/w, about 5.5% w/w to about 6.5% w/w, about 5.5% w/w to about 6% w/w, about 6% w/w to about 8% w/w, about 6% w/w to about 7.5% w/w, about 6% w/w to about 7% w/w, about 6.5% w/w to about 8% w/w, about 6.5% w/w to about 7.5% w/w, about 6.5% w/w to about 7% w/w, about 7% w/w to about 8% w/w, about 7% w/w to about 7.5% w/w, or about 7.5% w/w to about 8% w/w of the compressed core. In some embodiments, the amount of the ethylcellulose coat surrounding the compressed core is about 5% w/w, about 5.1% w/w, about 5.2% w/w, 5.3% w/w, about 5.4% w/w, about 5.5% w/w, about 5.6% w/w, about 5.7% w/w, about 5.8% w/w, about 5.9% w/w, about 6% w/w, about 6.1% w/w, about 6.2% w/w, about 6.3% w/w, about 6.4% w/w, about 6.5% w/w, about 6.6% w/w, about 6.7% w/w, about 6.8% w/w, about 6.9% w/w, or about 7% w/w of the compressed core. In a specific embodiment, the amount of the ethylcellulose coat surrounding the compressed core is about 5% w/w of the compressed core. In a specific embodiment, the amount of the ethylcellulose coat surrounding the compressed core is about 6% w/w of the compressed core. In a specific embodiment, the amount of the ethylcellulose coat surrounding the compressed core is about 7% w/w of the compressed core.

As will be clear, the amount of the ethylcellulose coat expressed in terms of % w/w of the compressed core is equivalent to the amount of the ethylcellulose coat expressed in terms of % weight gain. In other words, by way of example, if the weight of the compressed core is 100 mg, a 10% weight gain after coating the compressed core with the ethylcellulose coat is an amount of the ethylcellulose coat that is equal to 10% w/w of the compressed core, which is 10 mg.

In one embodiment, the amount of the ethylcellulose coat surrounding the compressed core is in the range of about 5% w/w to about 10% w/w of the compressed core, and the amount of 4-aminopyridine in the compressed core is in the range of about 4% w/w to about 6% w/w of the compressed core. In a specific embodiment, the amount of the ethylcellulose coat surrounding the compressed core is in the range of about 5% w/w to about 10% w/w of the compressed core, and the amount of 4-aminopyridine in the compressed core is about 22 mg. In another specific embodiment, the amount of the ethylcellulose coat surrounding the compressed core is about 5% w/w of the compressed core, and the amount of 4-aminopyridine in the compressed core is in the range of about 4% w/w to about 6% w/w of the compressed core. In another specific embodiment, the amount of the ethylcellulose coat surrounding the compressed core is about 5% w/w of the compressed core, and the amount of 4-aminopyridine in the compressed core is about 22 mg.

In a specific embodiment, the amount of the ethylcellulose coat surrounding the compressed core is in the range of about 8% w/w to about 10% w/w of the compressed core, and the amount of 4-aminopyridine in the compressed core is in the range of about 4% w/w to about 6% w/w of the compressed core. In another specific embodiment, the amount of the ethylcellulose coat surrounding the compressed core is in the range of about 8% w/w to about 10% w/w of the compressed core, and the amount of 4-aminopyridine in the compressed core is about 22 mg. In another specific embodiment, the amount of the ethylcellulose coat surrounding the compressed core is about 9% w/w of the compressed core, and the amount of 4-aminopyridine in the compressed core is in the range of about 4% w/w to about 6% w/w of the compressed core. In another specific embodiment, the amount of the ethylcellulose coat surrounding the compressed core is about 9% w/w of the compressed core, and the amount of 4-aminopyridine in the compressed core is about 22 mg.

In one embodiment, the amount of the ethylcellulose coat surrounding the compressed core is in the range of about 5% w/w to about 7% w/w of the compressed core, and the amount of 4-aminopyridine in the compressed core is in the range of about 3% w/w to about 5% w/w of the compressed core. In another specific embodiment, the amount of the ethylcellulose coat surrounding the compressed core is about 6% w/w of the compressed core, and the amount of 4-aminopyridine in the compressed core is in the range of about 3% w/w to about 5% w/w of the compressed core. In another specific embodiment, the amount of the ethylcellulose coat surrounding the compressed core is in the range of about 5% w/w to about 7% w/w of the compressed core, and the amount of 4-aminopyridine in the compressed core is about 16.5 mg.

In one embodiment, the amount of the ethylcellulose coat surrounding the compressed core is in the range of about 2% w/w to about 5% w/w of the compressed core, and the amount of 4-aminopyridine in the compressed core is about 16.5 mg. In another specific embodiment, the amount of the ethylcellulose coat surrounding the compressed core is about 3% w/w of the compressed core, and the amount of 4-aminopyridine in the compressed core is about 16.5 mg. In another specific embodiment, the amount of the ethylcellulose coat surrounding the compressed core is about 4% w/w of the compressed core, and the amount of 4-aminopyridine in the compressed core is about 16.5 mg. In another specific embodiment, the amount of the ethylcellulose coat surrounding the compressed core is about 5% w/w of the compressed core, and the amount of 4-aminopyridine in the compressed core is about 16.5 mg.

In one embodiment, the ratio of the amount of the ethylcellulose coat surrounding the compressed core to the amount of 4-aminopyridine in the compressed core is in the range of about 0.5:1 to about 3:1; wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core. In a preferred embodiment, this ratio of the amount of the ethylcellulose coat to 4-aminopyridine is in the range of about 1:1 to about 2:1. In a specific embodiment, the ratio of the amount of the ethylcellulose coat to 4-aminopyridine is about 1.6:1. In another specific embodiment, the ratio of the amount of the ethylcellulose coat to 4-aminopyridine is about 1.8:1.

In one embodiment, the ratio of the amount of the ethylcellulose coat surrounding the compressed core to the amount of 4-aminopyridine in the compressed core is in the range of about 0.1:1 to about 0.7:1; wherein for calculating said ratio, the amount of the ethylcellulose coat is the weight percentage of the ethylcellulose coat by weight of the compressed core, and the amount of 4-aminopyridine is the weight in milligrams of 4-aminopyridine. In a preferred embodiment, this ratio of the amount of the ethylcellulose coat to 4-aminopyridine is in the range of about 0.2:1 to about 0.5:1. In a specific embodiment, the ratio of the amount of the ethylcellulose coat to 4-aminopyridine is about 0.35:1. In another specific embodiment, the ratio of the amount of the ethylcellulose coat to 4-aminopyridine is about 0.4:1.

In some embodiments, the compressed core (i.e., the intermediate core tablet) described herein is optionally coated with a moisture barrier coat (e.g., an OPADRY^{®} Clear coat) prior to coating with the release modifying coating agent (e.g., SURELEASE^{®}). In a specific embodiment, the moisture barrier coat comprises a mixture of hypromellose and polyethylene glycol (e.g., OPADRY^{®} Clear, commercially available from Colorcon). In a specific embodiment, the moisture barrier coat comprises a mixture of poly (vinyl alcohol) and polyethylene glycol (e.g., OPADRY^{®} AMB (white) or OPADRY^{®} II Clear, both commercially available from Colorcon). In a specific embodiment, the moisture barrier coat comprises poly (vinyl alcohol) (e.g., OPADRY^{®} AMB II Clear, commercially available from Colorcon). In a specific embodiment, the moisture barrier coat comprises dewaxed orange shellac (e.g., MARCOAT^{®}, commercially available from Emerson Resources). In a specific embodiment, the moisture barrier coat comprises a mixture of methyl methacrylate and diethylaminoethyl methacrylate copolymer dispersion (e.g., KOLLICOAT^{®} Smartseal 30 D, commercially available from BASF). In a specific embodiment, the moisture barrier coat comprises a mixture of polyvinyl alcohol-polyethylene glycol copolymer and polyvinyl alcohol (e.g., KOLLICOAT^{®} Protect, commercially available from BASF). In a specific embodiment, the moisture barrier coat comprises a mixture of hypromellose and polyethylene glycol. In any of the foregoing specific embodiments, the moisture barrier coat alternatively can consist or consist essentially of, instead of comprising, the specified components. In a specific embodiment, the moisture barrier coat is OPADRY^{®} Clear, commercially available from Colorcon. In a specific embodiment, the sustained release 4-aminopyridine tablet comprising a moisture barrier coat (e.g., an OPADRY^{®} Clear coat) and a release modifying coating agent (e.g., SURELEASE^{®}) is prepared as described in Example 8.

In a specific embodiment, the coated compressed core is subjected to a post coating thermal treatment, also referred to as a curing step. In a specific embodiment, the curing step comprises heating the coated compressed core to a temperature above 23 °C for a period of time of at least 15 minutes. In a specific embodiment, the curing step comprises exposing the coated compressed core to a temperature in the range of 40-70 °C for a period of time of at least 15 minutes, 30 minutes, 1 hour, 6 hours, 12 hours, 18 hours, 24 hours, 30 hours, 36 hours, 42 hours or 48 hours. In a specific embodiment, the period of time is at least 1 hour. In a specific embodiment, the period of time is at least 6 hours. In a specific embodiment, the period of time is at least 12 hours. In a specific embodiment, the period of time is at least 18 hours. In a specific embodiment, the period of time is at least 24 hours. In a specific embodiment, the period of time is at least 30 hours. In a specific embodiment, the period of time is at least 36 hours. In a specific embodiment, the period of time is at least 42 hours. In a specific embodiment, the period of time is at least 48 hours. In a specific embodiment, the period of time is at least one of the foregoing, but not more than 48 hours. In a specific embodiment, the coated compressed core is exposed to a temperature in the range of 50-60 °C. In a specific embodiment, the curing step comprises exposing the coated compressed core to a temperature in the range of 40-70 °C for a period of time of at least 1 hour. In a specific embodiment, the curing step comprises exposing the coated compressed core to a temperature in the range of 50-60 °C for a period of time of at least 1 hour. In a specific embodiment, the curing step comprises exposing the coated compressed core to a temperature in the range of 50-60 °C for a period of time in the range of 1-48 hours. The curing step of the present invention aims at stabilizing the drug release profile on storage.

In one embodiment, provided herein is a sustained release tablet according to the invention comprising:
(a) a compressed core, wherein said compressed core comprises: (i) 4-aminopyridine; (ii) a polyethylene oxide with a molecular weight between about 1,000,000 and 10,000,000, wherein the amount of the polyethylene oxide is in the range of about 12% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core;
wherein the ratio of the amount of the ethylcellulose coat to the amount of 4-aminopyridine in the compressed core is in the range of about 1:1 to about 2:1; wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In one embodiment, provided herein is a sustained release tablet according to the invention comprising:
(a) a compressed core, wherein said compressed core comprises: (i) 4-aminopyridine; (ii) a polyethylene oxide with a molecular weight between about 1,000,000 and 10,000,000, wherein the amount of the polyethylene oxide is in the range of about 12% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core;
wherein the ratio of the amount of the ethylcellulose coat to the amount of 4-aminopyridine in the compressed core is in the range of about 0.2:1 to about 0.5:1; wherein for calculating said ratio, the amount of the ethylcellulose coat is the weight percentage of the ethylcellulose coat by weight of the compressed core, and the amount of 4-aminopyridine is the weight in milligrams of 4-aminopyridine.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In one embodiment, provided herein is a sustained release tablet according to the invention comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and
(b) an amount of an ethylcellulose coat surrounding said compressed core, said amount of the ethylcellulose coat being in the range of about 5% w/w to about 10% w/w of the compressed core.

In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes

In one embodiment, provided herein is a sustained release tablet according to the invention comprising:
(a) a compressed core, wherein said compressed core comprises: (i) 4-aminopyridine; (ii) a polyethylene oxide with a molecular weight between about 1,000,000 and 10,000,000; (iii) a mixture consisting of about 80% polyvinyl acetate, about 19% polyvinyl pyrrolidone, about 0.8% sodium lauryl sulfate, and about 0.2% of silica; (iv) dibasic calcium phosphate dihydrate; and (v) magnesium stearate; and
(b) an amount of an ethylcellulose coat surrounding said compressed core, said amount of the ethylcellulose coat being in the range of about 5% w/w to about 10% w/w of the compressed core, and
wherein the amount of 4-aminopyridine is in the range of about 4% w/w to about 6% w/w of the compressed core.

In a specific embodiment of the foregoing, the sustained release tablet is the product of a process comprising a curing step after coating the compressed core with ethylcellulose, and wherein said curing step comprises exposing the coated compressed core to a temperature in the range of 50-60 °C for a period of time of at least 1 hour

In one embodiment, provided herein is a sustained release tablet according to the invention comprising:
(a) a compressed core, wherein said compressed core comprises: (i) 4-aminopyridine, wherein the amount of 4-aminopyridine is in the range of about 4% w/w to about 6% w/w of the compressed core; (ii) a polyethylene oxide with a molecular weight between about 1,000,000 and 10,000,000, wherein the amount of the polyethylene oxide is in the range of about 12% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core, said amount of the ethylcellulose coat being in the range of about 5% w/w to about 10% w/w of the compressed core.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In a specific embodiment, the sustained release tablet according to the invention comprises:
(a) a compressed core, wherein said compressed core comprises: (i) 4-aminopyridine, wherein the amount of 4-aminopyridine is in the range of about 4% w/w to about 6% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core, (iv) dibasic calcium phosphate dihydrate in an amount of about 54% w/w of the compressed core, and (v) magnesium stearate in an amount of about 1%; and
(b) an amount of an ethylcellulose coat surrounding said compressed core, said amount of the ethylcellulose coat being about 9% w/w of the compressed core.

In a specific aspect of the foregoing embodiment, the amount of 4-aminopyridine in the compressed core is about 5% w/w of the compressed core.

In a specific embodiment of the foregoing, the sustained release tablet is the product of a process comprising a curing step after coating the compressed core with ethylcellulose, and wherein said curing step comprises exposing the coated compressed core to a temperature in the range of 50-60 °C for a period of time of at least 1 hour.

In another specific embodiment, the sustained release tablet according to the invention comprises:
(a) a compressed core, wherein said compressed core comprises: (i) 4-aminopyridine, wherein the amount of 4-aminopyridine is in the range of about 4% w/w to about 6% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core, (iv) dibasic calcium phosphate dihydrate in an amount of about 54% w/w of the compressed core, and (v) magnesium stearate in an amount of about 1%; and
(b) an amount of an ethylcellulose coat surrounding said compressed core, said amount of the ethylcellulose coat being about 5% w/w of the compressed core.

In a specific aspect of the foregoing embodiment, the amount of 4-aminopyridine in the compressed core is about 5% w/w of the compressed core.

In a specific embodiment of the foregoing, the sustained release tablet is the product of a process comprising a curing step after coating the compressed core with ethylcellulose, and wherein said curing step comprises exposing the coated compressed core to a temperature in the range of 50-60 °C for a period of time of at least 1 hour.

In one embodiment, provided herein is a sustained release tablet according to the invention comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone; and
(b) an amount of an ethylcellulose coat surrounding said compressed core, said amount of the ethylcellulose coat being in the range of about 5% w/w to about 7% w/w of the compressed core.

In a specific embodiment of the foregoing, the sustained release tablet is the product of a process comprising a curing step after coating the compressed core with ethylcellulose, and wherein said curing step comprises heating the coated compressed core to a temperature above 23 °C for a period of time of at least 15 minutes.

In one embodiment, provided herein is a sustained release tablet according to the invention comprising:
(a) a compressed core, wherein said compressed core comprises: (i) 4-aminopyridine; (ii) a polyethylene oxide with a molecular weight between about 1,000,000 and 10,000,000; (iii) a mixture consisting of about 80% polyvinyl acetate, about 19% polyvinyl pyrrolidone, about 0.8% sodium lauryl sulfate, and about 0.2% of silica; (iv) dibasic calcium phosphate dihydrate; and (v) magnesium stearate; and
(b) an amount of an ethylcellulose coat surrounding said compressed core, said amount of the ethylcellulose coat being in the range of about 5% w/w to about 7% w/w of the compressed core, and
wherein the amount of 4-aminopyridine is in the range of about 3% w/w to about 5% w/w of the compressed core.

In a specific embodiment of the foregoing, the sustained release tablet is the product of a process comprising a curing step after coating the compressed core with ethylcellulose, and wherein said curing step comprises exposing the coated compressed core to a temperature in the range of 50-60 °C for a period of time of at least 1 hour.

In one embodiment, provided herein is a sustained release tablet according to the invention comprising:
(a) a compressed core, wherein said compressed core comprises: (i) 4-aminopyridine, wherein the amount of 4-aminopyridine is about 3% w/w to about 5% w/w of the compressed core; (ii) a polyethylene oxide with a molecular weight between about 1,000,000 and 10,000,000, wherein the amount of the polyethylene oxide is in the range of about 12% w/w to about 20% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; (iv) dibasic calcium phosphate dihydrate, wherein the amount of dibasic calcium phosphate dihydrate is in the range of about 50% w/w to about 60% w/w of the compressed core; and (v) magnesium stearate, wherein the amount of magnesium stearate is in the range of about 0.7% w/w to about 1.3% w/w of the compressed core; and
(b) an amount of an ethylcellulose coat surrounding said compressed core, said amount of the ethylcellulose coat being in the range of about 5% w/w to about 7% w/w of the compressed core.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In a specific embodiment, the sustained release tablet according to the invention comprises:
(a) a compressed core, wherein said compressed core comprises: (i) 4-aminopyridine, wherein the amount of 4-aminopyridine is in the range of about 3% w/w to about 5% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core, (iv) dibasic calcium phosphate dihydrate in an amount of about 55% w/w of the compressed core, and (v) magnesium stearate in an amount of about 1%; and
(b) an amount of an ethylcellulose coat surrounding said compressed core, said amount of the ethylcellulose coat being about 6% w/w of the compressed core.

In a specific aspect of the foregoing embodiments, the amount of 4-aminopyridine in the compressed core is about 3.75% w/w of the compressed core.

In a specific embodiment of the foregoing, the sustained release tablet is the product of a process comprising a curing step after coating the compressed core with ethylcellulose, and wherein said curing step comprises exposing the coated compressed core to a temperature in the range of 50-60 °C for a period of time of at least 1 hour.

In one embodiment, the sustained release tablet provided herein is optionally coated further with an aqueous film coat containing a small quantity of 4-aminopyridine, referred to herein as the "4-AP drug overcoat" (or "immediate-release 4-AP drug overcoat"), which results in an initial burst (immediate release) of the 4-aminopyridine. This initial burst is preferably followed by a zero order or near zero order release of the drug from the remaining tablet over an extended period of time. In one embodiment, the 4-AP drug overcoat comprises 4-aminopyridine and one or more coating agents. In one embodiment, the coating agent in the 4-AP drug overcoat comprises hydroxypropylmethylcellulose. In another embodiment, the coating agent in the 4-AP drug overcoat comprises polyvinylalcohol. In a specific embodiment, the coating agent in the 4-AP drug overcoat is from OPADRY^{®} film coating system, commercially available from Colorcon. In a specific embodiment, the coating agent in the 4-AP drug overcoat comprises polyvinyl alcohol, talc, polyethylene glycol, and polysorbate 80. In one embodiment, the 4-AP drug overcoat further comprises a coloring agent. In one embodiment, the amount of 4-aminopyridine in the 4-AP drug overcoat is in the range of about 0.1% w/w to 1.5% w/w of the compressed core. In another embodiment, the amount of 4-aminopyridine in the 4-AP drug overcoat is in the range of about 0.1% w/w to about 1.25% w/w, about 0.1% w/w to about 1% w/w, about 0.1% w/w to about 0.75% w/w, about 0.1% w/w to about 0.50% w/w, about 0.1% w/w to about 0.25% w/w; about 0.25% w/w to about 1.5% w/w, about 0.25% w/w to about 1.25% w/w, about 0.25% w/w to about 1% w/w, about 0.25% w/w to about 0.75% w/w, about 0.25% w/w to about 0.5% w/w, about 0.5% w/w to about 1.5% w/w, about 0.5% w/w to about 1% w/w, about 0.5% w/w to about 0.75% w/w, about 1% w/w to about 1.5% w/w, or about 1% w/w to about 1.25% w/w of the compressed core. In a specific embodiment, the amount of 4-aminopyridine in the 4-AP drug overcoat is about 0.1% w/w, about 0.15% w/w, about 0.2% w/w, about 0.25% w/w, about 0.3% w/w, about 0.35% w/w, about 0.4% w/w, about 0.45% w/w, about 0.5% w/w, about 0.55% w/w, about 0.6% w/w, about 0.65% w/w, about 0.7% w/w, about 0.75% w/w, about 0.8% w/w, about 0.85% w/w, about 0.9% w/w, about 1% w/w, about 1.05% w/w, about 1.1% w/w, about 1.15% w/w, about 1.2% w/w, about 1.25% w/w, about 1.3% w/w, about 1.35% w/w, about 1.4% w/w, about 1.45% w/w, or about 1.5% w/w of the compressed core. In one embodiment, the amount of 4-aminopyridine in the 4-AP drug overcoat is in the range of about 0.5 mg to about 6.5 mg. In a specific embodiment, the amount of 4-aminopyridine in the 4-AP drug overcoat is in the range of about 0.5 mg to about 6 mg, about 0.5 mg to about 5 mg, about 0.5 mg to about 5 mg, about 0.5 mg to about 3 mg, about 0.5 mg to about 2 mg, about 0.5 mg to about 1 mg, about 1 mg to about 6.5 mg, about 1 mg to about 5 mg, about 1 mg to about 4 mg, about 1 mg to about 3 mg, about 1 mg to about 2 mg, about 2 mg to about 6.5 mg, about 2 mg to about 5 mg, about 2 mg to about 4 mg, about 2 mg to about 3 mg, about 3 mg to about 6.5 mg, about 3 mg to about 5 mg, about 3 mg to about 4 mg, about 4 mg to about 6.5 mg, or about 4 mg to about 5 mg. In another specific embodiment, the amount of 4-aminopyridine in the 4-AP drug overcoat is about 0.5 mg, 1 mg, 1.5 mg, 2 mg, 2.5 mg, 3 mg, 3.5 mg, 4 mg, 4.5 mg, or 5 mg. In one embodiment, the amount of the coating agent in the 4-AP drug overcoat is in the range of about 3% w/w to about 15% w/w of the compressed core. In another embodiment, the amount of the coating agent in the 4-AP drug overcoat is in the range of about 3% w/w to about 12% w/w, about 3% w/w to about 10% w/w, about 3% w/w to about 7.5% w/w, about 3% w/w to about 5% w/w, about 5% w/w to about 12% w/w, about 5% w/w to about 10% w/w, about 5% w/w to about 7.5% w/w, about 7.5% w/w to about 12% w/w, about 7.5% w/w to about 10% w/w, or about 10% w/w to about 12% w/w of the compressed core. In a specific embodiment, the amount of the coating agent in the 4-AP drug overcoat is 7.1%, 7.2%, 7.3%, 7.4%, 7.5%, 7.6%, 7.7%, 7.8%, 7.9%, 8%, 8.1%, 8.2%, 8.3%, 8.4%, 8.5%, 8.6%, 8.7%, 8.8%, 8.9%, 9%, 9.1%, 9.2%, 9.3%, 9.4%, 9.5%, 9.6%, 9.7%, 9.8%, 9.9%, 10%, 10.1%, 10.2%, 10.3%, 10.4%, 10.5%, 10.6%, 10.7%, 10.8%, 10.9%, or 11%, w/w of the compressed core. In one embodiment, the sustained release tablet provided herein is not coated with a 4-AP drug overcoat. In one embodiment, the sustained release tablet provided herein is not coated with an immediate release drug overcoat (of any drug).

In specific embodiments, the sustained release tablets described herein provide for the release of 4-aminopyridine at a sustained rate such that a therapeutically beneficial blood level of the 4-aminopyridine is maintained over a period of at least about 12 hours, preferably about 24 hours or more. In specific embodiments, the sustained release tablets described herein provide for the release of 4-aminopyridine at a sustained rate such that a therapeutically beneficial blood level of the 4-aminopyridine is maintained over a period of at least about 18 hours. Preferably, the amount of the 4-aminopyridine in the sustained release tablets of the present invention establishes a therapeutically useful plasma concentration through once daily administration of the sustained release tablet. In one embodiment, the sustained release tablet described herein releases an amount of 4-aminopyridine that is therapeutically effective over a period of 24 hours upon administration to a human patient or in physiological medium. In one embodiment, the sustained release tablet described herein releases an amount of 4-aminopyridine that is therapeutically effective over a period of 12 or 18 hours upon administration to a human patient or in physiological medium In one embodiment, the sustained release tablet is suitable for once daily oral administration; i.e., the sustained release tablet provides a therapeutically effective amount of 4-aminopyridine to a human patient upon once daily oral administration.

The sustained release tablets described herein provide sustained release of the active substance 4-aminopyridine when subjected to an in vitro dissolution test. The in vitro dissolution test can be carried out according to standard procedures published by USP NF, <711> Dissolution.

In one embodiment, the dissolution profile of the sustained release tablets described herein is determined by subjecting the sustained release tablets to an in vitro dissolution test employing 0.1 N hydrochloric acid as dissolution medium. In another embodiment, the dissolution profile of the sustained release tablets described herein is determined by subjecting the sustained release tablets to an in vitro dissolution test employing 50 mM Phosphate Buffer, pH 6.8 as dissolution medium.

In some embodiments, provided herein are sustained release tablets comprising 4-aminopyridine, wherein the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In some embodiments, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released; and (b) within the first 24 hours after the start of the test at least 80% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, provided herein are sustained release tablets comprising 4-aminopyridine, wherein the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 20% w/w of the total amount of the 4-aminopyridine contained in the sustained release tablet is released. In some embodiments, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 20% w/w of the total amount of the 4-aminopyridine contained in the sustained release tablet is released; and (b) within the first 24 hours after the start of the test at least 80% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released; (b) within the first 10 hours after the start of the test at least 50% w/w of the total amount of the 4-aminopyridine contained in the sustained release tablet is released; and (c) within the first 18 hours after the start of the test at least 75% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released; (b) within the first 10 hours after the start of the test at least 40% w/w of the total amount of the 4-aminopyridine contained in the sustained release tablet is released; and (c) within the first 24 hours after the start of the test at least 80% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released; (b) within the first 10 hours after the start of the test at least 40% w/w and at most 85% w/w of the total amount of the 4-aminopyridine contained in the sustained release tablet is released; and (c) within the first 24 hours after the start of the test at least 80% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released; (b) within the first 6 hours after the start of the test at least 10% w/w and at most 65% of the total amount of the 4-aminopyridine contained in the sustained release tablet is released; (c) within the first 10 hours after the start of the test at least 40% w/w and at most 85% w/w of the total amount of the 4-aminopyridine contained in the sustained release tablet is released; and (d) within the first 24 hours after the start of the test at least 80% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, the release of the 4-aminopyridine, upon subjecting the sustained release tablet to an in vitro dissolution test employing 50 mM Phosphate Buffer, pH 6.8 as dissolution medium and the conditions described in Table 2, is as follows: (a) within the first 2 hours after the start of the test at most 30% w/w of the total amount of the 4-aminopyridine contained in the sustained release tablet is released; (b) within the first 6 hours after the start of the test at most 60% w/w and at least 10% w/w of the total amount of the 4-aminopyridine contained in the sustained release tablet is released; (c) within the first 10 hours after the start of the test at most 80% w/w and at least 30% w/w of the total amount of the 4-aminopyridine contained in the sustained release tablet is released; and (d) within the first 24 hours after the start of the test at least 85% w/w of the total amount of the 4-aminopyridine contained in the sustained release tablet is released. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

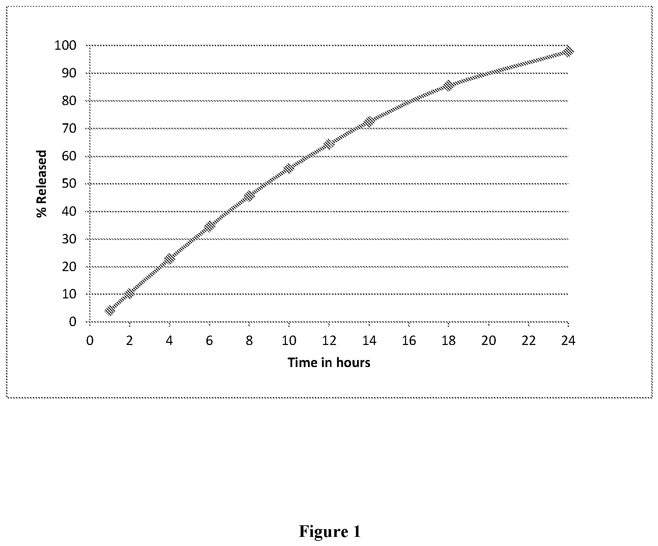

In one embodiment, the sustained release tablet described herein displays a release profile, when tested under the conditions described in Table 2, that when depicted as in Figure 1, 3, or 4 to graph % release versus time in hours is plus or minus 20% of the data plot depicted in Figure 1, 3 or 4. In a preferred embodiment, the sustained release tablet that is subjected to the dissolution test is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

The in vivo release mechanism of a drug from a sustained release tablet may be altered if the sustained release formulation is administered with alcohol, leading to dose dumping. *See,* Anand et al., 2011, AAPS J., 13(3):328-335. In a specific embodiment, the total amount of the 4-aminopyridine released, upon subjecting the sustained release tablet to an in vitro dissolution test employing 0.1 N hydrochloric acid containing 40% alcohol (ethanol) as a dissolution medium, within the first 2 hours of the in vitro dissolution test is not greater than the total amount of 4-aminopyridine released within the first 2 hours of an in vitro dissolution test employing 0.1 N hydrochloric acid as dissolution medium.

In a specific embodiment, a sustained release tablet provided herein exhibits substantially no dose dumping in the presence of food (*i.e.,* there are not clinically relevant differences in one or more pharmacokinetic parameters) between fed and fasted states upon administration to patients.

### Methods of Making

Provided herein are method of making a sustained release tablet comprising 4-aminopyridine, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 10% w/w of the compressed core; and
(c) curing the coated compressed core by exposing it to a temperature in the range of 40-70 °C for a period of time of at least 1 hour;

wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h ; or
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.
Also provided herein are methods of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core; and
(c) curing the coated compressed core by exposing it to a temperature in the range of 40-70 °C for a period of time of at least 15 minutes,
wherein the ratio of the amount of the ethylcellulose coat to the amount of 4-aminopyridine in the compressed core is in the range of about 0.5:1 to about 3:1; wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core.

In a preferred embodiment of the foregoing embodiment, the ratio of the amount of the ethylcellulose coat to 4-aminopyridine is in the range of about 1:1 to about 2:1.

Also, provided herein are methods of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core; and
(c) curing the coated compressed core by exposing it to a temperature in the range of 40-70 °C for a period of time of at least 15 minutes,
wherein the ratio of the amount of the ethylcellulose coat to the amount of 4-aminopyridine in the compressed core is in the range of about 0.1:1 to about 0.7:1; wherein for calculating said ratio, the amount of the ethylcellulose coat is the weight percentage of the ethylcellulose coat by weight of the compressed core, and the amount of 4-aminopyridine is the weight in milligrams of 4-aminopyridine.

In a preferred embodiment of the foregoing embodiment, the ratio of the amount of the ethylcellulose coat to 4-aminopyridine is in the range of about 0.2:1 to about 0.5:1.

Also, provided herein are methods of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 8% w/w to about 10% w/w of the sustained release tablet; and
(c) curing the coated compressed core by heating the coated compressed core to a temperature above 23 °C for a period of time of at least 15 minutes.

Also, provided herein is a method of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 10% w/w of the compressed core; and
(c) curing the coated compressed core by exposing it to a temperature in the range of 40-70 °C for a period of time of at least 15 minutes.

In a specific embodiment of the foregoing method of making, the amount of the ethylcellulose coat is about 9% w/w of the compressed core and the amount of 4-aminopyridine is in the range of about 4% w/w to about 6% w/w of the compressed core. In another specific embodiment of the foregoing method of making, the amount of the ethylcellulose coat is about 5% w/w of the compressed core and the amount of 4-aminopyridine is in the range of about 4% w/w to about 6% w/w of the compressed core.

Also, provided herein is a method of making a sustained release tablet comprising 4-aminopyridine according to the invention, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 7% w/w of the compressed core; and
(c) curing the coated compressed core by exposing it to a temperature in the range of 40-70 °C for a period of time of at least 15 minutes.

In a specific embodiment of the foregoing methods of making, the amount of the ethylcellulose coat is about 6% w/w of the compressed core and the amount of 4-aminopyridine is in the range of about 3% w/w to about 5% w/w of the compressed core.

In a specific embodiment, the curing step comprises exposing the coated compressed core to a temperature in the range of 40-70 °C for a period of time of at least 15 minutes, 30 minutes or 1 hour. In a specific embodiment, the curing step comprises exposing the coated compressed core to a temperature in the range of 40-70 °C for a period of time of at least 1 hour.

Also provided herein is a method of making a sustained release tablet comprising 4-aminopyridine according to the invention, which method comprises:
(a) forming a compressed core comprising (i) 4-aminopyridine, wherein the amount of 4-aminopyridine is in the range of about 4% w/w to about 6% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 9% w/w of the compressed core; and
(c) curing the coated compressed core by exposing it to a temperature in the range of 50-60 °C for a period of time of at least 1 hour.

In a specific embodiment of the foregoing embodiment, the amount of 4-aminopyridine in the compressed core is about 22 mg.

Also provided herein is a method of making a sustained release tablet comprising 4-aminopyridine according to the invention, which method comprises:
(a) forming a compressed core comprising (i) 4-aminopyridine, wherein the amount of 4-aminopyridine is in the range of about 4% w/w to about 6% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 5% w/w of the compressed core; and
(c) curing the coated compressed core by exposing it to a temperature in the range of 50-60 °C for a period of time of at least 1 hour.

In a specific embodiment of the foregoing embodiment, the amount of 4-aminopyridine in the compressed core is about 22 mg.

Also provided herein is a method of making a sustained release tablet comprising 4-aminopyridine according to the invention, which method comprises:
(a) forming a compressed core comprising (i) 4-aminopyridine, wherein the amount of 4-aminopyridine is in the range of about 3% w/w to about 5% w/w of the compressed core, (ii) a polyethylene oxide with a molecular weight of 7,000,000, wherein the amount of the polyethylene oxide is about 15% w/w of the compressed core, (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core;
(b) coating the compressed core with an amount of ethylcellulose to form a coated compressed core, said amount of the ethylcellulose coat being about 6% w/w of the compressed core; and
(c) curing the coated compressed core by exposing it to a temperature in the range of 50-60 °C for a period of time of at least 1 hour.

In a specific embodiment of the foregoing embodiment, the amount of 4-aminopyridine in the compressed core is about 16.5 mg.

In specific embodiments, the methods of making a sustained release tablet comprise, or consist essentially, or consist of, the steps as described herein.

The core is typically formed by first blending the active drug substance (4-aminopyridine), the polymeric components of the formulation, and additional excipients (e.g., filler or binder) for a brief period of time (e.g., 5-20 minutes). This is followed by addition of a suitable lubricant and further blending the mixture to a uniform new blend. The resulting blend is then compressed to intermediate tablets by methods known in the art.

In a specific embodiment, the method of forming the compressed core comprises:
(a) blending the 4-aminopyridine, polyethylene oxide, the mixture comprising polyvinyl acetate and polyvinyl pyrrolidone, and dibasic calcium phosphate dihydrate to form a blended mixture;
(b) adding magnesium stearate to the blended mixture to form a new blend; and
(c) compressing the new blend to form a compressed core. In a specific embodiment, the blended mixture and new blend are formed by dry blending.

In a specific embodiment, the polyethylene oxide has a molecular weight between 4,000,000 and 8,000,000. In another specific embodiment, the polyethylene oxide has a molecular weight of 7,000,000. In another specific embodiment, the polyethylene oxide is POLYOX WSR-303, commercially available from Dow Chemical Co. In a specific embodiment, the amount of the polyethylene oxide in the compressed core is in the range of about 10% w/w to about 20% w/w of the compressed core.

In a specific embodiment, the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% of silica. In one embodiment, the mixture comprising polyvinyl acetate and polyvinyl pyrrolidone is KOLLIDON^{®} SR polyvinyl acetate/polyvinyl pyrrolidone (BASF); KOLLIDON^{®} SR is a blend of 80% polyvinyl acetate and 19% povidone (also called polyvinyl pyrrolidone) along with small quantities of sodium lauryl sulfate and silica used as stabilizers (see Signet Chemical Corporation's website page for KOLLIDON^{®} SR). In a specific embodiment, the amount of the mixture comprising polyvinyl acetate and polyvinyl pyrrolidone in the compressed core is in the range of about 20% w/w to about 30% w/w of the compressed core.

In a specific embodiment, the amount of dibasic calcium phosphate dihydrate in the compressed core is in the range of about 47% w/w to about 53% w/w of the sustained release tablet. In another specific embodiment, the amount of dibasic calcium phosphate dihydrate in the compressed core is in the range of about 50% w/w to about 60% w/w of the compressed core.

In a specific embodiment, the amount of magnesium stearate in the compressed core is in the range of about 0.7% w/w to about 1.3% w/w of the sustained release tablet. In another specific embodiment, the amount of magnesium stearate in the compressed core is in the range of about 0.7% w/w to about 1.3% w/w of the compressed core.

The coating of the compressed core (i.e., intermediate tablets) is performed by using methods well known in the art, as described in US Patent 8,858,993. In a specific embodiment, the coating process described herein includes film coating. Tablet coating equipment may include spray guns, coating pans, polishing pans, solution tanks, blenders and mixers, homogenizers, mills, peristaltic pumps, fans, steam jackets, exhaust and heating pipes, scales and filters. In a specific embodiment, the coating method involves spray coating.

As described in US Patent 8,858,993, tablet coating typically takes place in a controlled atmosphere inside a perforated rotating drum. Angled baffles fitted into the drum and air flow inside the drum can provide means of mixing the tablet bed. As a result, the tablets are lifted and turned from the sides into the center of the drum, exposing each tablet surface to an even amount of deposited/sprayed coating. The liquid spray coating is then dried onto the tablets by heated air drawn through the tablet bed from an inlet fan. The air flow is typically regulated for temperature and volume to provide controlled drying and extracting rates, and at the same time, maintaining the drum pressure slightly negative relative to the room in order to provide a completely isolated process atmosphere for the operator.

In a specific embodiment, the coating step is performed using the parameters described in Example 7.

In some embodiments, the compressed core (i.e., the intermediate core tablet) described herein is optionally coated with a moisture barrier coat (e.g., an OPADRY^{®} Clear coat) prior to coating with the release modifying coating agent (e.g., SURELEASE^{®}). In a specific embodiment, the sustained release 4-aminopyridine tablet comprising a moisture barrier coat (e.g., an OPADRY^{®} Clear coat) and a release modifying coating agent (e.g., SURELEASE^{®}) is prepared as described in Example 8.

In a specific embodiment, the coated compressed core is subjected to a post coating thermal treatment, also referred to as a curing step. In a specific embodiment, the curing step comprises heating the coated compressed core to a temperature above 23 °C for a period of time of at least 15 minutes. In a specific embodiment, the curing step comprises exposing the coated compressed core to a temperature in the range of 40-70 °C for a period of time of at least 15 minutes, 30 minutes, 1 hour, 6 hours, 12 hours, 18 hours, 24 hours, 30 hours, 36 hours, 42 hours or 48 hours. In a specific embodiment, the period of time is at least 1 hour. In a specific embodiment, the period of time is at least 6 hours. In a specific embodiment, the period of time is at least 12 hours. In a specific embodiment, the period of time is at least 18 hours. In a specific embodiment, the period of time is at least 24 hours. In a specific embodiment, the period of time is at least 30 hours. In a specific embodiment, the period of time is at least 36 hours. In a specific embodiment, the period of time is at least 42 hours. In a specific embodiment, the period of time is at least 48 hours. In a specific embodiment, the coated compressed core is exposed to a temperature in the range of 50-60 °C. In a specific embodiment, the curing step comprises exposing the coated compressed core to a temperature in the range of 40-70 °C for a period of time of at least 1 hour. In a specific embodiment, the curing step comprises exposing the coated compressed core to a temperature in the range of 50-60 °C for a period of time of at least 1 hour. In a specific embodiment, the curing step comprises exposing the coated compressed core to a temperature in the range of 50-60 °C for a period of time in the range of 1-48 hours. The curing step of the present invention aims at stabilizing the drug release profile on storage.

In one embodiment, after the curing step the sustained release tablet is optionally coated further with an aqueous film coat containing a small quantity of 4-aminopyridine, referred to herein as the "4-AP drug overcoat" (or "immediate release 4-AP drug overcoat"), which results in an initial burst (immediate release) of the 4-aminopyridine. This initial burst is preferably followed by a zero order or near zero order release of the drug from the remaining tablet over an extended period of time. In one embodiment, the 4-AP drug overcoat comprises 4-aminopyridine and one or more coating agents. In one embodiment, the coating agent in the 4-AP drug overcoat comprises hydroxypropylmethylcellulose. In another embodiment, the coating agent in the 4-AP drug overcoat comprises polyvinylalcohol. In a specific embodiment, the coating agent in the 4-AP drug overcoat is from OPADRY^{®} film coating system, commercially available from Colorcon. In a specific embodiment, the coating agent in the 4-AP drug overcoat comprises polyvinyl alcohol, talc, polyethylene glycol, and polysorbate 80. In one embodiment, the 4-AP drug overcoat further comprises a coloring agent. A solution of the 4-AP drug overcoat is typically prepared by dissolving a coating agent in a suitable solvent (e.g., water), followed by adding 4-aminopyridine and stirring to obtain a clear solution. The solution of the 4-AP drug overcoat can be applied to the dried coated compressed cores by methods well known in the art to achieve the desired coat weight gain. In one embodiment, the solution is applied by spray coating. After the 4-AP drug overcoat is applied, the resulting tablets can be dried, e.g., for up to 1 h at a temperature of about 50-60 °C.

In a specific embodiment the sustained release tablet of the present invention, aside from any immediate release of 4-aminopyridine due to the presence of a 4-AP drug overcoat, provides a zero-order or near-zero-order release of the 4-aminopyridine. In a preferred embodiment, the release is zero-order.

In specific embodiments, the sustained release tablets discussed herein provide for the release of 4-aminopyridine at a sustained rate such that a therapeutically beneficial blood level of the 4-aminopyridine is maintained over a period of at least about 12 hours, preferably about 24 hours or more. In another specific embodiment, the sustained release tablets discussed herein provide for the release of 4-aminopyridine at a sustained rate such that a therapeutically beneficial blood level of the 4-aminopyridine is maintained over a period of at least about 18 hours. Preferably, the amount of the 4-aminopyridine in the sustained release tablets of the present invention establish a therapeutically useful plasma concentration through once daily administration of the sustained release tablet. In one embodiment, the sustained release tablet described herein comprises an amount of 4-aminopyridine that is therapeutically effective over a period of 24 hours. In one embodiment, the sustained release tablet is suitable for once daily oral administration; i.e., the sustained release tablet provides a therapeutically effective of 4-aminopyridine to a human patient upon once daily oral administration.

As used herein, and unless otherwise indicated, the term "about" or "approximately" means an acceptable error for a particular value as determined by one of ordinary skill in the art, which depends in part on how the value is measured or determined. In certain embodiments, the term "about" or "approximately" means within 1, 2, or 3 standard deviations. In certain embodiments, the term "about" or "approximately" means within 10%, 9%, 8%, 7%, 6%, 5%, 4%, 3%, 2%, 1%, 0.5%, 0.4%, 0.3%, 0.25%, 0.2%, 0.1% or 0.05% of a given value or range.

### 5.2 Methods of Treatment

The invention also provides sustained release tablets according to the invention for use in methods of treating a neurological disorder in a patient in need thereof comprising orally administering to the patient once daily the sustained release 4-aminopyridine tablet as disclosed herein.

In one embodiment, provided herein are sustained release tablets according to the invention for use in methods of treating a neurological disorder in a patient in need thereof comprising orally administering to the patient once daily a sustained release tablet according to the invention comprising:
(a) a compressed core, wherein said compressed core comprises (i) 4-aminopyridine, wherein the amount of 4-aminopyridine is in the range of about 4% w/w to about 6% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 9% w/w of the compressed core.

In one embodiment of the foregoing embodiment, the amount of 4-aminopyridine in the compressed core is about 17.5 mg, about 18 mg, about 18.5 mg, about 19 mg, about 19.5 mg, about 20 mg, about 20.5 mg, about 21 mg, about 21.5 mg, about 22 mg. In a specific embodiment of the foregoing embodiment, the amount of 4-aminopyridine in the compressed core is about 22 mg.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In one embodiment, provided herein are sustained release tablets according to the invention for use in methods of treating a neurological disorder in a patient in need thereof comprising orally administering to the patient once daily a sustained release tablet according to the invention comprising:
(a) a compressed core, wherein said compressed core comprises (i) 4-aminopyridine, wherein the amount of 4-aminopyridine is in the range of about 4% w/w to about 6% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 54% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 5% w/w of the compressed core.

In one embodiment of the foregoing embodiment, the amount of 4-aminopyridine in the compressed core is about 17.5 mg, about 18 mg, about 18.5 mg, about 19 mg, about 19.5 mg, about 20 mg, about 20.5 mg, about 21 mg, about 21.5 mg, about 22 mg. In a specific embodiment of the foregoing embodiment, the amount of 4-aminopyridine in the compressed core is about 22 mg.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

In one embodiment, provided herein are sustained release tablets according to the invention for use in methods of treating a neurological disorder in a patient in need thereof comprising orally administering to the patient once daily a sustained release tablet according to the invention comprising:
(a) a compressed core, wherein said compressed core comprises (i) 4-aminopyridine, wherein the amount of 4-aminopyridine is in the range of about 3% w/w to about 5% w/w of the compressed core; (ii) a polyethylene oxide with a molecular weight of 7,000,000, wherein the amount of the polyethylene oxide is about 15% w/w of the compressed core; (iii) a mixture consisting of about 80% polyvinyl acetate, about 19% polyvinyl pyrrolidone, about 0.8% sodium lauryl sulfate, and about 0.2% of silica, wherein the amount of the mixture is about 25% w/w of the compressed core; (iv) dibasic calcium phosphate dihydrate, wherein the amount of dibasic calcium phosphate dihydrate is about 55% w/w of the compressed core; and (v) magnesium stearate, wherein the amount of magnesium stearate is about 1% w/w of the compressed core; and
(b) an amount of an ethylcellulose coat surrounding said compressed core, said amount of the ethylcellulose coat being about 6% w/w of the compressed core.

In one embodiment of the foregoing embodiment, the amount of 4-aminopyridine in the compressed core is about 13 mg, about 13.5 mg, about 14 mg, about 14.5 mg, about 15 mg, about 15.5 mg, about 16 mg, about 16.5 mg, about 17 mg, about 17.5 mg, about 18 mg, about 18.5 mg, about 19 mg, about 19.5 mg, about 20 mg, about 20.5 mg, about 21 mg, about 21.5 mg or about 22 mg. In a specific embodiment of the foregoing embodiment, the amount of 4-aminopyridine in the compressed core is about 16.5 mg.

In a specific embodiment of the foregoing, the sustained release tablet is the product of a process comprising a curing step after coating the compressed core with ethylcellulose, wherein said curing step comprises exposing the coated compressed core to a temperature in the range of 50-60 °C for a period of time of at least 1 hour.

"Patient" is defined herein to include animals, such as mammals, including, but not limited to, primates (e.g., humans), cows, sheep, goats, horses, dogs, cats, rabbits, rats, mice, monkeys, chickens, turkeys, quails, or guinea pigs and the like. In one embodiment, the patient is a mammal. In a preferred embodiment, the patient is a human patient.

In a specific embodiment, the human patient is an adult (e.g., greater than 16 years of age). In another specific embodiment, the patient is a pediatric patient (e.g., age 2-16). In one embodiment, the age of the pediatric patient is at least 6 years. In another specific embodiment, the human patient is an adolescent (e.g., age 12-16). In another specific embodiment, the patient is a child (e.g. age 2-12). In one embodiment, the age of the child is at least 6 years.

In one embodiment, the administering to the patient is performed once daily. In another embodiment, the administering to the patient is performed twice daily.

The neurological disorder is a neurological disorder amenable to treatment by 4-aminopyridine. In certain embodiments, the neurological disorder is a neurological disease including, but not limited to, multiple sclerosis and stroke. In a specific embodiment, the neurological disorder is multiple sclerosis. In another specific embodiment, the neurological disorder is stroke. In some embodiments, the neurological disorder is an impairment associated with a neurological disease. In one embodiment, the neurological disorder is a walking impairment associated with multiple sclerosis. In a specific embodiment, the sustained release tablet according to the invention is for use in a method of treatment, wherein the method of treatment is to improve walking or to increase walking speed in a multiple sclerosis patient. In a specific embodiment, the method of treatment is to improve walking or to increase walking speed in a stroke patient. In one embodiment, the neurological disorder is a neurocognitive or a neuropsychiatric impairment associated with multiple sclerosis. In one embodiment, the neurological disorder is a sensorimotor impairment associated with stroke. In one embodiment, the neurological disorder is a walking impairment associated with stroke.

In a specific embodiment, the invention provides a sustained release tablet according to the invention for use in a method of improving walking comprising administering once daily to a patient (e.g., a multiple sclerosis patient) a sustained release tablet provided herein.

In one embodiment, the sustained release 4-aminopyridine tablet as disclosed herein is orally administered to a patient with renal impairment, for example, a patient who is mildly or moderately renally impaired. In one embodiment, the renal impairment is mild renal impairment. In one embodiment, the renal impairment is moderate renal impairment. In one embodiment, the renal impairment is severe renal impairment. In one embodiment, the renal impairment is moderate or severe renal impairment. In another embodiment, the sustained release 4-aminopyridine tablet is orally administered to a patient who is not severely renal impaired. In one embodiment, the patient is classified as having mild renal impairment when the patient has a creatinine clearance value in the range of 51-80 mL/min. In another embodiment, the patient is classified as having mild renal impairment when the patient has a creatinine clearance value in the range of 60-89 mL/min. In yet another embodiment, the patient is classified as having mild renal impairment when the patient has an estimated glomerular filtration rate in the range of 60-89 mL/min/1.73m². In one embodiment, the patient is classified as having moderate renal impairment when the patient has a creatinine clearance value in the range of 30-59 mL/min. In yet another embodiment, the patient is classified as having moderate renal impairment when the patient has an estimated glomerular filtration rate in the range of 30-59 mL/min/1.73m². In one embodiment, the patient is classified as having severe renal impairment when the patient has a creatinine clearance value in the range of 15-29 mL/min. In yet another embodiment, the patient is classified as having severe renal impairment when the patient has an estimated glomerular filtration rate in the range of 15-29 mL/min/1.73m².

In a specific embodiment, the sustained release tablet as disclosed herein is orally administered once daily to an adult or adolescent patient, wherein the amount of 4-aminopyridine in the compressed core is in the range of about 5 mg to about 20 mg, about 5 mg to about 15 mg, about 5 mg to about 10 mg, about 10 mg to about 20 mg, about 10 mg to about 15 mg, about 15 mg to about 20 mg. In another specific embodiment of the foregoing, the amount of 4-aminopyridine in the compressed core is about 16 mg, about 16.2 mg, about 16.4 mg, about 16.6 mg, about 16.8 mg, about 17 mg, about 17.2 mg, about 17.4 mg, about 17.6 mg, about 17.8 mg, about 18 mg, about 18.2 mg, about 18.4 mg, about 18.6 mg, about 18.8 mg, about 19 mg, about 19.2 mg, about 19.4 mg, about 19.6 mg, about 19.8 mg, about 20 mg, about 20.2 mg, about 20.4 mg, about 20.6 mg, about 20.8 mg, about 21 mg, about 21.2 mg, about 21.4 mg, about 21.6 mg, about 21.8 mg, about 22 mg. In another embodiment of the foregoing, the amount of 4-aminopyridine in the compressed core is in the range of about 12 mg to about 15 mg. In another embodiment of the foregoing, the amount of 4-aminopyridine in the compressed core is in the range of about 5 mg to about 12 mg. In a specific embodiment of the foregoing, the amount of 4-aminopyridine in the compressed core is about 22 mg. In another specific embodiment of the foregoing, the amount of 4-aminopyridine in the compressed core is about 16.5 mg. In another specific embodiment of the foregoing, the amount of 4-aminopyridine in the compressed core is about 11 mg. In a specific embodiment, the sustained release tablet as disclosed herein is orally administered once daily to an adult patient, wherein the compressed core of the sustained release tablet comprises 4-aminopyridine in an amount in the range of about 16 mg to about 22 mg. In another specific embodiment, the sustained release tablet as disclosed herein is orally administered once daily to an adolescent patient, wherein the compressed core of the sustained release tablet comprises 4-aminopyridine in an amount in the range of about 12 mg to about 22 mg. In another specific embodiment, the sustained release tablet as disclosed herein is orally administered once daily to a child patient of age 6-12, wherein the compressed core of the sustained release tablet comprises 4-aminopyridine in an amount in the range of about 4 mg to about 13 mg.

In a specific embodiment, the sustained release tablet as disclosed herein is orally administered once daily to a pediatric patient, wherein the compressed core of the sustained release tablet comprises 4-aminopyridine in an amount in the range of about 4 mg to about 13 mg. In a specific embodiment of the foregoing, the amount of 4-aminopyridine in the compressed core is in the range of about 4 mg to about 11 mg, about 4 mg to about 9 mg, about 4 mg to about 7 mg, about 4 mg to about 5 mg, about 5 mg to about 13 mg, about 5 mg to about 11 mg, about 5 mg to about 9 mg, about 5 mg to about 7 mg, about 7 mg, to about 13 mg, about 7 mg to about 11 mg, about 7 mg to about 9 mg, about 9 mg to about 13 mg, about 9 mg to about 11 mg, or about 11 mg to about 13 mg. In another specific embodiment of the foregoing, the amount of 4-aminopyridine in the compressed core is about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, or about 12 mg. In another specific embodiment of the foregoing, the amount of 4-aminopyridine in the compressed core is about 11 mg. In another specific embodiment of the foregoing, the amount of 4-aminopyridine in the compressed core is about 8 mg. In one embodiment, the pediatric dose is half of the adult dose. For example, if the amount of 4-aminopyridine in the compressed core orally administered once daily to an adult patient is about 22 mg, the amount of 4-aminopyridine in the compressed core orally administered to a pediatric patient is about 11 mg.

In one embodiment, the sustained release tablet as disclosed herein is orally administered once daily to a patient with renal impairment, wherein the compressed core of the sustained release tablet comprises 4-aminopyridine in an amount in the range of about 4 mg to about 20 mg. In a specific embodiment of the foregoing, the amount of 4-aminopyridine in the compressed core is about 6 mg to about 20 mg, about 6 mg to about 16 mg, about 6 mg to about 12 mg, about 6 mg to about 18 mg, about 8 mg to about 20 mg, about 8 mg to about 16 mg, about 8 mg to about 12 mg, about 10 mg to about 20 mg, about 10 mg to about 16 mg, about 10 mg to about 12 mg, about 12 mg to about 20 mg, about 12 mg to about 16 mg, about 14 mg to about 20 mg, about 14 mg to about 16 mg, or about 16 mg to about 20 mg. In a specific embodiment of the foregoing, the amount of 4-aminopyridine in the compressed core is about 7 mg to about 9 mg. In another specific embodiment of the foregoing, the amount of 4-aminopyridine in the compressed core is about 11 mg to about 13 mg. In another specific embodiment of the foregoing, the amount of 4-aminopyridine in the compressed core is about 12 mg to about 15 mg. In another specific embodiment of the foregoing, the amount of 4-aminopyridine in the compressed core is about 15 mg to about 17 mg. In another specific embodiment of the foregoing, the amount of 4-aminopyridine in the compressed core is about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg or about 17 mg. In a specific embodiment, the sustained release tablet of any of the foregoing embodiments is orally administered once daily to a patient with mild or moderate renal impairment.

In one embodiment, the sustained release 4-aminopyridine tablet as disclosed herein is orally administered once daily to a patient with no renal impairment. In one embodiment, the patient is classified as having no renal impairment when the patient has an estimated glomerular filtration rate of ≥ 90 mL/min/1.73m². In one embodiment, the patient is classified as having no renal impairment when the patient has a creatinine clearance value of ≥ 90 mL/min.

Preferably, a sustained release 4-aminopyridine tablet as disclosed herein, upon oral administration to human subjects does not result in substantial dose dumping in the presence of food. In one embodiment, the sustained release 4-aminopyridine tablet, upon oral administration to human subjects does not result in clinically relevant differences in pharmacokinetic parameters such as AUC or Cₘₐₓ between the fed or fasted conditions when administered to human subjects in fed and fasted conditions. In one embodiment, a human subject having consumed a high-fat high-calorie meal before oral administration (as described in Guidance for Industry, Food-Effect Bioavailability and Fed Bioequivalence Studies, U.S. Department of Health and Human Services, Food and Drug Administration, December 2002) is considered to be in a fed state. In one embodiment, a human subject observing an overnight fast of at least 10 h is considered to be in a fasted state.

In a specific embodiment, a sustained release 4-aminopyridine tablet according to the invention as disclosed herein, upon once daily oral administration to human subjects in a fasted state, provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h; wherein the amount of 4-aminopyridine in the sustained release tablet is about 16.5 mg. In one embodiment, administering the 4-aminopyridine tablet to a human subject in a fasted state is equivalent to administering the 4-aminopyridine tablet to the human subject in the morning, wherein a standard meal is consumed not before, and no earlier than 4 hours after dose administration.

In a specific embodiment, once daily oral administration of a sustained release 16.5 mg 4-aminopyridine tablet of the invention to human subjects in a fasted state provides the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h. In a specific embodiment, once daily oral administration of a sustained release 16.5 mg 4-aminopyridine tablet of the invention to human subjects in a fasted state provides the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, and (c) a median steady state plasma 4-aminopyridine Tₘₐₓ in the range of about 3 h to about 12 h. In a specific embodiment, once daily oral administration of a sustained release 16.5 mg 4-aminopyridine tablet of the invention to human subjects in a fasted state provides a mean steady state plasma 4-aminopyridine Cₘₐₓ in the range of about 13.96 ng/mL to about 27.89 ng/mL. In a specific embodiment, once daily oral administration of a sustained release 16.5 mg 4-aminopyridine tablet of the invention to human subjects in a fasted state provides a mean steady state plasma 4-aminopyridine Cₘᵢₙ in the range of about 5.80 ng/mL to about 14.60 ng/mL. In a specific embodiment, once daily oral administration of a sustained release 16.5 mg 4-aminopyridine tablet of the invention to human subjects in a fasted state provides a median steady state plasma 4-aminopyridine Tₘₐₓ in the range of about 3 h to about 12 h. In one embodiment, once daily oral administration of a sustained release 16.5 mg 4-aminopyridine tablet of the invention to human subjects in a fasted state provides a median steady state plasma 4-aminopyridine Tₘₐₓ of about 6 h.

AUC₀₋₂₄ is the area under the curve for 0 - 24 h. In a preferred embodiment, the sustained release tablet which is characterized by the foregoing pharmacokinetic parameters is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In a specific embodiment, a sustained release 4-aminopyridine tablet as disclosed herein, upon once daily oral administration to human subjects in a fasted state, provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h; wherein the amount of 4-aminopyridine in the sustained release tablet is about 22 mg. In one embodiment, administering the 4-aminopyridine tablet to a human subject in a fasted state is equivalent to administering the 4-aminopyridine tablet to the human subject in the morning, wherein a standard meal is consumed not before, and no earlier than 4 hours after dose administration.

In a specific embodiment, once daily oral administration of a sustained release 22 mg 4-aminopyridine tablet of the invention to human subjects in a fasted state provides the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h. In a specific embodiment, once daily oral administration of a sustained release 22 mg 4-aminopyridine tablet of the invention to human subjects in a fasted state provides the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, and (c) a median steady state plasma 4-aminopyridine Tₘₐₓ in the range of about 3 h to about 12 h. In a specific embodiment, once daily oral administration of a sustained release 22 mg 4-aminopyridine tablet of the invention to human subjects in a fasted state provides a mean steady state plasma 4-aminopyridine Cₘₐₓ in the range of about 18.61 ng/mL to about 37.19 ng/mL. In a specific embodiment, once daily oral administration of a sustained release 22 mg 4-aminopyridine tablet of the invention to human subjects in a fasted state provides a mean steady state plasma 4-aminopyridine Cₘᵢₙ in the range of about 7.73 ng/mL to about 19.47 ng/mL. In a specific embodiment, once daily oral administration of a sustained release 22 mg 4-aminopyridine tablet of the invention to human subjects in a fasted state provides a median steady state plasma 4-aminopyridine Tₘₐₓ in the range of about 3 h to about 12 h. In a specific embodiment, once daily oral administration of a sustained release 22 mg 4-aminopyridine tablet of the invention to human subjects in a fasted state provides a median steady state plasma 4-aminopyridine Tₘₐₓ of about 6 h.

AUC₀₋₂₄ is the area under the curve for 0 - 24 h. In a preferred embodiment, the sustained release tablet which is characterized by the foregoing pharmacokinetic parameters is a tablet in which 4-aminopyridine is present only in the compressed core (which tablet can be the final oral dosage form of a tablet that does not have an immediate release drug overcoat, or can be the tablet that has not yet been coated with the drug overcoat in an embodiment where the final oral dosage form will have a drug overcoat).

In one embodiment, a sustained release 4-aminopyridine tablet as disclosed herein, upon once daily oral administration to human subjects provides one or more of the pharmacokinetic parameters having the values described in Example 6. In a specific embodiment of the foregoing, the amount of 4-aminopyridine in the sustained release tablet is about 22 mg. In a specific embodiment of the foregoing, the amount of 4-aminopyridine in the sustained release tablet is about 16.5 mg.

### 5.3 Combination Therapy

In certain embodiments, the sustained release 4-aminopyridine tablet as disclosed herein may be orally administered in combination with one or more additional medicaments. Such combination therapy may be achieved by way of the simultaneous, sequential, or separate dosing of the individual components of the treatment. In one embodiment, the combination therapy is achieved by way of separate dosing of the sustained release 4-aminopyridine tablet and the other medicament.

In certain embodiments, the other medicament is a therapeutic agent effective for the treatment of multiple sclerosis. In specific embodiments, the other medicament is selected from a group consisting of, but not limited to, AVONEX (interferon beta-1a), REBIF (interferon beta-1a), BETASERON and EXTAVIA (interferon beta-1b), COPAXONE (glatiramer acetate), NOVANTRONE (mitoxantrone), TYSABRI (natalizumab), AUBAGIO (teriflunomide), GILENYA (fingolimod), LEMTRADA (alemtuzumab), PLEGRIDY (peginterferon beta-1a), TECFIDERA (dimethyl fumarate), ocrelizumab, prednisone, prednisolone, methylprednisolone, betamethasone, and dexamethasone.

In certain embodiments, the other medicament is a therapeutic agent intended for the prophylaxis of stroke. In specific embodiments, the other medicament is selected from the group consisting of, but not limited to, anti-coagulant agents (e.g. aspirin, clopidogrel, etc.) and antihypertensive agents.

### 6 EXAMPLES

Certain embodiments provided herein are illustrated by the following non-limiting examples.

### 6.1 Example 1: Preparation of Sustained Release 4-Aminopyridine Tablets

### 6.1.1 Dispensing, Sifting and Blending

4-Aminopyridine (110.0 g), Kollidon SR Polymer (550.0 g), Polyethylene Oxide, NF (Sentry Polyox WSR 303) (330.0 g), and Dibasic Calcium Phosphate Dihydrate, USP/NF (1188 g) were sifted through a 595 µm (30 mesh) hand screen into a container double-lined with polyethylene bags. The sifted powders obtained above were placed in a 7.57 liter (8 quart) V-Blender, and blended for 10 minutes. To this blended mixture was added Magnesium Stearate, NF Non-Bovine Hyqual (22.0 g) that was sifted through a 841 µm (20 mesh) hand screen. The entire mixture was blended for additional 5 minutes, and then the blend was discharged into a container lined with two polyethylene bags after performing content uniformity and physical testing on the final blend.

### 6.1.2 Tableting

The completed blend from above was fed into a tablet press hopper. A rotary tablet press (power assisted) was set up with a tablet deduster and metal detector in-line. Batch set-up parameters were then determined to achieve tablets a target weight of 440 mg (individual range 407-473 mg).

The blend was then compressed using a Compact-19 Tablet Press with Tooling Set No. 138 to furnish the desired core tablets, while performing checks on individual tablet weight, mean tablet weight, hardness, thickness, appearance and friability at defined times during the compression run. The weight of the desired tablets was within +/- 7.5% of theoretical tablet weight of 440 mg (i.e., within 407.0 - 473.0 mg).

### 6.1.3 Coating and Curing

Purified Water (686.0 g) was weighed into a suitable solution tank. The Purified Water was mixed with Ethylcellulose Dispersion Type B, NF (Surelease E-7-19040, Clear) (1030 g) while stirring using an overhead mixer with a suitable impeller. Mixing was continued for at least 15 minutes with low to moderate agitation, while ensuring there are no visual signs for any lumps, foam, settling, etc. prior to coating.

The core tablets prepared in Section 0 were loaded into a LabCoat MX tablet coater equipped with a 15" pan, a peristaltic pump with new tubing (Maserflex Pt/Si Size 14) and a single spray gun. The tablets were pre-warm for approximately 10 minutes by jogging the pan with the fan and heater on at 50 °C. After setting the Supply Temperature to target 60 °C, the above prepared film-coat was applied in a controlled manner to the pre-warmed core tablets in the Labcoat MX to approximately 9% weight gain (i.e., 39.6 mg). The coated tablets thus formed were then subjected to a curing step by drying them at 55 °C for 1 hour and then allowed to cool for at least 5 minutes to a target exhaust temperature of approximately 25-30 °C while jogging the pan. The tablets were then discharged into a suitable container lined with two polyethylene bags to afford the final coated tablets.

Table 1 provides the amounts and weight percentages of ingredients present in the sustained release tablet prepared using the method described above.

**Table 1. Composition of sustained release tablets containing 22 mg 4-aminopyridine and 9% ethylcellulose coat by weight of the compressed core.**

| **Ingredient** | **% w/w of compressed core** | **Amount per tablet (mg)** |
|---|---|---|
| **Compressed Core** | | |
| 4-aminopyridine | 5.0 | 22.0 |
| KOLLIDON^{®} SR | 25.0 | 110.0 |
| Polyethylene oxide (Sentry Polyox WSR 303) | 15.0 | 66.0 |
| Dibasic calcium phosphate dihydrate | 54.0 | 237.6 |
| Magnesium stearate | 1.0 | 4.4 |
| **Weight of Compressed Core** | 100 | 440 |

| **Functional Coating** | | |
|---|---|---|
| Ethylcellulose Dispersion Type B | 9 | 39.6 |

Thus, for the sustained release tablet of Example 1, containing 22 mg 4-aminopyridine (i.e., 5% w/w of the compressed cored) and 9% ethylcellulose coat by weight of the compressed core, the ratio of the amount of the ethylcellulose coat to the amount of 4-aminopyridine is 1.8:1; wherein for calculating said ratio, the amount of the ethylcellulose coat is the weight percentage of the ethylcellulose coat by weight of the compressed core (i.e., 9%), and the amount of 4-aminopyridine is the weight percentage of 4-aminopyridine by weight of the compressed core (i.e. 5%).

Similarly, for the sustained release tablet of Example 1, containing 22 mg 4-aminopyridine (i.e., 5% w/w of the compressed cored) and 9% ethylcellulose coat by weight of the compressed core, the ratio of the amount of the ethylcellulose coat to the amount of 4-aminopyridine in the compressed core is about 0.4:1; wherein for calculating said ratio, the amount of the ethylcellulose coat is the weight percentage of the ethylcellulose coat by weight of the compressed core (i.e., 9%), and the amount of 4-aminopyridine is the weight in milligrams of 4-aminopyridine (i.e., 22 mg).

The dissolution profile of the sustained release tablet in Example 1 is shown in Figure 1. The dissolution profile was generated using the conditions shown in Table 2 below.

**Table 2. Dissolution profile parameters.**

| Apparatus | USP Type II (Paddles) |
|---|---|
| Paddle Speed | 50 RPM |
| Dissolution Medium | 50 mM Phosphate Buffer, pH 6.8 |
| Dissolution Medium Temperature | 37.0 ± 0.5° C |
| Sampling Time Profile | 1,2,4,6,8,10,12,14,18, and 24 hours |
| Vessel Volume | 900 mL |
| UV Wavelength | 262 nm |
| Cell | 1 mm, quartz |
| Sinkers | QLA, 2S |

At each sampling time point, a 10 mL sample aliquot was removed from each vessel and collected in a test tube. Each sample aliquot was filtered through a 0.45 µm nylon syringe filter. The first 2 mL of the filtrate was discarded, and the remaining filtrate was collected for UV analysis.

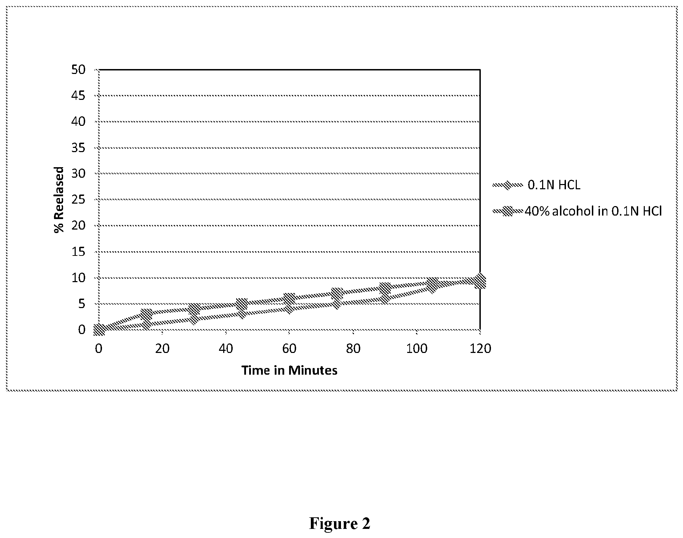

To test alcohol dose dumping, dissolution for 2 hours was performed by employing 0.1 N HCl containing 40% alcohol (ethanol) as a dissolution medium. The results are shown in Figure 2.

### 6.2 Example 2: Sustained Release Tablets Containing 22 mg 4-Aminopyridine and Coated to 8%, 9%, and 10% weight gain

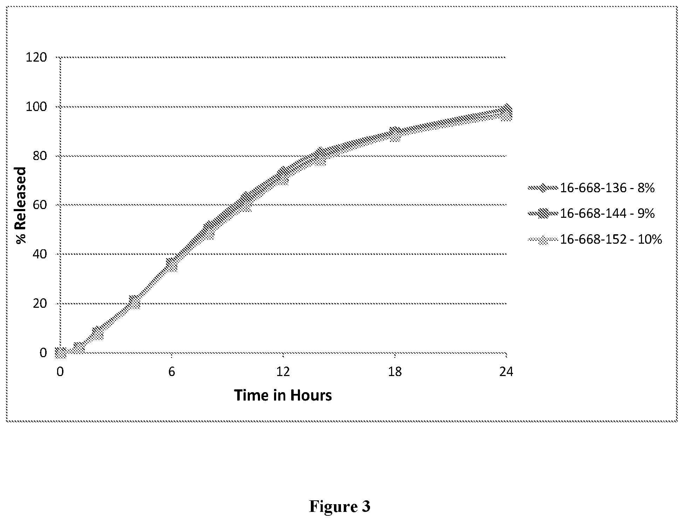

Core tablets (i.e., compressed cores) containing 22 mg 4-aminopyridine were prepared using the formulation described in Table 1 and the method described in Example 1, Sections 0 and 0. These core tablets were coated with an aqueous Ethylcellulose Dispersion Type B (Surelease E-7-19040, Clear) coat to approximately 8% and 10% weight gain, following the procedure described in Example 1, Section 0. A comparison of the dissolution profiles (generated using the parameters shown in Table 2) of the sustained release tablets containing 22 mg 4-aminopyridine, coated with an aqueous Ethylcellulose Dispersion Type B coat to approximately 8% (Example 2), 9% (tablets generated pursuant to Example 1), and 10% (Example 2) weight gain, is shown in Figure 3 and Table 3.

**Table 3. Dissolution profiles of sustained release tablets containing 22 mg 4-aminopyridine, coated to 8%, 9%, and 10% weight gain.**

| | **% 4AP released** | | | | | | | | | | |
|---|---|---|---|---|---|---|---|---|---|---|---|
| **Coat %** | **0 h** | **1 h** | **2h** | **4 h** | **6 h** | **8 h** | **10 h** | **12 h** | **14 h** | **18 h** | **24 h** |
| 8% | 0 | 2 | 9 | 21 | 37 | 52 | 64 | 74 | 82 | 90 | 99 |
| 9% | 0 | 2 | 8 | 21 | 36 | 50 | 62 | 72 | 80 | 90 | 97 |
| 10% | 0 | 2 | 8 | 20 | 35 | 48 | 60 | 71 | 78 | 88 | 97 |

### 6.3 Example 3: Preparation of Sustained Release Tablets Containing 16.5 mg 4-Aminopyridine

Sustained release tablets containing 16.5 mg 4-aminopyridine were prepared using the method described in Example 1, except using the amounts and weight percentages of ingredients shown in Table 4.

**Table 4. Composition of sustained release tablets containing 16.5 mg 4-aminopyridine and 6% ethylcellulose coat by weight of the compressed core.**

| **Ingredient** | **% w/w of compressed core** | **Amount per tablet (mg)** |
|---|---|---|
| **Compressed Core** | | |
| 4-aminopyridine | 3.75 | 16.5 |
| KOLLIDON^{®} SR | 25.0 | 110.0 |
| Polyethylene oxide (Sentry Polyox WSR 303) | 15.0 | 66.0 |
| Dibasic calcium phosphate dihydrate | 55.25 | 243.1 |
| Magnesium stearate | 1.0 | 4.4 |
| **Weight of Compressed Core** | 100 | 440 |

| **Functional Coating** | | |
|---|---|---|
| Ethylcellulose Dispersion Type B | 6 | 26.4 |

Thus, for the sustained release tablet containing 16.5 mg 4-aminopyridine (i.e., 3.75% w/w of the compressed cored) and 6% ethylcellulose coat by weight of the compressed core, the ratio of the amount of the ethylcellulose coat to the amount of 4-aminopyridine is 1.6:1; wherein for calculating said ratio, the amount of the ethylcellulose coat is the weight percentage of the ethylcellulose coat by weight of the compressed core (i.e., 6%), and the amount of 4-aminopyridine is the weight percentage of 4-aminopyridine by weight of the compressed core (i.e. 3.75%).

Similarly, for the sustained release tablet containing 16.5 mg 4-aminopyridine (i.e., 3.75% w/w of the compressed cored) and 6% ethylcellulose coat by weight of the compressed core, the ratio of the amount of the ethylcellulose coat to the amount of 4-aminopyridine in the compressed core is about 0.35:1; wherein for calculating said ratio, the amount of the ethylcellulose coat is the weight percentage of the ethylcellulose coat by weight of the compressed core (i.e., 6%), and the amount of 4-aminopyridine is the weight in milligrams of 4-aminopyridine (i.e., 16.5 mg).

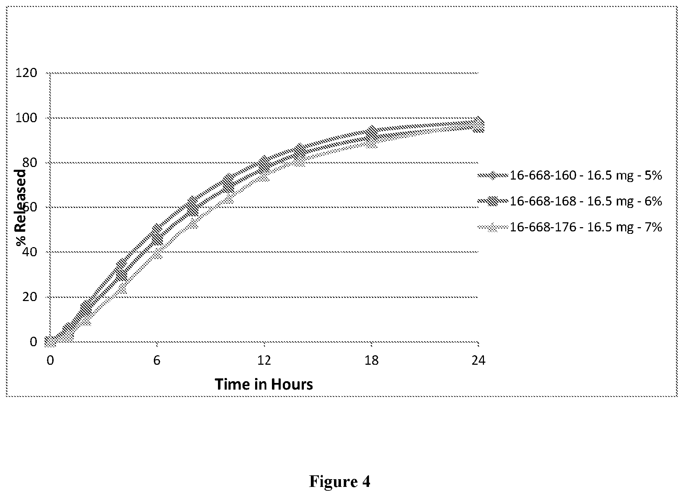

In addition, the core tablets prepared using the formulation described in Table 4 were coated with an aqueous Ethylcellulose Dispersion Type B (Surelease E-7-19040, Clear) coat to approximately 5% and 7% weight gain. A comparison of the dissolution profiles (generated using the parameters shown in Table 2) of the sustained release tablets containing 16.5 mg 4-aminopyridine, coated with an aqueous Ethylcellulose Dispersion Type B coat to approximately 5%, 6%, and 7% weight gain, is shown in Figure 4 and Table 5.

**Table 5. Dissolution profiles of sustained release tablets containing 16.5 mg 4-aminopyridine, coated to 5%, 6%, and 7% weight gain.**

| | **% 4AP released** | | | | | | | | | | |
|---|---|---|---|---|---|---|---|---|---|---|---|
| **Coat %** | **0 h** | **1 h** | **2 h** | **4h** | **6 h** | **8 h** | **10 h** | **12 h** | **14 h** | **18 h** | **24 h** |
| 5% | 0 | 6 | 16 | 35 | 51 | 63 | 73 | 81 | 87 | 94 | 98 |
| 6% | 0 | 5 | 14 | 30 | 46 | 59 | 69 | 77 | 84 | 91 | 96 |
| 7% | 0 | 3 | 10 | 24 | 40 | 53 | 64 | 74 | 81 | 89 | 98 |

### 6.4 Example 4: Preparation of Sustained Release Tablets Containing 22 mg 4-Aminopyridine and Coated to 5% weight gain

### 6.4.1 Dispensing, Sifting and Blending

4-Aminopyridine (5000.0 g), Kollidon SR Polymer (25000.0 g), Polyethylene Oxide, NF (Sentry Polyox WSR 303) (15000.0 g), and Dibasic Calcium Phosphate Dihydrate, USP/NF (54000.0 g) were sifted through a 595 µm (30 mesh) hand screen into a container double-lined with polyethylene bags. The sifted powders obtained above were placed in a 0.24 m³ (8.5 cu foot) tote, and blended for 5 minutes. To this blended mixture was added Magnesium Stearate, NF Non-Bovine Hyqual (1000.0 g) that was sifted through a 841 µm (20 mesh) hand screen. The entire mixture was blended for additional 19 minutes, and then the blend was discharged into a container lined with two polyethylene bags after performing blend uniformity and physical testing on the final blend.

### 6.4.2 Tableting

The completed blend from above was fed into a tablet press hopper. A single rotary tablet press (power assisted) was set up with a tablet deduster and metal detector in-line. Batch set-up parameters were then determined to achieve tablets a target weight of 440 mg (individual range 407-473 mg).

The blend was then compressed using a Fette 1200i Tablet Press with Tooling Set No. 138 to furnish the desired core tablets, while performing checks on individual tablet weight, mean tablet weight, hardness, thickness, appearance and friability at defined times during the compression run. The weight of the desired tablets was within +/- 7.5% of theoretical tablet weight of 440 mg (i.e., within 407.0 - 473.0 mg).

### 6.4.3 Coating and Curing

Purified Water (31200.0 g) was weighed into a suitable solution tank. The Purified Water was mixed with Ethylcellulose Dispersion Type B, NF (Surelease E-7-19040, Clear) (46800 g) while stirring using an overhead mixer with a suitable impeller. Mixing was continued for at least 15 minutes with low to moderate agitation, while ensuring there are no visual signs for any lumps, foam, settling, etc. prior to coating.

The core tablets prepared in the above section were loaded into a O'Hara fastcoat48 tablet coater equipped with a 48" pan, a peristaltic pump with a new tubing (Maserflex Pt/Si Size 35) and 4 spray guns. The tablets were pre-warmed for approximately 10 minutes by jogging the pan with the fan and heater on at 50 °C. After setting the Supply Temperature to target 65 °C, the above prepared film-coat was applied in a controlled manner to the pre-warmed core tablets in the O'Hara fastcoat48 tablet coater to approximately 5% weight gain (i.e., 22.0 mg). The coated tablets thus formed were then subjected to a curing step by drying them at 55 °C for 1 hour and then allowed to cool for at least 5 minutes to a target exhaust temperature of approximately 25-30 °C while jogging the pan. The tablets were then discharged into a suitable container lined with two polyethylene bags to afford the final coated tablets.

Table 6 provides the amounts and weight percentages of ingredients present in the sustained release tablet prepared using the method described above:

**Table 6. Composition of sustained release tablets containing 22 mg 4-aminopyridine and 5% ethylcellulose coat by weight of the compressed core.**

| **Ingredient** | **% w/w of compressed core** | **Amount per tablet (mg)** |
|---|---|---|
| **Compressed Core** | | |
| 4-aminopyridine | 5.0 | 22.0 |
| KOLLIDON^{®} SR | 25.0 | 110.0 |
| Polyethylene oxide (Sentry Polyox WSR 303) | 15.0 | 66.0 |
| Dibasic calcium phosphate dihydrate | 54.0 | 237.6 |
| Magnesium stearate | 1.0 | 4.4 |
| **Weight of Compressed Core** | 100 | 440 |

| **Functional Coating** | | |
|---|---|---|
| Ethylcellulose Dispersion Type B | 5 | 22.0 |

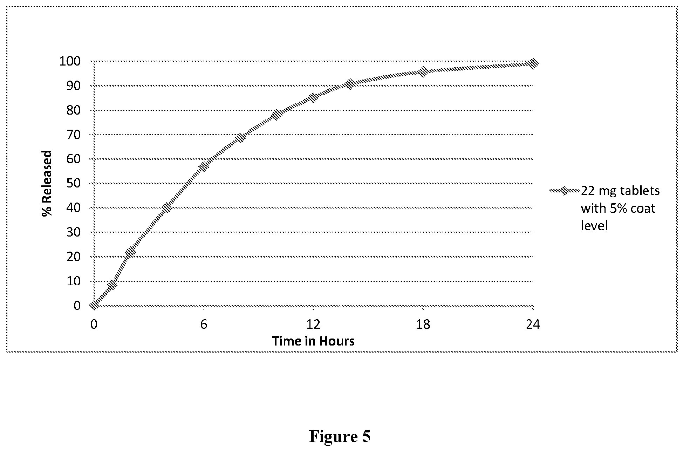

The dissolution profile (generated using the conditions shown in Table 2) of the sustained release tablet in Example 4 is shown in Figure 5.

### 6.5 Example 5: Pharmacokinetic Studies

A study was conducted evaluating the effect of food on the pharmacokinetics of the prototype 22 mg 4-aminopyridine sustained release tablet prepared as described in Example 1, following oral administration of a single dose in healthy human subjects. In the study, subjects observed an overnight fast of at least 10 hours prior to dosing. The following morning, a single dose was orally administered with 240 mL water. The dose was administered on an empty stomach in the fasted condition (N = 20) and following a standard high fat-high content meal (*see,* Guidance for Industry: Food-Effect Bioavailability and Fed Bioequivalence Studies, Food and Drug Administration, Center for Drug Evaluation and Research (CDER), December 2002) in the fed condition (N = 20).

A summary of the pharmacokinetic parameters for dalfampridine following oral administration of the 22 mg prototype tablet of Example 1 in healthy human subjects in fed (N = 20) and fasted (N = 20) states is presented in Table 7. Mean Cₘₐₓ and AUC values were comparable following administration in both fed and fasted states.

Following administration of the prototype formulation in the fed state, absorption was slightly delayed than that of the fasted state, median tₘₐₓ of 12.0 and 7.50 hours, respectively. There were no other notable differences in the pharmacokinetics of the prototype formulation when administered in the fed versus the fasted state.

**Table 7. Summary of Pharmacokinetic Parameters of Dalfampridine Following Administration of a Single Oral Dose of the 22mg 4-Aminopyridine Sustained Release Tablet of Example 1 in Healthy Human Subjects in Fed or Fasted States.**

| | **Prototype-Fed** | **Prototype-Fasted** |
|---|---|---|
| **Pharmacokinetic Parameter (units)** | Arithmetic Mean ± SD (N=20) | Arithmetic Mean ± SD (N=20) |
| Cₘₐₓ (ng/mL) | 18.9 ± 5.17 | 17.7 ± 2.96 |
| AUC₀₋ₗₐₛₜ (ng·hr/mL) | 342 ± 131 | 330 ± 104 |
| AUC₀₋₃₆ (ng·hr/mL) | 344 ± 128 | 333 ± 101 |
| AUC_{0-∞} (ng·hr/mL) | 354 ± 136 | 351 ± 121 |
| tₘₐₓ (hr)^{a} | 12.0 [6.0, 21.0] | 7.50 [3.0, 14.0] |
| t_{lag} (hr)^{a} | 1.0 [0, 2.0] | 0.50 [0, 1.0] |
| λ_{z} (1/hr)^{b} | 0.176 ± 0.0331 | 0.172 ± 0.0551 |
| t_{1/2} (hr) | 4.1 ± 0.87 | 4.7 ± 2.30 |

| | | |
|---|---|---|
| ^{a} Median [min, max] reported for tₘₐₓ and t_{lag}; t_{lag} is defined as time prior to the first measurable (non-zero) plasma concentration ^{b} λ_{z} is defined as apparent elimination rate constant | | |

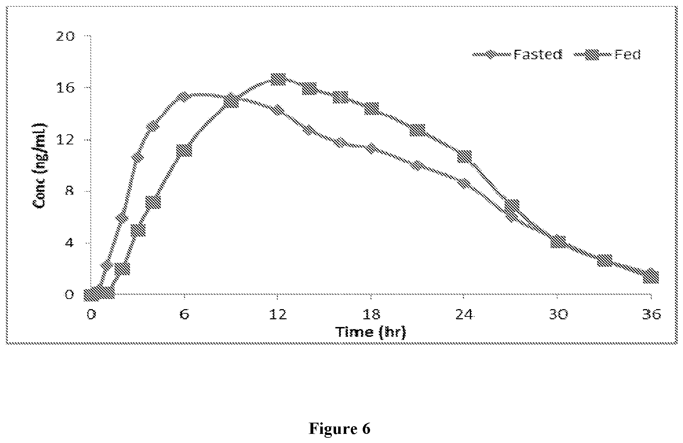

The mean plasma concentrations over time are shown in Figure 6, which demonstrates that sustained plasma levels are achieved over a 24-hour period with a single dose. These sustained plasma levels demonstrate feasibility of once-daily dosing.

### 6.6 Example 6: A 3-Period Crossover, Open Label Randomized Study to Evaluate the Steady State Pharmacokinetic Properties of the 4-AP sustained release tablets for once daily administration in healthy volunteers

### 6.6.1 Overall Study Design and Plan: Description

This was an open-label, 3-period crossover, randomized, PK study of a prototype dalfampridine-ER tablet formulation for once-daily administration to healthy volunteers conducted at a single site in the US. Two dose strengths were tested to verify the performance of the formulation: a 16.5 mg (low dose) and a 22 mg (high dose) tablet. Sustained release 4-aminopyridine tablets of the invention were used in this study. The 16.5 mg 4-aminopyridine tablets were prepared as described in Example 3, utilizing the coating parameters described in Example 7, Table 11 (16.5 mg Batch C). The 22 mg 4-aminopyridine tablets were prepared as described in Example 1, utilizing the coating parameters described in Example 7, Table 11 (22 mg Batch B). Single dose and steady state PK parameters were evaluated, with the high dose administered in the morning or evening (defined in this study as approximately at bedtime). All subjects were to receive 7 daily doses of each of the following treatments: low dose administered in the morning, high dose administered in the morning, and high dose administered in the evening. There was a 5-day washout between the 3 dosing periods. The order of the treatments was to be randomly assigned.

On Day 1, following randomization, subjects were to receive a single tablet of their assigned dosage strength and regimen for Dosing Period 1 (low dose in morning; high dose in morning; or high dose in evening). Blood samples were to be collected for determination of plasma dalfampridine concentrations over a 24-hour period. Additional once-daily doses were to be administered over the following 6 days. Blood samples were to be collected predose on Day 5 and Day 6 to establish trough concentrations, and serial blood sampling was to be performed again on the final day of dosing in Period 1 (Day 7) to evaluate the steady state PK profile of the test formulation.

After a 5-day washout, subjects were to begin their next assigned treatment (Dosing Period 2). The blood sampling schedules were to be the same as Dosing Period 1. Another 5-day washout was to be imposed before the crossover to the third and final series of doses (Dosing Period 3).

### 6.6.2 Treatments

Each subject received 7 once-daily oral doses of 16.5 mg dalfampridine-ER formulation tablets administered in the morning, 7 once-daily oral doses of 22 mg dalfampridine-ER formulation tablets administered in the morning, and 7 once-daily oral doses of 22 mg dalfampridine-ER formulation tablets administered in the evening, according to a randomized sequence.

Doses of 16.5 mg dalfampridine-ER formulation were administered in the morning and doses of 22 mg dalfampridine-ER formulation were administered in the morning and evening.

Subjects received standardized meals (breakfast, lunch, dinner, snacks) that were scheduled at consistent times through the study that did not coincide with study assessments. On the morning dosing days when serial PK sampling was performed, the first clinic meal was served no earlier than 4 hours after dose administration. If the dose was administered in the evening, defined as approximately at bedtime (e.g., 22:00), dinner was served at least 4 hours prior to evening dose administration and no snack was permitted. On dosing days when there was no PK sampling or only a single PK sample, food consumption was restricted from 3 hours before dosing to 1 hour after dosing.

With the exception of the 240 mL water used to take the oral tablet, water was permitted to be consumed ad libitum up until 1 hour before and after each dose.

There was no blinding in this study.

### 6.6.3 Pharmacokinetics (PK) and Safety Variables

Single-dose PK was assessed from blood samples collected on Days 1 and 2 of Period 1 and the first 2 days of Periods 2 and 3. Samples were collected within 15 minutes prior to administration of the first dose of IP in each period and at 13 serial timepoints over a 24-hour period. Blood samples for the analysis of trough concentrations were collected predose on Days 5 and 6 of each period. Steady-state PK was assessed after completion of 7 consecutive daily doses in each period. Blood samples were collected within 15 minutes prior to dose administration and at 15 serial timepoints over a 36-hour period.

The PK parameters referred to in this Example are indicated in Table 8.

**Table 8: Pharmacokinetic Parameters.**

| **Parameter** | **Definition** |
|---|---|
| AUC₀₋₂₄ₕ (h·ng/mL) | area under the plasma concentration versus time curve from time 0 to the concentration at 24 hours |
| AUC_{0-∞} (h·ng/mL) | area under the concentration-time curve from time 0 to infinity (single dose only) |
| Cₘₐₓ (ng/mL) | maximum measured plasma drug concentration |
| Cₘᵢₙ (ng/mL) | minimum concentration at the end of the dosing interval (steady state only) |
| λ_{z} (1/h) | apparent terminal rate constant (single dose only) |
| tₘₐₓ **(h)** | time to maximum observed plasma drug concentration |
| t_{1/2} (h) | apparent terminal half-life, calculated as ln(2)/ λ_{z} |

### 6.6.4 Study Subjects

The entire study population was divided into 2 separate populations, Initial and Restarted Populations.

Thirty-one subjects were screened and enrolled into the Initial Population, with no subjects failing Screening based on the inclusion and exclusion criteria. All 31 subjects were assigned to a treatment sequence. Of the 31 subjects enrolled in the study, all 31 subjects were withdrawn from the study by the Sponsor due to an error in randomization.

Thirty of the 31 subjects from the Initial Population were rescreened and re-enrolled into the Restarted Population. Two additional subjects were screened for a total of 32 subjects screened, with no subjects failing Screening based on the inclusion and exclusion criteria. All of the 32 subjects in the Restarted Population that were randomized completed the study.

### 6.6.5 Pharmacokinetics Evaluation

### 6.6.5.1 Demographic and Other Baseline Characteristics

Twelve women and 19 men, aged between 27 and 59 years, and with a BMI between 21.6 and kg/m², were enrolled into the Initial Population of this study.

Twelve women and 20 men, aged between 27 and 59 years, and with a BMI between 21.3 and kg/m², were enrolled into the Restarted Population of this study.

All subjects in both the Initial and Restarted Populations met the inclusion criteria and none of the exclusion criteria prior to enrollment into the study.

### 6.6.5.2 Steady State Pharmacokinetics of Dalfampridine

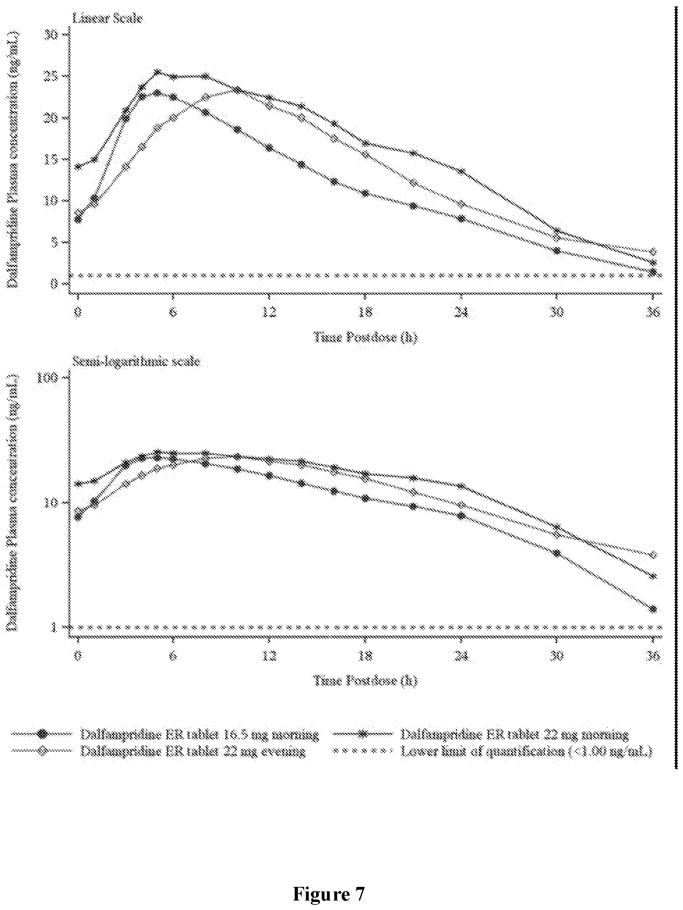

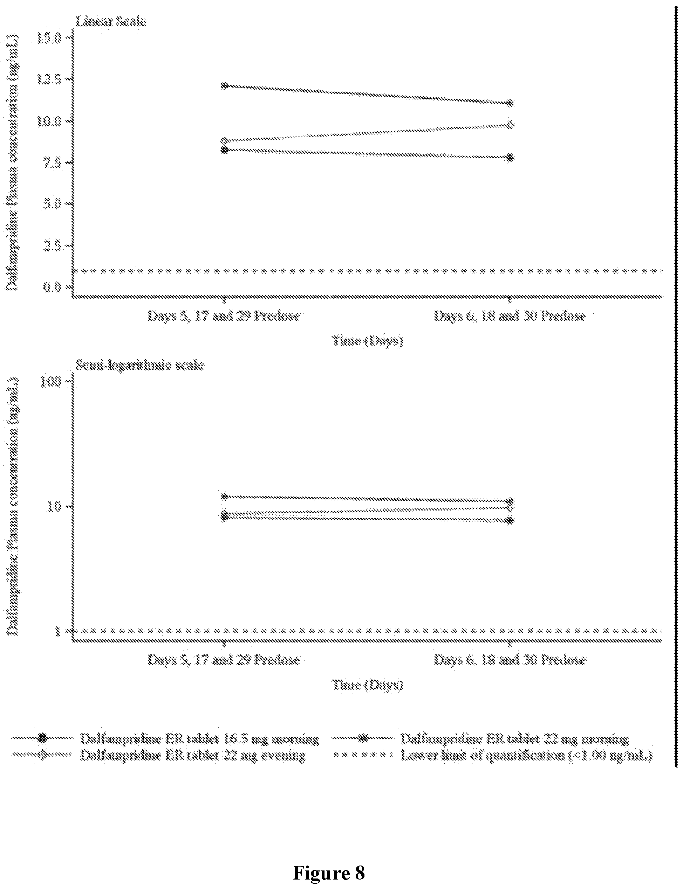

Mean plasma concentrations of dalfampridine at steady state are shown in Figure 7, with trough concentrations displayed in Figure 8. A summary of the PK parameters for dalfampridine at steady state for the 16.5 mg tablet in the morning or the 22 mg tablet in the morning or evening are presented in Table 9. Box plots showing comparisons of AUC₀₋₂₄ₕ and Cₘₐₓ of Dalfampridine for each treatment of dalfampridine-ER at steady state are presented in Figure 9.

**Table 9: Summary of Steady State Pharmacokinetics Parameters for Dalfampridine.**

| Pharmacokinetic Parameter | Arithmetic Mean (± SD) | | |
|---|---|---|---|
| | Dalfampridine ER 16.5 mg morning (N = 32) | Dalfampridine ER 22 mg morning (N = 32) | Dalfampridine ER 22 mg evening (N = 32) |
| Cₘₐₓ (ng/mL) | 24.9 ± 6.91 | 27.9 ± 9.29 | 25.2 ± 8.81 |
| AUC₀₋₂₄ₕ (h·ng/mL) | 362 ± 105 | 481 ± 157 | 405 ± 163 |
| Tₘₐₓ (h)^{a} | 4.99 [3.00, 9.98] | 6.01 [0.00, 16.0] | 9.98 [2.97, 20.9] |
| Cₘᵢₙ (ng/mL) | 7.91 ± 3.78 | 13.6 ± 5.87 | 9.62 ± 7.80 |
| t_{1/2} (h) | 6.28 ± 2.39 | 5.47 ± 2.16 | 8.09 ± 6.97 |

| | | | |
|---|---|---|---|
| Abbreviations: AUC₀₋₂₄ₕ = area under the plasma concentration versus time curve from time 0 to the concentration at 24 hours; Cₘₐₓ = maximum measured plasma concentration; Cₘᵢₙ = minimum concentration at the end of the dosing interval; Tₘₐₓ = time to maximum observed plasma drug concentration; t_{1/2} = apparent terminal half-life ^{a} Median (range) | | | |

Plasma concentrations of dalfampridine appeared to have attained steady state levels by the fifth day of once-daily dosing for each of the 3 dose regimens. At steady state for both the 16.5 mg and 22 mg tablets of dalfampridine-ER administered in the morning, absorption of Dalfampridine was steady, with median tₘₐₓ values of approximately 5.0 hours and 6.0 hours postdose, respectively. Steady state tₘₐₓ occurred earlier for the 16.5 mg morning dose compared to the 22 mg morning dose. After reaching Cₘₐₓ, plasma concentrations declined in an apparent monophasic manner, with mean apparent terminal half-life (t_{1/2}) values for each dose also being similar (approximately 5.9 hours and 5.1 hours for the 16.5 mg and 22 mg tablets, respectively). The range for all subjects across morning doses was 2.9 to 12.5 hours.

### 6.6.5.3 Single Dose Pharmacokinetics of Dalfampridine

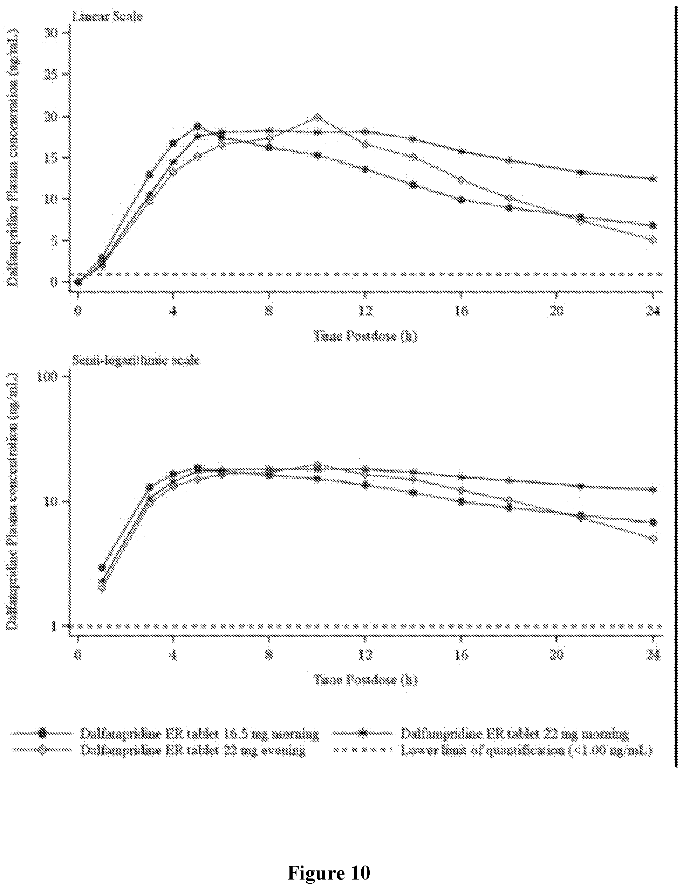

Mean plasma concentrations of dalfampridine after single doses are summarized in Figure 10. A summary of the PK parameters for dalfampridine after single doses of the 16.5 mg tablet in the morning or the 22 mg tablet in the morning or evening is presented in Table 10. Box plots for comparisons of AUC₀₋ₗₐₛₜ and Cₘₐₓ of dalfampridine for each treatment of dalfampridine-ER following single doses are presented in Figure 11.

**Table 10: Summary of Single Dose Pharmacokinetics Parameters for Dalfampridine.**

| Pharmacokinetic Parameter | Arithmetic Mean (± SD) | | |
|---|---|---|---|
| | Dalfampridine ER 16.5 mg morning (N = 32) | Dalfampridine ER 22 mg morning (N = 32) | Dalfampridine ER 22 mg evening (N = 32) |
| Cₘₐₓ (ng/mL) | 19.7 ± 3.56 | 21.0 ± 5.74 | 21.6 ± 8.21 |
| AUC₀₋₂₄ₕ (h·ng/mL) | 277 ± 84.5 | 352 ± 105 | 305 ± 101 |
| AUC_{0-∞} (h·ng/mL) | 296 ± 136 | 489 ± 305 | 340 ± 138 |
| Tmax (h)^{a} | 5.00 [3.00, 12.0] | 9.94 [4.00, 23.8] | 9.96 [5.00, 24.0] |
| t_{1/2} (h) | 12.1 ± 6.88 | 17.8 ± 11.8 | 5.61 ± 3.49 |

| | | | |
|---|---|---|---|
| Abbreviations: AUC₀₋₂₄ₕ = area under the plasma concentration versus time curve from time 0 to the concentration at 24 hours; AUC_{0-∞} = area under the concentration-time curve from time 0 to infinity; Cₘₐₓ = maximum measured plasma concentration; Tₘₐₓ = time to maximum observed plasma drug concentration; t_{1/2} = apparent terminal elimination half-life ^{a} Median (range) | | | |

Following a single dose of the 16.5 mg tablet of dalfampridine-ER administered in the morning, absorption of dalfampridine was steady, with a median tₘₐₓ of 5.0 hours postdose. After reaching Cₘₐₓ, plasma concentrations declined in an apparent monophasic manner. The mean t_{1/2} was approximately 10.0 hours, with values for individual subjects ranging from 2.7 to 24.8 hours.

Following a single dose of the 22 mg tablet of dalfampridine-ER administered in the morning, absorption of dalfampridine was slower than that observed for the 16.5 mg tablet, with a median tₘₐₓ of approximately 9.9 hours postdose. Single dose tₘₐₓ occurred earlier for the 16.5 mg morning dose compared to the 22 mg morning dose, while tₘₐₓ was similar for the morning and evening doses of the 22 mg dalfampridine-ER tablet. After reaching Cₘₐₓ, plasma concentrations declined in an apparent monophasic manner.

The mean t_{1/2} was approximately 14.9 hours, with values for individual subjects ranging from 3.3 to 57.5 hours.

Following the first dose of the 22 mg tablet of dalfampridine-ER administered in the evening, absorption of dalfampridine was slower than that observed following morning dosing, with a median tₘₐₓ of approximately 10.0 hours postdose. After reaching Cₘₐₓ, plasma concentrations appeared to decline in a monophasic manner. The mean t_{1/2} was approximately 4.9 hours, with values for individual subjects ranging from 2.5 to 13.8 hours.

### 6.6.5.4 Pharmacokinetics Conclusions

Plasma concentrations of dalfampridine reached steady state by the fifth day of once-daily administration of 16.5 mg or 22 mg dalfampridine-ER tablets. Median tₘₐₓ at steady state for the 16.5 mg tablet administered in the morning was approximately 5.0 hours postdose and occurred earlier than for the 22 mg tablet administered in the morning or evening, which had steady state median tₘₐₓ values of approximately 6.0 and 10.0 hours, respectively.

Steady state tₘₐₓ occurred earlier for the 16.5 mg morning dose compared to the 22 mg morning dose, while the steady state tₘₐₓ for the 22 mg morning dose occurred earlier compared to the 22 mg evening dose.

At steady state for once-daily administration of the 16.5 mg or 22 mg dalfampridine-ER tablets in the morning or the 22 mg tablet in the evening, plasma concentrations appeared to decline in a monophasic manner after reaching Cₘₐₓ, with similar mean t_{1/2} values of approximately 5.0 to 6.0 hours.

Following a single dose of the 16.5 mg or 22 mg dalfampridine-ER tablets in the morning or the 22 mg tablet in the evening, median tₘₐₓ values were 5.0, 9.9, and 10.0 hours postdose, respectively. Single dose tₘₐₓ occurred earlier for the 16.5 mg morning dose compared to the 22 mg morning dose, while tₘₐₓ was similar for the morning and evening doses of the 22 mg dalfampridine-ER tablet.

### 6.6.6 Safety Evaluation and Conclusion

Once-daily dalfampridine-ER, 16.5 mg and 22 mg tablets administered in the morning and 22 mg tablets administered in the evening, was safe and well tolerated when administered to the healthy subjects in this study.

### 6.6.7 Discussion and Overall Conclusions

### Discussion

This was an open-label, 3-period crossover, randomized, study of a prototype dalfampridine-ER tablet formulation that evaluated the steady-state PK of once-daily administration and its safety and tolerability in healthy adult subjects.

Based on Cₘₐₓ and AUC₀₋₂₄ₕ, steady state exposure for the 16.5 mg and 22 mg dalfampridine-ER tablets administered once daily in the morning and the 22 mg tablet administered once daily in the evening, was increased when compared to Cₘₐₓ and AUC₀₋₂₄ₕ after single doses. While the median tₘₐₓ for the 16.5 mg tablet administered in the morning and the 22 mg tablet administered in the evening was similar following single doses versus at steady state, the median tₘₐₓ for the 22 mg tablet administered in the morning occurred earlier (approximately 6.0 hours postdose) at steady state, when compared to after a single dose (approximately 9.9 hours postdose).

Once-daily administration of dalfampridine-ER, 16.5 mg and 22 mg tablets administered in the morning and 22 mg tablets administered in the evening, was well tolerated by the healthy subjects in this study with low and similar numbers of subjects reporting TEAEs for each of the 3 dose regimens.

The median tₘₐₓ for dalfampridine at steady state occurred earlier for the 22 mg morning dose compared to the 22 mg evening dose.

For single doses, Cₘₐₓ for dalfampridine was similar for the morning and evening doses, while AUC₀₋₂₄ₕ was higher for the morning dose. The median tₘₐₓ was similar for the morning and evening doses of the 22 mg dalfampridine-ER tablet (approximately 9.9 hours postdose) after a single dose.

There were no clinically significant abnormalities or changes observed in the clinical laboratory, vital signs, ECG, C-SSRS, or physical examination findings for any subjects.

### Conclusions

Plasma concentrations of dalfampridine reached steady state by the fifth day of once-daily administration of the 16.5 mg or 22 mg dalfampridine-ER tablets. Median tₘₐₓ at steady state for the 16.5 mg tablet administered in the morning was approximately 5.0 hours postdose and occurred earlier than for the 22 mg tablet administered in the morning or evening, which had steady state median tₘₐₓ values of approximately 6.0 and 10.0 hours, respectively.

Steady state tₘₐₓ occurred earlier for the 16.5 mg morning dose compared to the 22 mg morning dose, while the steady state tₘₐₓ for the 22 mg morning dose occurred earlier compared to the 22 mg evening dose.

At steady state for once-daily administration of the 16.5 mg or 22 mg dalfampridine-ER tablets in the morning or the 22 mg tablet in the evening, plasma concentrations appeared to decline in a monophasic manner after reaching Cₘₐₓ, with similar mean t_{1/2} values of approximately 5.0 to 6.0 hours.

Following a single dose of the 16.5 mg or 22 mg dalfampridine-ER tablets in the morning or the 22 mg tablet in the evening, median tₘₐₓ values were 5.0, 9.9, and 10.0 hours postdose, respectively. Single dose tₘₐₓ occurred earlier for the 16.5 mg morning dose compared to the 22 mg morning dose, while tₘₐₓ was similar for the morning and evening doses of the 22 mg dalfampridine-ER tablet.

Once-daily dalfampridine-ER, 16.5 mg and 22 mg tablets administered in the morning and 22 mg tablets administered in the evening, was safe and well tolerated when administered to the healthy subjects in this study.

### 6.7 Example 7: Coating Parameters for Sustained Release 4-Aminopyridine Tablets

Coating parameters that were utilized for preparing sustained release tablets containing 22 mg 4-aminopyridine, coated with an aqueous Ethylcellulose Dispersion Type B coat to approximately 9% (tablets generated pursuant to Example 1) and 16.5 mg 4-aminopyridine, coated with an aqueous Ethylcellulose Dispersion Type B coat to approximately 6% (tablets generated pursuant to Example 3) weight gain, are shown in Table 11.

**Table 11. Coating Parameters for Sustained Release 4-AP Tablets.**

| | **16.5 mg 4-AP** | | **22 mg 4-AP** | | **16.5 mg 4-AP** | |
|---|---|---|---|---|---|---|
| **Batch Size and ID** | 272,272 tablets (100Kg) 16.5 mg Batch A (Scale up batch) | | 5000 tablets (2.2Kg) 22mg Batch B | | 5000 tablets (2.2Kg) 16.5 mg Batch C | |
| **Parameter** | **Target** | **Range/Actual** | **Target** | **Range/Actual** | **Target** | **Range/Actual** |
| Supply Air (°C) | 65 | 59 - 70 | 60 | 60-65 | 60 | 56-60 |
| Bed Temperature (°C) | 46 | ~46-52 | 42 | ~38-41 | 42 | ~39-41 |
| Airflow (CFM) | 1950 | 1903 - 1998 | 110-120 | 111-116 | 110-120 | 110 |
| Spray Rate | 300 (g/min) | 300 (g/min) | 5-12 mL/min | 8.5-10 mL/min | 5-12 mL/min | 7mL/min |
| Pan Speed (RPM) | 8 | 8 | 18-20 | 19 | 18-20 | 20 |
| Atomizing Air (psi) | 25 | 25 | 15-25 | 20 | 15-25 | 20 |
| Pattern Air (psi) | 25 | 25 | 15-25 | 20 | 15-25 | 20 |
| Weight Gain (%) | 6.0 | 5.8 | 9 | 9.1 | 9 | 6.1 |
| Drying Time (hours) | 1 | 1 | 1 | 1 | 1 | 1 |
| Drying Temperature (°C) | 55 | 55 | 55 | 55 | 55 | 55 |
| Coating equipment | O'Hara Fastcoat 48" with a peristaltic pump | | Labcoat MX coater 15" with a peristaltic pump | | Labcoat MX coater 15" with a peristaltic pump | |

Table 12 shows a comparison of the dissolution profiles (generated using the parameters shown in Table 2) of the sustained release tablets containing 16.5 mg and 22 mg 4-aminopyridine, coated according to the parameters shown in Table 11 above.

**Table 12. Dissolution profiles of sustained release tablets containing 16.5 mg 4-aminopyridine, coated to approximately 6% weight gain, and 22 mg 4-aminopyridine, coated to approximately 9% weight gain.**

| **Time (hrs)** | **16.5 mg Batch C** | **22 mg Batch B** | **16.5 mg Batch A (Scale Up)** |
|---|---|---|---|
| 0 | 0 | 0 | 0 |
| 1 | 12 | 8 | 6 |
| 2 | 24 | 17 | 16 |
| 4 | 43 | 35 | 32 |
| 6 | 58 | 50 | 49 |
| 8 | 69 | 62 | 62 |
| 10 | 78 | 72 | 72 |
| 12 | 85 | 79 | 80 |
| 14 | 91 | 85 | 87 |
| 18 | 97 | 94 | 93 |
| 24 | 101 | 99 | 98 |

### 6.8 Example 8: Sustained release 4-AP tablets containing a moisture barrier coat and a release modifying coating agent.

Prior to coating with a release modifying coating agent, the 4-aminopyridine core tablets (i.e., compressed cores) can be coated with a moisture barrier coat.

### Step 1

Prepare core tablets (i.e., compressed cores; 100,000 g) containing 16.5 mg 4-aminopyridine using the formulation described in Table 4 and the method described in Example 3.

### Step 2

Prepare an OPADRY^{®} Clear coating suspension by mixing OPADRY^{®} Clear (6,817 g) and water (47,196 g).

### Step 3

Prepare a SURELEASE^{®} coating suspension by mixing Ethylcellulose Dispersion Type B, NF (SURELEASE^{®} E-7-19040, Clear) (31,824 g) and water (21,216 g), while stirring using an overhead mixer with a suitable impeller. Continue mixing for at least 15 minutes with low to moderate agitation, while ensuring there are no visual signs for any lumps, foam, settling, etc. prior to coating.

### Step 4

Load the core tablets into O'Hara Fastcoat 48 and pre-warm the tablets for approximately 10 minutes by jogging the pan with the fan and heater on at 50 °C. Set supply temperature to target 65 °C and apply OPADRY^{®} film-coat to the pre-warmed core tablets to a weight gain of approximately 2.0% of the pre-warmed core tablet weight, utilizing the target process parameters shown below:
Target Pan Speed: 8.0 RPM
Target Air Flow: 1950 cfm
Target Spray Rate: 300.0 g/min (May be adjusted)
Target Supply Temperature: 65 °C (May be adjusted)
Pattern Air Pressure: 30 psi (May be adjusted)
Atomizing Air Pressure: 30 psi (May be adjusted)

Once the minimum weight gain is reached, set the inlet temperature to 50 °C and jog the pan between 3-6 rpm. Continue jogging the pan for minimum of 10 minutes to allow the tablets to dry.

### Step 5

Set supply temperature to target 65 °C and apply SURELEASE^{®} film-coat to the pre-warmed tablets from Step 4 above to a weight gain of approximately 6.0% of the pre-warmed tablet from Step 4 above, utilizing the target process parameters shown below:
Target Pan Speed: 8.0 RPM
Target Air Flow: 1950 cfm
Target Spray Rate: 300.0 g/min (may be adjusted to maintain a target bed temperature of 46 °C)
Target Supply Temperature: 65 °C (may be adjusted to maintain a target bed temperature of 46 °C)
Pattern Air Pressure: 25 psi (May be adjusted)
Atomizing Air Pressure: 25 psi (May be adjusted)

### Step 6

Cure the tablets from Step 5 above for approximately 1 hour at a target of 60 °C while jogging the pan (air flow: 1950 cfm; pan jogging interval: 5 seconds on / 30 seconds off; pan jog speed: 3-6 rpm). Allow the tablets to cool for at least 5 minutes to a target exhaust temperature of approximately 25-30 °C while jogging the pan.

## Claims

1. A sustained release tablet comprising:
(a) a compressed core, said compressed core comprising 4-aminopyridine, a polyethylene oxide with a molecular weight between about 1,000,000 and 10,000,000, and a mixture comprising polyvinyl acetate and polyvinyl pyrrolidone, and
(b) an amount of an ethylcellulose coat surrounding said compressed core;
(b1) wherein said amount of the ethylcellulose coat is in the range of about 5% w/w to about 10% w/w of the compressed core; or
(b2) wherein the ratio of the amount of the ethylcellulose coat to the amount of 4-aminopyridine in the compressed core is in the range of about 0.5:1 to about 3:1, wherein for calculating said ratio, the amount of the ethylcellulose coat is the weight percentage of the ethylcellulose coat by weight of the compressed core, and the amount of 4-aminopyridine is the weight percentage of 4-aminopyridine by weight of the compressed core; or
(b3) wherein the ratio of the amount of the ethylcellulose coat to the amount of 4-aminopyridine in the compressed core is in the range of about 0.1:1 to about 0.7:1, wherein for calculating said ratio, the amount of the ethylcellulose coat is the weight percentage of the ethylcellulose coat by weight of the compressed core, and the amount of 4-aminopyridine is the weight in milligrams of 4-aminopyridine;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h; or
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

2. The sustained release tablet of claim 1, wherein (b) is (b1); and
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

3. The sustained release tablet of claim 1 wherein (b) is (b1); and
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

4. The sustained release tablet of claim 2 or claim 3:
i) wherein said mixture further comprises one or more pharmaceutically acceptable excipients; and/or
ii) wherein said mixture comprises 70-90% polyvinyl acetate and 15-20% polyvinyl pyrrolidone; and/or
iii) wherein said mixture further comprises a surfactant; and/or
iv) wherein said mixture further comprises silica; and/or
v) wherein said mixture consists of about 80% polyvinyl acetate, about 19% polyvinyl pyrrolidone, about 0.8% sodium lauryl sulfate, and about 0.2% silica; and/or
vi) wherein the compressed core further comprises a filler and a lubricant; and/or
vii) wherein the compressed core further comprises a filler and a lubricant and wherein the filler is dibasic calcium phosphate dihydrate, and the lubricant is magnesium stearate; and/or
viii) wherein the polyethylene oxide has a molecular weight between 4,000,000 and 8,000,000; and/or
ix) wherein the polyethylene oxide has a molecular weight of 7,000,000; and/or
x) wherein the total amount of the 4-aminopyridine in the tablet is in the range of about 1% w/w to about 10% w/w of the sustained release tablet; and/or
xi) wherein the total amount of the 4-aminopyridine in the tablet is in the range of about 1% w/w to about 10% w/w of the compressed core.

5. The sustained release tablet of claim 2:
wherein the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% of silica; and
wherein the compressed core further comprises:
dibasic calcium phosphate dihydrate, and
magnesium stearate; and
wherein the amount of 4-aminopyridine is in the range of about 4% w/w to about 6% w/w of the compressed core, said amount of the 4-aminopyridine being equal to 22 mg.

6. The sustained release tablet of any of claims 2-5, wherein the amount of the ethylcellulose coat surrounding the compressed core is about 9% w/w of the compressed core.

7. The sustained release tablet of claim 3 wherein the amount of the ethylcellulose coat surrounding said compressed core is in the range of about 5% w/w to about 7% w/w of the compressed core.

8. The sustained release tablet of claim 7, wherein the amount of 4-aminopyridine is in the range of about 3% w/w to about 5% w/w of the compressed core and the amount of the ethylcellulose coat surrounding the compressed core is about 6% w/w of the compressed core.

9. The sustained release tablet of claim 2:
wherein the amount of 4-aminopyridine in the compressed core is in the range of about 4% w/w to about 6% w/w of the compressed core; and
wherein the amount of the polyethylene oxide in the compressed core is in the range of about 10% w/w to about 20% w/w of the compressed core; and
wherein the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; and
wherein the compressed core further comprises:
dibasic calcium phosphate dihydrate, wherein the amount of dibasic calcium phosphate dihydrate is in the range of about 50% w/w to about 60% w/w of the compressed core; and
magnesium stearate, wherein the amount of magnesium stearate is in the range of about 0.7% w/w to about 1.3% w/w of the compressed core.

10. The sustained release tablet of claim 9:
wherein the polyethylene oxide in the compressed core has a molecular weight of 7,000,000, wherein the amount of the polyethylene oxide is about 15% w/w of the compressed core; and
wherein the amount of the mixture comprising polyvinyl acetate and polyvinyl pyrrolidone consisting of about 80% polyvinyl acetate, about 19% polyvinyl pyrrolidone, about 0.8% sodium lauryl sulfate, and about 0.2% of silica is about 25% w/w of the compressed core; and
wherein the amount of dibasic calcium phosphate dihydrate is about 54% w/w of the compressed core; and
wherein the amount of magnesium stearate is about 1% w/w of the compressed core; and
wherein the amount of the ethylcellulose coat surrounding said compressed core is about 9% w/w of the compressed core.

11. The sustained release tablet of claim 7:
wherein the amount of 4-aminopyridine in the compressed core is in the range of about 3% w/w to about 5% w/w of the compressed core; and
wherein the amount of the polyethylene oxide in the compressed core is in the range of about 10% w/w to about 20% w/w of the compressed core; and
wherein the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% of silica, wherein the amount of the mixture is in the range of about 20% w/w to about 30% w/w of the compressed core; and
wherein the compressed core further comprises:
dibasic calcium phosphate dihydrate, wherein the amount of dibasic calcium phosphate dihydrate is in the range of about 50% w/w to about 60% w/w of the compressed core; and
magnesium stearate, wherein the amount of magnesium stearate is in the range of about 0.7% w/w to about 1.3% w/w of the compressed core.

12. The sustained release tablet of claim 11:
wherein the polyethylene oxide in the compressed core has a molecular weight of 7,000,000, wherein the amount of the polyethylene oxide is about 15% w/w of the compressed core; and
wherein the amount of the mixture comprising polyvinyl acetate and polyvinyl pyrrolidone consisting of about 80% polyvinyl acetate, about 19% polyvinyl pyrrolidone, about 0.8% sodium lauryl sulfate, and about 0.2% of silica is about 25% w/w of the compressed core; and
wherein the amount of dibasic calcium phosphate dihydrate in the compressed core is about 55% w/w of the compressed core; and
wherein the amount of magnesium stearate in the compressed core is about 1% w/w of the compressed core; and
wherein the amount of the ethylcellulose coat surrounding said compressed core is about 6% w/w of the compressed core.

13. The sustained release tablet of claim 1, wherein (b) is (b2); and
wherein the amount of 4-aminopyridine in the sustained release tablet is about 22 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

14. The sustained release tablet of claim 1, wherein (b) is (b2); and
wherein the amount of 4-aminopyridine in the sustained release tablet is about 16.5 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

15. The sustained release tablet of claim 1, wherein (b) is (b3); and
wherein the amount of 4-aminopyridine in the sustained release tablet is about 22 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

16. The sustained release tablet of claim 1, wherein (b) is (b3); and
wherein the amount of 4-aminopyridine in the sustained release tablet is about 16.5 mg; and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

17. The sustained release tablet of any of claims 1-16:
i) wherein the sustained release tablet is the product of a process comprising a curing step after coating the compressed core with ethylcellulose, and wherein said curing step comprises heating the coated compressed core to a temperature above 23 °C for a period of time of at least 15 minutes; and optionally
wherein said curing step comprises exposing the coated compressed core to a temperature in the range of 50-60 °C for a period of time of at least 1 hour; and/or
ii) wherein the sustained release tablet does not further comprise an immediate release drug overcoat containing 4-aminopyridine; and/or
iii) wherein the sustained release tablet provides a zero-order or near-zero-order release of the 4-aminopyridine; and/or
iv) wherein the sustained release tablet provides a zero-order or near-zero-order release of the 4-aminopyridine and wherein the release is zero-order; and/or
v) wherein the release of the 4-aminopyridine, upon subjecting the tablet to an in vitro dissolution test employing 50 mM Phosphate Buffer, pH 6.8 as dissolution medium, is as follows:
within the first 2 hours after the start of the test at most 30% w/w of the total amount of the 4-aminopyridine contained in the sustained release tablet is released, and optionally
wherein the release of the 4-aminopyridine is as follows:
within the first 24 hours after the start of the test at least 80% w/w of the total amount of the 4-aminopyridine contained in the sustained release tablet is released.

18. A method of making a sustained release tablet comprising 4-aminopyridine, which method comprises:
(a) forming a compressed core comprising (i) 4-aminopyridine, (ii) a polyethylene oxide with a molecular weight between about 1,000,000 and 10,000,000, (iii) a mixture comprising polyvinyl acetate and polyvinyl pyrrolidone; (iv) dibasic calcium phosphate dihydrate, and (v) magnesium stearate;
(b) coating the compressed core with an amount of ethylcellulose to form a coated compressed core, said amount of the ethylcellulose coat being in the range of about 5% w/w to about 10% w/w of the compressed core; and
(c) curing the coated compressed core by exposing it to a temperature in the range of 40-70 °C for a period of time of at least 1 hour;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h ; or
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

19. The method of claim 18;
wherein the amount of 4-aminopyridine in the sustained release tablet is about 22 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 18.61 ng/mL to about 37.19 ng/mL, (b) a mean steady state plasma 4-aminopyridine Cₘᵢₙ in the range of about 7.73 ng/mL to about 19.47 ng/mL, (c) a mean steady state plasma 4-aminopyridine AUC₀₋₂₄ in the range of about 324 ng·h/mL to about 638 ng·h/mL, and (d) a median steady state plasma 4-aminopyridine Tₘₐₓ in the range of about 3 h to about 12 h.

20. The method of claim 18;
wherein the amount of 4-aminopyridine in the sustained release tablet is about 16.5 mg, and upon once daily oral administration to a human subject in a fasted state, the sustained release tablet provides one or more of the following pharmacokinetic parameters: (a) a mean steady state plasma 4-aminopyridine Cₘₐₓ in the range of about 13.96 ng/mL to about 27.89 ng/mL, (b) a mean steady state plasma 4-aminopyridine Cₘᵢₙ in the range of about 5.80 ng/mL to about 14.60 ng/mL, (c) a mean steady state plasma 4-aminopyridine AUC₀₋₂₄ in the range of about 243 ng·h/mL to about 479 ng·h/mL, and (d) a median steady state plasma 4-aminopyridine Tₘₐₓ in the range of about 3 h to about 12 h.

21. The method of claim 19 or claim 20:
i) wherein forming the compressed core comprises:
(a) blending the 4-aminopyridine, polyethylene oxide, the mixture comprising polyvinyl acetate and polyvinyl pyrrolidone, and dibasic calcium phosphate dihydrate to form a blended mixture;
(b) adding magnesium stearate to the blended mixture to form a new blend; and
(c) compressing the new blend to form a compressed core; and/or
ii) wherein said mixture consists of about 80% polyvinyl acetate, about 19% polyvinyl pyrrolidone, about 0.8% sodium lauryl sulfate, and about 0.2% silica; and/or
iii) wherein the polyethylene oxide has a molecular weight between 4,000,000 and 8,000,000; and/or
iv) wherein the polyethylene oxide has a molecular weight of 7,000,000; and/or
v) wherein the coating comprises applying an aqueous ethylcellulose dispersion to the compressed core; and/or
vi) wherein the total amount of the 4-aminopyridine in the sustained release tablet is in the range of about 1% w/w to about 10% w/w of the compressed core.

22. The method of claim 19:
wherein the amount of 4-aminopyridine in the compressed core is in the range of about 4% w/w to about 6% w/w of the compressed core; and
wherein the polyethylene oxide in the compressed core has a molecular weight of 7,000,000, wherein the amount of the polyethylene oxide is about 15% w/w of the compressed core; and
wherein the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% of silica, wherein the amount of the mixture is about 25% w/w of the compressed core; and
wherein the amount of dibasic calcium phosphate dihydrate in the compressed core is about 54% w/w of the compressed core; and
wherein the amount of magnesium stearate in the compressed core is about 1% w/w of the compressed core; and
wherein the amount of the ethylcellulose coat is about 9% w/w of the compressed core; and
wherein step c) of the method comprises curing the coated compressed core by exposing it to a temperature in the range of 50-60 °C for a period of time of at least 1 hour.

23. The method of claim 20:
wherein the amount of 4-aminopyridine in the compressed core is in the range of about 3% w/w to about 5% w/w of the compressed core; and
wherein the polyethylene oxide in the compressed core has a molecular weight of 7,000,000, wherein the amount of the polyethylene oxide is about 15% w/w of the compressed core; and
wherein the mixture comprising polyvinyl acetate and polyvinyl pyrrolidone consists of about 80% polyvinyl acetate, about 19% polyvinyl pyrrolidone, about 0.8% sodium lauryl sulfate, and about 0.2% of silica, wherein the amount of the mixture is about 25% w/w of the compressed core; and
wherein the amount of dibasic calcium phosphate dihydrate in the compressed core is about 55% w/w of the compressed core; and
wherein the amount of magnesium stearate in the compressed core is about 1% w/w of the compressed core; and
wherein the amount of the ethylcellulose coat is about 6% w/w of the compressed core; and
wherein step c) of the method comprises curing the coated compressed core by exposing it to a temperature in the range of 50-60 °C for a period of time of at least 1 hour.

24. The method of any of claims 18-23, wherein the sustained release tablet comprises an amount of 4-aminopyridine that is therapeutically effective over a period of 24 hours.

25. The sustained release tablet of any one of claims 1-17 for use in a method of treating a neurological disorder in a patient in need thereof, said method comprising orally administering to the patient once daily the sustained release tablet.

26. The sustained release tablet for use according to claim 25:
i) wherein the neurological disorder is multiple sclerosis or stroke; or
ii) wherein the neurological disorder is multiple sclerosis; or
iii) wherein the neurological disorder is a walking impairment associated with multiple sclerosis; or
iv) wherein the neurological disorder is a neurocognitive or neuropsychiatric impairment associated with multiple sclerosis; or
v) wherein the neurological disorder is stroke; or
vi) wherein the neurological disorder is a sensorimotor impairment associated with stroke; or
vii) wherein the neurological disorder is a walking impairment associated with stroke; and/or
viii) wherein the patient is a human patient.

## Patentansprüche

1. Retardtablette, umfassend:
(a) einen komprimierten Kern, wobei der komprimierte Kern Folgendes umfasst: 4-Aminopyridin, ein Polyethylenoxid mit einem Molekulargewicht zwischen etwa 1.000.000 und 10.000.000 und eine Mischung, die Polyvinylacetat und Polyvinylpyrrolidon umfasst, und
(b) eine Menge eines den komprimierten Kern umgebenden Ethylcelluloseüberzugs;
(b1) wobei die Menge des Ethylcelluloseüberzugs im Bereich von etwa 5 Gew.-% bis etwa 10 Gew.-% des komprimierten Kerns liegt; oder
(b2) wobei das Verhältnis der Menge des Ethylcelluloseüberzugs zur Menge an 4-Aminopyridin in dem komprimierten Kern im Bereich von etwa 0,5:1 bis etwa 3:1 liegt, wobei zur Berechnung des Verhältnisses die Menge des Ethylcelluloseüberzugs der Gewichtsprozentanteil des Ethylcelluloseüberzugs, bezogen auf das Gewicht des komprimierten Kerns, ist und die Menge an 4-Aminopyridin der Gewichtsprozentanteil an 4-Aminopyridin, bezogen auf das Gewicht des komprimierten Kerns, ist; oder
(b3) wobei das Verhältnis der Menge des Ethylcelluloseüberzugs zur Menge an 4-Aminopyridin in dem komprimierten Kern im Bereich von etwa 0,1:1 bis etwa 0,7:1 liegt, wobei zur Berechnung des Verhältnisses die Menge des Ethylcelluloseüberzugs der Gewichtsprozentanteil des Ethylcelluloseüberzugs, bezogen auf das Gewicht des komprimierten Kerns, ist und die Menge an 4-Aminopyridin das Gewicht in Milligramm von 4-Aminopyridin, ist;
wobei die Menge an 4-Aminopyridin in der Retardtablette etwa 22 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 18,61 ng/ml bis etwa 37,19 ng/ml, (b) eine mittlere Steady-State-Plasma-Cₘᵢₙ von 4-Aminopyridin im Bereich von etwa 7,73 ng/ml bis etwa 19,47 ng/ml, (c) eine mittlere Steady-State-Plasma-AUC₀₋₂₄ AUC von 4-Aminopyridin im Bereich von etwa 324 ng·h/ml bis etwa 638 ng·h/ml und (d) einen Medianwert der Steady-State-Plasma-Tₘₐₓ von 4-Aminopyridin im Bereich von etwa 3 h bis etwa 12 h; oder
wobei die Menge an 4-Aminopyridin in der Retardtablette etwa 16,5 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 13,96 ng/ml bis etwa 27,89 ng/ml, (b) eine mittlere Steady-State-Plasma-Cₘᵢₙ von 4-Aminopyridin im Bereich von etwa 5,80 ng/ml bis etwa 14,60 ng/ml, (c) eine mittlere Steady-State-Plasma-AUC₀₋₂₄ AUC von 4-Aminopyridin im Bereich von etwa 243 ng·h/ml bis etwa 479 ng·h/ml und (d) einen Medianwert der Steady-State-Plasma-Tₘₐₓ von 4-Aminopyridin im Bereich von etwa 3 h bis etwa 12 h.

2. Retardtablette nach Anspruch 1, wobei es sich bei (b) um (b1) handelt; und
wobei die Menge an 4-Aminopyridin in der Retardtablette etwa 22 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 18,61 ng/ml bis etwa 37,19 ng/ml, (b) eine mittlere Steady-State-Plasma-Cₘᵢₙ von 4-Aminopyridin im Bereich von etwa 7,73 ng/ml bis etwa 19,47 ng/ml, (c) eine mittlere Steady-State-Plasma-AUC₀₋₂₄ AUC von 4-Aminopyridin im Bereich von etwa 324 ng·h/ml bis etwa 638 ng·h/ml und (d) einen Medianwert der Steady-State-Plasma-Tₘₐₓ von 4-Aminopyridin im Bereich von etwa 3 h bis etwa 12 h.

3. Retardtablette nach Anspruch 1, wobei es sich bei (b) um (b1) handelt; und
wobei die Menge an 4-Aminopyridin in der Retardtablette etwa 16,5 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 13,96 ng/ml bis etwa 27,89 ng/ml, (b) eine mittlere Steady-State-Plasma-Cₘᵢₙ von 4-Aminopyridin im Bereich von etwa 5,80 ng/ml bis etwa 14,60 ng/ml, (c) eine mittlere Steady-State-Plasma-AUC₀₋₂₄ AUC von 4-Aminopyridin im Bereich von etwa 243 ng·h/ml bis etwa 479 ng·h/ml und (d) einen Medianwert der Steady-State-Plasma-Tₘₐₓ von 4-Aminopyridin im Bereich von etwa 3 h bis etwa 12 h.

4. Retardtablette nach Anspruch 2 oder Anspruch 3:
i) wobei die Mischung ferner einen oder mehrere pharmazeutisch unbedenkliche Hilfsstoffe umfasst; und/oder
ii) wobei die Mischung 70-90 % Polyvinylacetat und 15-20 % Polyvinylpyrrolidon umfasst; und/oder
iii) wobei die Mischung ferner ein Tensid umfasst; und/oder
iv) wobei die Mischung ferner Siliciumdioxid umfasst; und/oder
v) wobei die Mischung aus etwa 80 % Polyvinylacetat, etwa 19 % Polyvinylpyrrolidon, etwa 0,8 % Natriumlaurylsulfat und etwa 0,2 % Siliciumdioxid besteht; und/oder
vi) wobei der komprimierte Kern ferner einen Füllstoff und ein Schmiermittel umfasst; und/oder
vii) wobei der komprimierte Kern ferner einen Füllstoff und ein Schmiermittel umfasst und wobei der Füllstoff Calciumhydrogenphosphatdihydrat ist und das Schmiermittel Magnesiumstearat ist; und/oder
viii) wobei das Polyethylenoxid ein Molekulargewicht zwischen 4.000.000 und 8.000.000 aufweist; und/oder
ix) wobei das Polyethylenoxid ein Molekulargewicht von 7.000.000 aufweist; und/oder
x) wobei die Gesamtmenge des 4-Aminopyridins in der Tablette im Bereich von etwa 1 Gew.-% bis etwa 10 Gew.-% der Retardtablette liegt; und/oder
xi) wobei die Gesamtmenge des 4-Aminopyridins in der Tablette im Bereich von etwa 1 Gew.-% bis etwa 10 Gew.-% des komprimierten Kerns liegt.

5. Retardtablette nach Anspruch 2:
wobei die Mischung, die Polyvinylacetat und Polyvinylpyrrolidon umfasst, aus etwa 80 % Polyvinylacetat, etwa 19 % Polyvinylpyrrolidon, etwa 0,8 % Natriumlaurylsulfat und etwa 0,2 % Siliciumdioxid besteht; und
wobei der komprimierte Kern ferner Folgendes umfasst:
Calciumhydrogenphosphatdihydrat und
Magnesiumstearat; und
wobei die Menge an 4-Aminopyridin im Bereich von etwa 4 Gew.-% bis etwa 6 Gew.-% des komprimierten Kerns liegt, wobei die Menge an 4-Aminopyridin gleich 22 mg ist.

6. Retardtablette nach einem der Ansprüche 2-5, wobei die Menge des den komprimierten Kern umgebenden Ethylcelluloseüberzugs etwa 9 Gew.-% des komprimierten Kerns beträgt.

7. Retardtablette Freisetzung nach Anspruch 3, wobei die Menge des den komprimierten Kern umgebenden Ethylcelluloseüberzugs im Bereich von etwa 5 Gew.-% bis etwa 7 Gew.-% des komprimierten Kerns liegt.

8. Retardtablette nach Anspruch 7, wobei die Menge an 4-Aminopyridin im Bereich von etwa 3 Gew.-% bis etwa 5 Gew.-% des komprimierten Kerns liegt und die Menge des den komprimierten Kern umgebenden Ethylcelluloseüberzugs etwa 6 Gew.-% des komprimierten Kerns beträgt.

9. Retardtablette nach Anspruch 2:
wobei die Menge an 4-Aminopyridin in dem komprimierten Kern im Bereich von etwa 4 Gew.-% bis etwa 6 Gew.-% des komprimierten Kerns liegt; und
wobei die Menge des Polyethylenoxids in dem komprimierten Kern im Bereich von etwa 10 Gew.-% bis etwa 20 Gew.-% des komprimierten Kerns liegt; und
wobei die Mischung, die Polyvinylacetat und Polyvinylpyrrolidon umfasst, aus etwa 80 % Polyvinylacetat, etwa 19 % Polyvinylpyrrolidon, etwa 0,8 % Natriumlaurylsulfat und etwa 0,2 % Siliciumdioxid besteht, wobei die Menge der Mischung im Bereich von etwa 20 Gew.-% bis etwa 30 Gew.-% des komprimierten Kerns liegt; und
wobei der komprimierte Kern ferner Folgendes umfasst:
Calciumhydrogenphosphatdihydrat, wobei die Menge an Calciumhydrogenphosphatdihydrat im Bereich von etwa 50 Gew.-% bis etwa 60 Gew.-% des komprimierten Kerns liegt; und
Magnesiumstearat, wobei die Menge an Magnesiumstearat im Bereich von etwa 0,7 Gew.-% bis etwa 1,3 Gew.-% des komprimierten Kerns liegt.

10. Retardtablette nach Anspruch 9:
wobei das Polyethylenoxid in dem komprimierten Kern ein Molekulargewicht von 7.000.000 aufweist, wobei die Menge des Polyethylenoxids etwa 15 Gew.-% des komprimierten Kerns beträgt; und
wobei die Menge der Mischung, die Polyvinylacetat und Polyvinylpyrrolidon umfasst, die aus etwa 80 % Polyvinylacetat, etwa 19 % Polyvinylpyrrolidon, etwa 0,8 % Natriumlaurylsulfat und etwa 0,2 % Siliciumdioxid besteht, etwa 25 Gew.-% des komprimierten Kerns beträgt; und
wobei die Menge an Calciumhydrogenphosphatdihydrat etwa 54 Gew.-% des komprimierten Kerns beträgt; und
wobei die Menge an Magnesiumstearat etwa 1 Gew.-% des komprimierten Kerns beträgt; und
wobei die Menge des den komprimierten Kern umgebenden Ethylcelluloseüberzugs etwa 9 Gew.-% des komprimierten Kerns beträgt.

11. Retardtablette nach Anspruch 7:
wobei die Menge an 4-Aminopyridin in dem komprimierten Kern im Bereich von etwa 3 Gew.-% bis etwa 5 Gew.-% des komprimierten Kerns liegt; und
wobei die Menge des Polyethylenoxids in dem komprimierten Kern im Bereich von etwa 10 Gew.-% bis etwa 20 Gew.-% des komprimierten Kerns liegt; und
wobei die Mischung, die Polyvinylacetat und Polyvinylpyrrolidon umfasst, aus etwa 80 % Polyvinylacetat, etwa 19 % Polyvinylpyrrolidon, etwa 0,8 % Natriumlaurylsulfat und etwa 0,2 % Siliciumdioxid besteht, wobei die Menge der Mischung im Bereich von etwa 20 Gew.-% bis etwa 30 Gew.-% des komprimierten Kerns liegt; und
wobei der komprimierte Kern ferner Folgendes umfasst:
Calciumhydrogenphosphatdihydrat, wobei die Menge an Calciumhydrogenphosphatdihydrat im Bereich von etwa 50 Gew.-% bis etwa 60 Gew.-% des komprimierten Kerns liegt; und
Magnesiumstearat, wobei die Menge an Magnesiumstearat im Bereich von etwa 0,7 Gew.-% bis etwa 1,3 Gew.-% des komprimierten Kerns liegt.

12. Retardtablette nach Anspruch 11:
wobei das Polyethylenoxid in dem komprimierten Kern ein Molekulargewicht von 7.000.000 aufweist, wobei die Menge des Polyethylenoxids etwa 15 Gew.-% des komprimierten Kerns beträgt; und
wobei die Menge der Mischung, die Polyvinylacetat und Polyvinylpyrrolidon umfasst, die aus etwa 80 % Polyvinylacetat, etwa 19 % Polyvinylpyrrolidon, etwa 0,8 % Natriumlaurylsulfat und etwa 0,2 % Siliciumdioxid besteht, etwa 25 Gew.-% des komprimierten Kerns beträgt; und
wobei die Menge an Calciumhydrogenphosphatdihydrat in dem komprimierten Kern etwa 55 Gew.-% des komprimierten Kerns beträgt; und
wobei die Menge an Magnesiumstearat in dem komprimierten Kern etwa 1 Gew.-% des komprimierten Kerns beträgt; und
wobei die Menge des den komprimierten Kern umgebenden Ethylcelluloseüberzugs etwa 6 Gew.-% des komprimierten Kerns beträgt.

13. Retardtablette nach Anspruch 1, wobei es sich bei (b) um (b2) handelt; und
wobei die Menge an 4-Aminopyridin in der Retardtablette etwa 22 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 18,61 ng/ml bis etwa 37,19 ng/ml, (b) eine mittlere Steady-State-Plasma-Cₘᵢₙ von 4-Aminopyridin im Bereich von etwa 7,73 ng/ml bis etwa 19,47 ng/ml, (c) eine mittlere Steady-State-Plasma-AUC₀₋₂₄ AUC von 4-Aminopyridin im Bereich von etwa 324 ng·h/ml bis etwa 638 ng·h/ml und (d) einen Medianwert der Steady-State-Plasma-Tₘₐₓ von 4-Aminopyridin im Bereich von etwa 3 h bis etwa 12 h.

14. Retardtablette nach Anspruch 1, wobei es sich bei (b) um (b2) handelt; und
wobei die Menge an 4-Aminopyridin in der Retardtablette etwa 16,5 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 13,96 ng/ml bis etwa 27,89 ng/ml, (b) eine mittlere Steady-State-Plasma-Cₘᵢₙ von 4-Aminopyridin im Bereich von etwa 5,80 ng/ml bis etwa 14,60 ng/ml, (c) eine mittlere Steady-State-Plasma-AUC₀₋₂₄ AUC von 4-Aminopyridin im Bereich von etwa 243 ng·h/ml bis etwa 479 ng·h/ml und (d) einen Medianwert der Steady-State-Plasma-Tₘₐₓ von 4-Aminopyridin im Bereich von etwa 3 h bis etwa 12 h.

15. Retardtablette nach Anspruch 1, wobei es sich bei (b) um (b3) handelt; und
wobei die Menge an 4-Aminopyridin in der Retardtablette etwa 22 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 18,61 ng/ml bis etwa 37,19 ng/ml, (b) eine mittlere Steady-State-Plasma-Cₘᵢₙ von 4-Aminopyridin im Bereich von etwa 7,73 ng/ml bis etwa 19,47 ng/ml, (c) eine mittlere Steady-State-Plasma-AUC₀₋₂₄ AUC von 4-Aminopyridin im Bereich von etwa 324 ng·h/ml bis etwa 638 ng·h/ml und (d) einen Medianwert der Steady-State-Plasma-Tₘₐₓ von 4-Aminopyridin im Bereich von etwa 3 h bis etwa 12 h.

16. Retardtablette nach Anspruch 1, wobei es sich bei (b) um (b3) handelt; und
wobei die Menge an 4-Aminopyridin in der Retardtablette etwa 16,5 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 13,96 ng/ml bis etwa 27,89 ng/ml, (b) eine mittlere Steady-State-Plasma-Cₘᵢₙ von 4-Aminopyridin im Bereich von etwa 5,80 ng/ml bis etwa 14,60 ng/ml, (c) eine mittlere Steady-State-Plasma-AUC₀₋₂₄ AUC von 4-Aminopyridin im Bereich von etwa 243 ng·h/ml bis etwa 479 ng·h/ml und (d) einen Medianwert der Steady-State-Plasma-Tₘₐₓ von 4-Aminopyridin im Bereich von etwa 3 h bis etwa 12 h.

17. Retardtablette nach einem der Ansprüche 1-16:
i) wobei die Retardtablette das Produkt eines Verfahrens ist, das einen Härtungsschritt nach dem Überziehen des komprimierten Kerns mit Ethylcellulose umfasst, und wobei der Härtungsschritt das Erhitzen des überzogenen komprimierten Kerns auf eine Temperatur über 23 °C über einen Zeitraum von mindestens 15 Minuten umfasst; und gegebenenfalls
wobei der Härtungsschritt das Einwirkenlassen einer Temperatur im Bereich 50-60 °C über einen Zeitraum von mindestens 1 Stunde auf den überzogenen komprimierten Kern umfasst; und/oder
ii) wobei die Retardtablette nicht ferner einen Arzneimitteldecküberzug mit sofortiger Freisetzung, der 4-Aminopyridin enthält, umfasst; und/oder
iii) wobei die Retardtablette eine Freisetzung des 4-Aminopyridins nullter oder nahezu nullter Ordnung bereitstellt; und/oder
iv) wobei die Retardtablette eine Freisetzung des 4-Aminopyridins nullter oder nahezu nullter Ordnung bereitstellt und wobei die Freisetzung nullter Ordnung ist; und/oder
v) wobei die Freisetzung des 4-Aminopyridins nach einem in-vitro-Auflösungstest an der Tablette unter Verwendung von 50 mM Phosphatpuffer, pH 6,8, als Auflösungsmedium wie folgt ist:
innerhalb der ersten 2 Stunden nach Beginn des Tests werden höchstens 30 Gew.-% der Gesamtmenge des in der Retardtablette enthaltenen 4-Aminopyridins freigesetzt, und gegebenenfalls
wobei die Freisetzung des 4-Aminopyridins wie folgt ist: innerhalb der ersten 24 Stunden nach Beginn des Tests werden mindestens 80 Gew.-% der Gesamtmenge des in der Retardtablette enthaltenen 4-Aminopyridins freigesetzt.

18. Verfahren zur Herstellung einer Retardtablette, die 4-Aminopyridin umfasst, wobei das Verfahren Folgendes umfasst:
(a) Bilden eines komprimierten Kerns, umfassend (i) 4-Aminopyridin, (ii) ein Polyethylenoxid mit einem Molekulargewicht zwischen etwa 1.000.000 und 10.000.000, (iii) eine Mischung, die Polyvinylacetat und Polyvinylpyrrolidon umfasst; (iv) Calciumhydrogenphosphatdihydrat und (v) Magnesiumstearat;
(b) Überziehen des komprimierten Kerns mit einer Menge an Ethylcellulose zur Bildung eines überzogenen komprimierten Kerns, wobei die Menge des Ethylcelluloseüberzugs im Bereich von etwa 5 Gew.-% bis etwa 10 Gew.-% des komprimierten Kerns liegt; und
(c) Härten des beschichteten komprimierten Kerns durch Einwirkenlassen einer Temperatur im Bereich von 40-70 °C über einen Zeitraum von mindestens 1 Stunde darauf; wobei die Menge an 4-Aminopyridin in der Retardtablette etwa 22 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 18,61 ng/ml bis etwa 37,19 ng/ml, (b) eine mittlere Steady-State-Plasma-Cₘᵢₙ von 4-Aminopyridin im Bereich von etwa 7,73 ng/ml bis etwa 19,47 ng/ml, (c) eine mittlere Steady-State-Plasma-AUC₀₋₂₄ AUC von 4-Aminopyridin im Bereich von etwa 324 ng·h/ml bis etwa 638 ng·h/ml und (d) einen Medianwert der Steady-State-Plasma-Tₘₐₓ von 4-Aminopyridin im Bereich von etwa 3 h bis etwa 12 h; oder
wobei die Menge an 4-Aminopyridin in der Retardtablette etwa 16,5 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 13,96 ng/ml bis etwa 27,89 ng/ml, (b) eine mittlere Steady-State-Plasma-Cₘᵢₙ von 4-Aminopyridin im Bereich von etwa 5,80 ng/ml bis etwa 14,60 ng/ml, (c) eine mittlere Steady-State-Plasma-AUC₀₋₂₄ AUC von 4-Aminopyridin im Bereich von etwa 243 ng·h/ml bis etwa 479 ng·h/ml und (d) einen Medianwert der Steady-State-Plasma-Tₘₐₓ von 4-Aminopyridin im Bereich von etwa 3 h bis etwa 12 h.

19. Verfahren nach Anspruch 18,
wobei die Menge an 4-Aminopyridin in der Retardtablette etwa 22 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 18,61 ng/ml bis etwa 37,19 ng/ml, (b) eine mittlere Steady-State-Plasma-Cₘᵢₙ von 4-Aminopyridin im Bereich von etwa 7,73 ng/ml bis etwa 19,47 ng/ml, (c) eine mittlere Steady-State-Plasma-AUC₀₋₂₄ AUC von 4-Aminopyridin im Bereich von etwa 324 ng·h/ml bis etwa 638 ng·h/ml und (d) einen Medianwert der Steady-State-Plasma-Tₘₐₓ von 4-Aminopyridin im Bereich von etwa 3 h bis etwa 12 h.

20. Verfahren nach Anspruch 18,
wobei die Menge an 4-Aminopyridin in der Retardtablette etwa 16,5 mg beträgt und die Retardtablette nach einmal täglicher oraler Verabreichung an ein menschliches Individuum in nüchternem Zustand einen oder mehrere der folgenden pharmakokinetischen Parameter bereitstellt: (a) eine mittlere Steady-State-Plasma-Cₘₐₓ von 4-Aminopyridin im Bereich von etwa 13,96 ng/ml bis etwa 27,89 ng/ml, (b) eine mittlere Steady-State-Plasma-Cₘᵢₙ von 4-Aminopyridin im Bereich von etwa 5,80 ng/ml bis etwa 14,60 ng/ml, (c) eine mittlere Steady-State-Plasma-AUC₀₋₂₄ AUC von 4-Aminopyridin im Bereich von etwa 243 ng·h/ml bis etwa 479 ng·h/ml und (d) einen Medianwert der Steady-State-Plasma-Tₘₐₓ von 4-Aminopyridin im Bereich von etwa 3 h bis etwa 12 h.

21. Verfahren nach Anspruch 19 oder Anspruch 20:
i) wobei das Bilden des komprimierten Kerns Folgendes umfasst:
(a) Mischen des 4-Aminopyridins, des Polyethylenoxids, der Mischung, die Polyvinylacetat und Polyvinylpyrrolidon umfasst, und des Calciumhydrogenphosphatdihydrats zur Bildung einer gemischten Mischung;
(b) Zugeben von Magnesiumstearat zu der gemischten Mischung zur Bildung einer neuen Mischung; und
(c) Komprimieren der neuen Mischung zur Bildung eines komprimierten Kerns; und/oder
ii) wobei die Mischung aus etwa 80 % Polyvinylacetat, etwa 19 % Polyvinylpyrrolidon, etwa 0,8 % Natriumlaurylsulfat und etwa 0,2 % Siliciumdioxid besteht; und/oder
iii) wobei das Polyethylenoxid ein Molekulargewicht zwischen 4.000.000 und 8.000.000 aufweist; und/oder
iv) wobei das Polyethylenoxid ein Molekulargewicht von 7.000.000 aufweist; und/oder
v) wobei das Überziehen das Aufbringen einer wässrigen Ethylcellulosedispersion auf den komprimierten Kern umfasst; und/oder
vi) wobei die Gesamtmenge des 4-Aminopyridins in der Retardtablette im Bereich von etwa 1 Gew.-% bis etwa 10 Gew.-% des komprimierten Kerns liegt.

22. Verfahren nach Anspruch 19:
wobei die Menge an 4-Aminopyridin in dem komprimierten Kern im Bereich von etwa 4 Gew.-% bis etwa 6 Gew.-% des komprimierten Kerns liegt; und
wobei das Polyethylenoxid in dem komprimierten Kern ein Molekulargewicht von 7.000.000 aufweist, wobei die Menge des Polyethylenoxids etwa 15 Gew.-% des komprimierten Kerns beträgt; und
wobei die Mischung, die Polyvinylacetat und Polyvinylpyrrolidon umfasst, aus etwa 80 % Polyvinylacetat, etwa 19 % Polyvinylpyrrolidon, etwa 0,8 % Natriumlaurylsulfat und etwa 0,2 % Siliciumdioxid besteht, wobei die Menge der Mischung etwa 25 Gew.-% des komprimierten Kerns beträgt; und
wobei die Menge an Calciumhydrogenphosphatdihydrat in dem komprimierten Kern etwa 54 Gew.-% des komprimierten Kerns beträgt; und
wobei die Menge an Magnesiumstearat in dem komprimierten Kern etwa 1 Gew.-% des komprimierten Kerns beträgt; und
Wobei die Menge des Ethylcelluloseüberzugs etwa 9 Gew.-% des komprimierten Kerns beträgt; und
wobei Schritt c) des Verfahrens das Härten des überzogenen komprimierten Kerns durch Einwirkenlassen einer Temperatur im Bereich von 50-60 °C darauf über einen Zeitraum von mindestens 1 Stunde umfasst.

23. Verfahren nach Anspruch 20:
wobei die Menge an 4-Aminopyridin in dem komprimierten Kern im Bereich von etwa 3 Gew.-% bis etwa 5 Gew.-% des komprimierten Kerns liegt; und
wobei das Polyethylenoxid in dem komprimierten Kern ein Molekulargewicht von 7.000.000 aufweist, wobei die Menge des Polyethylenoxids etwa 15 Gew.-% des komprimierten Kerns beträgt; und
wobei die Mischung, die Polyvinylacetat und Polyvinylpyrrolidon umfasst, aus etwa 80 % Polyvinylacetat, etwa 19 % Polyvinylpyrrolidon, etwa 0,8 % Natriumlaurylsulfat und etwa 0,2 % Siliciumdioxid besteht, wobei die Menge der Mischung etwa 25 Gew.-% des komprimierten Kerns beträgt; und
wobei die Menge an Calciumhydrogenphosphatdihydrat in dem komprimierten Kern etwa 55 Gew.-% des komprimierten Kerns beträgt; und
wobei die Menge an Magnesiumstearat in dem komprimierten Kern etwa 1 Gew.-% des komprimierten Kerns beträgt; und
wobei die Menge des Ethylcelluloseüberzugs etwa 6 Gew.-% des komprimierten Kerns beträgt; und
wobei Schritt c) des Verfahrens das Härten des überzogenen komprimierten Kerns durch Einwirkenlassen einer Temperatur im Bereich von 50-60 °C darauf über einen Zeitraum von mindestens 1 Stunde umfasst.

24. Verfahren nach einem der Ansprüche 18-23, wobei die Retardtablette eine Menge an 4-Aminopyridin umfasst, die über einen Zeitraum von 24 Stunden therapeutisch wirksam ist.

25. Retardtablette nach einem der Ansprüche 1-17 zur Verwendung in einem Verfahren zur Behandlung einer neurologischen Störung bei einem Patienten, bei dem diesbezüglicher Bedarf besteht, wobei das Verfahren die orale Verabreichung der Retardtablette einmal täglich an den Patienten umfasst.

26. Retardtablette zur Verwendung nach Anspruch 25:
i) wobei die neurologische Störung multiple Sklerose oder Schlaganfall ist; oder
ii) wobei die neurologische Störung multiple Sklerose ist; oder
iii) wobei die neurologische Störung eine mit multipler Sklerose assoziierte Gehbehinderung ist; oder
iv) wobei die neurologische Störung eine mit multipler Sklerose assoziierte neurokognitive oder neuropsychiatrische Beeinträchtigung ist; oder
v) wobei die neurologische Störung Schlaganfall ist; oder
vi) wobei die neurologische Störung eine mit einem Schlaganfall assoziierte sensomotorische Beeinträchtigung ist; oder
vii) wobei die neurologische Störung eine mit Schlaganfall assoziierte Gehbehinderung ist; und/oder
viii) wobei der Patient ein menschlicher Patient ist.

## Revendications

1. Comprimé à libération prolongée comprenant :
(a) un noyau comprimé, ledit noyau comprimé comprenant de la 4-aminopyridine, un poly(oxyde d'éthylène) ayant un poids moléculaire compris entre environ 1 000 000 et 10 000 000, et un mélange comprenant un poly(acétate de vinyle) et une polyvinylpyrrolidone, et
(b) une quantité d'un revêtement d'éthylcellulose entourant ledit noyau comprimé ;
(b1) dans lequel ladite quantité du revêtement d'éthylcellulose est dans la plage d'environ 5 % p/p à environ 10 % p/p du noyau comprimé ; ou
(b2) dans lequel le rapport de la quantité du revêtement d'éthylcellulose sur la quantité de 4-aminopyridine dans le noyau comprimé est dans la plage d'environ 0,5:1 à environ 3:1, dans lequel pour calculer ledit rapport, la quantité du revêtement d'éthylcellulose est le pourcentage en poids du revêtement d'éthylcellulose par poids du noyau comprimé, et la quantité de 4-aminopyridine est le pourcentage en poids de 4-aminopyridine en poids du noyau comprimé ; ou
(b3) dans lequel le rapport de la quantité du revêtement d'éthylcellulose sur la quantité de 4-aminopyridine dans le noyau comprimé se situe dans la plage d'environ 0,1:1 à environ 0,7:1, dans lequel pour calculer ledit rapport, la quantité du revêtement d'éthylcellulose est le pourcentage en poids du revêtement d'éthylcellulose en poids du noyau comprimé, et la quantité de 4-aminopyridine est le poids en milligrammes de 4-aminopyridine ;
la quantité de 4-aminopyridine dans le comprimé à libération prolongée étant d'environ 22 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 18,61 ng/mL à environ 37,19 ng/ml, (b) un Cₘᵢₙ de 4-aminopyridine plasmatique moyenne à l'état d'équilibre dans la plage d'environ 7,73 ng/mL à environ 19,47 ng/mL, (c) un AUC₀₋₂₄ de 4-aminopyridine plasmatique moyenne à l'état d'équilibre dans la plage d'environ 324 ng·h/mL à environ 638 ng·h/mL, et (d) une Tₘₐₓ de 4-aminopyridine plasmatique médiane à l'état d'équilibre dans la plage d'environ 3 h à environ 12 h ; ou
la quantité de 4-aminopyridine dans le comprimé à libération prolongée étant d'environ 16,5 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 13,96 ng/mL à environ 27,89 ng/mL, (b) un Cₘᵢₙ de 4-aminopyridine plasmatique moyenne à l'état d'équilibre dans la plage d'environ 5,80 ng/mL à environ 14,60 ng/mL, (c) un AUC₀₋₂₄ de 4-aminopyridine plasmatique moyenne à l'état d'équilibre dans la plage d'environ 243 ng·h/mL à environ 479 ng·h/mL, et (d) une Tₘₐₓ de 4-aminopyridine plasmatique médiane à l'état d'équilibre dans la plage d'environ 3 h à environ 12 h.

2. Comprimé à libération prolongée selon la revendication 1, dans lequel (b) est (b1) ; et
la quantité de 4-aminopyridine dans le comprimé à libération prolongée étant d'environ 22 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 18,61 ng/mL à environ 37,19 ng/mL, (b) un Cₘᵢₙ de 4-aminopyridine plasmatique moyenne à l'état d'équilibre dans la plage d'environ 7,73 ng/mL à environ 19,47 ng/mL, (c) un AUC₀₋₂₄ de 4-aminopyridine plasmatique moyenne à l'état d'équilibre dans la plage d'environ 324 ng·h/mL à environ 638 ng·h/mL, et (d) une Tₘₐₓ de 4-aminopyridine plasmatique médiane à l'état d'équilibre dans la plage d'environ 3 h à environ 12 h.

3. Comprimé à libération prolongée selon la revendication 1, dans lequel (b) est (b1) ; et
la quantité de 4-aminopyridine dans le comprimé à libération prolongée étant d'environ 16,5 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 13,96 ng/mL à environ 27,89 ng/mL, (b) un Cₘᵢₙ de 4-aminopyridine plasmatique moyenne à l'état d'équilibre dans la plage d'environ 5,80 ng/mL à environ 14,60 ng/mL, (c) un AUC₀₋₂₄ de 4-aminopyridine plasmatique moyenne à l'état d'équilibre dans la plage d'environ 243 ng·h/mL à environ 479 ng·h/mL, et (d) une Tₘₐₓ de 4-aminopyridine plasmatique médiane à l'état d'équilibre dans la plage d'environ 3 h à environ 12 h.

4. Comprimé à libération prolongée selon la revendication 2 ou la revendication 3 :
i) dans lequel ledit mélange comprend en outre un ou plusieurs excipients pharmaceutiquement acceptables ; et/ou
ii) dans lequel ledit mélange comprend 70-90 % de poly(acétate de vinyle) et 15-20 % de poly(pyrrolidone) ; et/ou
iii) dans lequel ledit mélange comprend en outre un tensioactif ; et/ou
iv) dans lequel ledit mélange comprend en outre une silice ; et/ou
v) dans lequel ledit mélange est constitué d'environ 80 % de poly(acétate de vinyle), d'environ 19 % de polyvinylpyrrolidone, d'environ 0,8 % de laurylsulfate de sodium, et d'environ 0,2 % de silice ; et/ou
vi) dans lequel le noyau comprimé comprend en outre une charge et un lubrifiant ; et/ou
vii) dans lequel le noyau comprimé comprend en outre une charge et un lubrifiant et la charge étant du dihydrate de phosphate de calcium dibasique, et le lubrifiant étant le stéarate de magnésium ; et/ou
viii) dans lequel le poly(oxyde d'éthylène) possède un poids moléculaire compris entre 4 000 000 et 8 000 000 ; et/ou
ix) dans lequel le poly(oxyde d'éthylène) possède un poids moléculaire de 7 000 000 ; et/ou
x) dans lequel la quantité totale de la 4-aminopyridine dans le comprimé est dans la plage d'environ 1 % p/p à environ 10 % p/p du comprimé à libération prolongée ; et/ou
xi) dans lequel la quantité totale de la 4-aminopyridine dans le comprimé est dans la plage d'environ 1 % p/p à environ 10 % p/p du noyau comprimé.

5. Comprimé à libération prolongée selon la revendication 2 :
dans lequel le mélange comprenant un poly(acétate de vinyle) et une poly(vinylpyrrolidone) est constitué d'environ 80 % de poly(acétate de vinyle), d'environ 19 % de poly(vinylpyrrolidone), d'environ 0,8 % de laurylsulfate de sodium, et d'environ 0,2 % de silice ; et
dans lequel le noyau comprimé comprend en outre :
du dihydrate de phosphate de calcium dibasique, et
du stéarate de magnésium ; et
dans lequel la quantité de 4-aminopyridine est dans la plage d'environ 4 % p/p à environ 6 % p/p du noyau comprimé, ladite quantité de 4-aminopyridine étant égale à 22 mg.

6. Comprimé à libération prolongée selon l'une quelconque des revendications 2 à 5, dans lequel la quantité du revêtement d'éthylcellulose entourant le noyau comprimé est d'environ 9 % p/p du noyau comprimé.

7. Comprimé à libération prolongée selon la revendication 3, dans lequel la quantité du revêtement d'éthylcellulose entourant ledit noyau comprimé est dans la plage d'environ 5 % p/p à environ 7 % p/p du noyau comprimé.

8. Comprimé à libération prolongée selon la revendication 7, dans lequel la quantité de 4-aminopyridine est dans la plage d'environ 3 % p/p à environ 5 % p/p du noyau comprimé et la quantité du revêtement d'éthylcellulose entourant le noyau comprimé est d'environ 6 % p/p du noyau comprimé.

9. Comprimé à libération prolongée selon la revendication 2 :
dans lequel la quantité de 4-aminopyridine dans le noyau comprimé est dans la plage d'environ 4 % p/p à environ 6 % p/p du noyau comprimé ; et
dans lequel la quantité du poly(oxyde d'éthylène) dans le noyau comprimé est dans la plage d'environ 10 % p/p à environ 20 % p/p du noyau comprimé ; et
dans lequel le mélange comprenant un poly(acétate de vinyle) et une poly(pyrrolidone de vinyle) est constitué d'environ 80 % de poly(acétate de vinyle), d'environ 19 % de poly(pyrrolidone de vinyle), d'environ 0,8 % de laurylsulfate de sodium, et d'environ 0,2 % de silice, la quantité du mélange étant dans la plage d'environ 20 % p/p à environ 30 % p/p du noyau comprimé ; et
dans lequel le noyau comprimé comprend en outre :
du dihydrate de phosphate de calcium dibasique, dans lequel la quantité de dihydrate de phosphate de calcium dibasique est dans la plage d'environ 50 % p/p à environ 60 % p/p du noyau comprimé ; et
du stéarate de magnésium, dans lequel la quantité de stéarate de magnésium est dans la plage d'environ 0,7 % p/p à environ 1,3 % p/p du noyau comprimé.

10. Comprimé à libération prolongée selon la revendication 9 :
dans lequel le poly(oxyde d'éthylène) dans le noyau comprimé a un poids moléculaire de 7 000 000, dans lequel la quantité du poly(oxyde d'éthylène) est d'environ 15 % p/p du noyau comprimé ; et
dans lequel la quantité du mélange comprenant un poly(acétate de vinyle) et une poly(pyrrolidone de vinyle) constitué d'environ 80 % de poly(acétate de vinyle), d'environ 19 % de poly(pyrrolidone de vinyle), d'environ 0,8 % de lauryl sulfate de sodium, et d'environ 0,2 % de silice est d'environ 25 % p/p du noyau comprimé ; et
dans lequel la quantité de dihydrate de phosphate de calcium dibasique est d'environ 54 % p/p du noyau comprimé ; et
dans lequel la quantité de stéarate de magnésium est d'environ 1 % p/p du noyau comprimé ; et
dans lequel la quantité du revêtement d'éthylcellulose entourant ledit noyau comprimé est d'environ 9 % p/p du noyau comprimé.

11. Comprimé à libération prolongée selon la revendication 7 :
dans lequel la quantité de 4-aminopyridine dans le noyau comprimé est dans la plage d'environ 3 % p/p à environ 5 % p/p du noyau comprimé ; et
dans lequel la quantité du poly(oxyde d'éthylène) dans le noyau comprimé est dans la plage d'environ 10 % p/p à environ 20 % p/p du noyau comprimé ; et
dans lequel le mélange comprenant un poly(acétate de vinyle) et une poly(pyrrolidone de vinyle) est constitué d'environ 80 % de poly(acétate de vinyle), d'environ 19 % de poly(pyrrolidone de vinyle), d'environ 0,8 % de laurylsulfate de sodium, et d'environ 0,2 % de silice, la quantité du mélange étant dans la plage d'environ 20 % p/p à environ 30 % p/p du noyau comprimé ; et
dans lequel le noyau comprimé comprend en outre :
du dihydrate de phosphate de calcium dibasique, dans lequel la quantité de dihydrate de phosphate de calcium dibasique est dans la plage d'environ 50 % p/p à environ 60 % p/p du noyau comprimé ; et
du stéarate de magnésium, dans lequel la quantité de stéarate de magnésium est dans la plage d'environ 0,7 % p/p à environ 1,3 % p/p du noyau comprimé.

12. Comprimé à libération prolongée selon la revendication 11 :
dans lequel le poly(oxyde d'éthylène) dans le noyau comprimé a un poids moléculaire de 7 000 000, dans lequel la quantité du poly(oxyde d'éthylène) est d'environ 15 % p/p du noyau comprimé ; et
dans lequel la quantité du mélange comprenant un poly(acétate de vinyle) et une poly(pyrrolidone de vinyle) constitué d'environ 80 % de poly(acétate de vinyle), d'environ 19 % de poly(pyrrolidone de vinyle), d'environ 0,8 % de lauryl sulfate de sodium, et d'environ 0,2 % de silice est d'environ 25 % p/p du noyau comprimé ; et
dans lequel la quantité de dihydrate de phosphate de calcium dibasique dans le noyau comprimé est d'environ 55 % p/p du noyau comprimé ; et
dans lequel la quantité de stéarate de magnésium dans le noyau comprimé est d'environ 1 % p/p du noyau comprimé ; et
dans lequel la quantité du revêtement d'éthylcellulose entourant ledit noyau comprimé est d'environ 6 % p/p du noyau comprimé.

13. Comprimé à libération prolongée selon la revendication 1, dans lequel (b) est (b2) ; et
dans lequel la quantité de 4-aminopyridine dans le comprimé à libération prolongée est d'environ 22 mg ; et lors d'administration orale une fois par jour à un sujet humain à jeun, le comprimé à libération prolongée fournit l'un ou plusieurs des paramètres pharmacocinétiques suivants : (a) un Cₘₐₓ de 4-aminopyridine plasmatique moyenne à l'état d'équilibre dans la plage d'environ 18,61 ng/mL à environ 37,19 ng/mL, (b) un Cₘᵢₙ de 4-aminopyridine plasmatique moyenne à l'état d'équilibre dans la plage d'environ 7,73 ng/mL à environ 19,47 ng/mL, (c) un AUC₀₋₂₄ de 4-aminopyridine plasmatique moyenne à l'état d'équilibre dans la plage d'environ 324 ng·h/mL à environ 638 ng·h/mL, et (d) une Tₘₐₓ de 4-aminopyridine plasmatique médiane à l'état d'équilibre dans la plage d'environ 3 h à environ 12 h.

14. Comprimé à libération prolongée selon la revendication 1, dans lequel (b) est (b2) ; et
dans lequel la quantité de 4-aminopyridine dans le comprimé à libération prolongée est d'environ 16,5 mg ; et lors d'administration orale une fois par jour à un sujet humain à jeun, le comprimé à libération prolongée fournit l'un ou plusieurs des paramètres pharmacocinétiques suivants : (a) un Cₘₐₓ de 4-aminopyridine plasmatique moyenne à l'état d'équilibre dans la plage d'environ 13,96 ng/mL à environ 27,89 ng/mL, (b) un Cₘᵢₙ de 4-aminopyridine plasmatique moyenne à l'état d'équilibre dans la plage d'environ 5,80 ng/mL à environ 14,60 ng/mL, (c) un AUC₀₋₂₄ de 4-aminopyridine plasmatique moyenne à l'état d'équilibre dans la plage d'environ 243 ng·h/mL à environ 479 ng·h/mL, et (d) une Tₘₐₓ de 4-aminopyridine plasmatique médiane à l'état d'équilibre dans la plage d'environ 3 h à environ 12 h.

15. Comprimé à libération prolongée selon la revendication 1, dans lequel (b) est (b3) ; et
dans lequel la quantité de 4-aminopyridine dans le comprimé à libération prolongée est d'environ 22 mg ; et lors d'administration orale une fois par jour à un sujet humain à jeun, le comprimé à libération prolongée fournit l'un ou plusieurs des paramètres pharmacocinétiques suivants : (a) un Cₘₐₓ de 4-aminopyridine plasmatique moyenne à l'état d'équilibre dans la plage d'environ 18,61 ng/mL à environ 37,19 ng/mL, (b) un Cₘᵢₙ de 4-aminopyridine plasmatique moyenne à l'état d'équilibre dans la plage d'environ 7,73 ng/mL à environ 19,47 ng/mL, (c) un AUC₀₋₂₄ de 4-aminopyridine plasmatique moyenne à l'état d'équilibre dans la plage d'environ 324 ng·h/mL à environ 638 ng·h/mL, et (d) une Tₘₐₓ de 4-aminopyridine plasmatique médiane à l'état d'équilibre dans la plage d'environ 3 h à environ 12 h.

16. Comprimé à libération prolongée selon la revendication 1, dans lequel (b) est (b3) ; et
dans lequel la quantité de 4-aminopyridine dans le comprimé à libération prolongée est d'environ 16,5 mg ; et lors d'administration orale une fois par jour à un sujet humain à jeun, le comprimé à libération prolongée fournit l'un ou plusieurs des paramètres pharmacocinétiques suivants : (a) un Cₘₐₓ de 4-aminopyridine plasmatique moyenne à l'état d'équilibre dans la plage d'environ 13,96 ng/mL à environ 27,89 ng/mL, (b) un Cₘᵢₙ de 4-aminopyridine plasmatique moyenne à l'état d'équilibre dans la plage d'environ 5,80 ng/mL à environ 14,60 ng/mL, (c) un AUC₀₋₂₄ de 4-aminopyridine plasmatique moyenne à l'état d'équilibre dans la plage d'environ 243 ng·h/mL à environ 479 ng·h/mL, et (d) une Tₘₐₓ de 4-aminopyridine plasmatique médiane à l'état d'équilibre dans la plage d'environ 3 h à environ 12 h.

17. Comprimé à libération prolongée selon l'une quelconque des revendications 1 à 16 :
i) dans lequel le comprimé à libération prolongée est le produit d'un procédé comprenant une étape de durcissement après revêtement du noyau comprimé avec de l'éthylcellulose, et dans lequel ladite étape de durcissement comprend un chauffage du noyau comprimé revêtu jusqu'à une température supérieure à 23 °C pendant une période de temps d'au moins 15 minutes ; et éventuellement
dans lequel ladite étape de durcissement comprend l'exposition du noyau comprimé revêtu à une température dans la plage de 50-60 °C pendant une période de temps d'au moins 1 heure ; et/ou
ii) dans lequel le comprimé à libération prolongée ne comprend pas en outre une surcouche de médicament à libération immédiate contenant de la 4-aminopyridine ; et/ou
iii) dans lequel le comprimé à libération prolongée fourni une libération d'ordre zéro ou d'ordre proche de zéro de la 4-aminopyridine ; et/ou
iv) dans lequel le comprimé à libération prolongée produit une libération d'ordre zéro ou d'ordre proche de zéro de la 4-aminopyridine et dans lequel la libération est d'ordre zéro ; et/ou
v) dans lequel la libération de la 4-aminopyridine, après soumission du comprimé à un test de dissolution *in vitro* en utilisant du tampon phosphate 50 mM, pH 6,8 en tant que milieu de dissolution, est comme suit :
dans les 2 premières heures après le début du test, au plus 30 % p/p de la quantité totale de la 4-aminopyridine contenue dans le comprimé à libération prolongée est libérée, et éventuellement
dans lequel la libération de la 4-aminopyridine est comme suit :
dans les 24 premières heures après le début du test, au moins 80 % p/p de la quantité totale de la 4-aminopyridine contenue dans le comprimé à libération prolongée est libérée.

18. Procédé de préparation d'un comprimé à libération prolongée comprenant de la 4-aminopyridine, ledit procédé comprenant :
(a) la formation d'un noyau comprimé comprenant (i) de la 4-aminopyridine, (ii) un poly(oxyde d'éthylène) ayant un poids moléculaire compris entre environ 1 000 000 et 10 000 000, (iii) un mélange comprenant un poly(acétate de vinyle) et une polyvinylpyrrolidone ; (iv) du dihydrate de phosphate de calcium dibasique, et (v) du stéarate de magnésium ;
(b) le revêtement du noyau comprimé avec une quantité d'éthylcellulose pour former un noyau comprimé revêtu, ladite quantité du revêtement d'éthylcellulose étant dans la plage d'environ 5 % p/p à environ 10 % p/p du noyau comprimé ; et
(c) le durcissement du noyau comprimé revêtu en l'exposant à une température dans la plage de 40-70 °C pendant une période de temps d'au moins 1 heure ;
la quantité de 4-aminopyridine dans le comprimé à libération prolongée étant d'environ 22 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 18,61 ng/mL à environ 37,19 ng/mL, (b) un Cₘᵢₙ de 4-aminopyridine plasmatique moyenne à l'état d'équilibre dans la plage d'environ 7,73 ng/mL à environ 19,47 ng/mL, (c) un AUC₀₋₂₄ de 4-aminopyridine plasmatique moyenne à l'état d'équilibre dans la plage d'environ 324 ng·h/mL à environ 638 ng·h/mL, et (d) une Tₘₐₓ de 4-aminopyridine plasmatique médiane à l'état d'équilibre dans la plage d'environ 3 h à environ 12 h ; ou
la quantité de 4-aminopyridine dans le comprimé à libération prolongée étant d'environ 16,5 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 13,96 ng/mL à environ 27,89 ng/mL, (b) un Cₘᵢₙ de 4-aminopyridine plasmatique moyenne à l'état d'équilibre dans la plage d'environ 5,80 ng/mL à environ 14,60 ng/mL, (c) un AUC₀₋₂₄ de 4-aminopyridine plasmatique moyenne à l'état d'équilibre dans la plage d'environ 243 ng·h/mL à environ 479 ng·h/mL, et (d) une Tₘₐₓ de 4-aminopyridine plasmatique médiane à l'état d'équilibre dans la plage d'environ 3 h à environ 12 h.

19. Procédé selon la revendication 18 :
la quantité de 4-aminopyridine dans le comprimé à libération prolongée étant d'environ 22 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 18,61 ng/mL à environ 37,19 ng/mL, (b) un Cₘᵢₙ de 4-aminopyridine plasmatique moyenne à l'état d'équilibre dans la plage d'environ 7,73 ng/mL à environ 19,47 ng/mL, (c) un AUC₀₋₂₄ de 4-aminopyridine plasmatique moyenne à l'état d'équilibre dans la plage d'environ 324 ng·h/mL à environ 638 ng·h/mL, et (d) une Tₘₐₓ de 4-aminopyridine plasmatique médiane à l'état d'équilibre dans la plage d'environ 3 h à environ 12 h.

20. Procédé selon la revendication 18 :
la quantité de 4-aminopyridine dans le comprimé à libération prolongée étant d'environ 16,5 mg, et lors d'administration orale une fois par jour à un sujet humain à jeun, le comprimé à libération prolongée fournissant l'un ou plusieurs des paramètres pharmacocinétiques suivants : (a) un Cₘₐₓ de 4-aminopyridine plasmatique moyenne à l'état d'équilibre dans la plage d'environ 13,96 ng/mL à environ 27,89 ng/mL, (b) un Cₘᵢₙ de 4-aminopyridine plasmatique moyenne à l'état d'équilibre dans la plage d'environ 5,80 ng/mL à environ 14,60 ng/mL, (c) un AUC₀₋₂₄ de 4-aminopyridine plasmatique moyenne à l'état d'équilibre dans la plage d'environ 243 ng·h/mL à environ 479 ng·h/mL, et (d) une Tₘₐₓ de 4-aminopyridine plasmatique médiane à l'état d'équilibre dans la plage d'environ 3 h à environ 12 h.

21. Procédé selon la revendication 19 ou ou la revendication 20 :
i) dans lequel la mise en forme du noyau comprimé comprend :
(a) le mélange de la 4-aminopyridine, d'un poly(oxyde d'éthylène), du mélange comprenant un poly(acétate de vinyle) et une polyvinylpyrrolidone, et du dihydrate de phosphate de calcium dibasique pour former un mélange mélangé ;
(b) l'ajout de stéarate de magnésium au mélange mélangé pour former un nouveau mélange ; et
(c) la compression du nouveau mélange pour former un noyau comprimé ; et/ou
ii) dans lequel ledit mélange est constitué d'environ 80 % de poly(acétate de vinyle), d'environ 19 % de polyvinylpyrrolidone, d'environ 0,8 % de laurylsulfate de sodium, et d'environ 0,2 % de silice ; et/ou
iii) dans lequel le poly(oxyde d'éthylène) possède un poids moléculaire compris entre 4 000 000 et 8 000 000 ; et/ou
iv) dans lequel le poly(oxyde d'éthylène) possède un poids moléculaire de 7 000 000 ; et/ou
v) dans lequel le revêtement comprend l'application d'une dispersion aqueuse d'éthylcellulose sur le noyau comprimé ; et/ou
vi) dans lequel la quantité totale de la 4-aminopyridine dans le comprimé à la libération prolongée est dans la plage d'environ 1 % p/p à environ 10 % p/p du noyau comprimé.

22. Procédé selon la revendication 19 :
dans lequel la quantité de 4-aminopyridine dans le noyau comprimé est dans la plage d'environ 4 % p/p à environ 6 % p/p du noyau comprimé ; et
dans lequel le poly(oxyde d'éthylène) dans le noyau comprimé a un poids moléculaire de 7 000 000, dans lequel la quantité du poly(oxyde d'éthylène) est d'environ 15 % p/p du noyau comprimé ; et
dans lequel le mélange comprenant un poly(acétate de vinyle) et une poly(pyrrolidone de vinyle) est constitué d'environ 80 % de poly(acétate de vinyle), d'environ 19 % de poly(pyrrolidone de vinyle), d'environ 0,8 % de laurylsulfate de sodium, et d'environ 0,2 % de silice, la quantité du mélange étant d'environ 25 % p/p du noyau comprimé ; et
dans lequel la quantité de dihydrate de phosphate de calcium dibasique dans le noyau comprimé est d'environ 54 % p/p du noyau comprimé ; et
dans lequel la quantité de stéarate de magnésium dans le noyau comprimé est d'environ 1 % p/p du noyau comprimé ; et
dans lequel la quantité du revêtement d'éthylcellulose est d'environ 9 % p/p du noyau comprimé ; et
dans lequel l'étape c) du procédé comprend le durcissement du noyau comprimé revêtu en l'exposant à une température dans la plage de 50-60 °C pendant une période de temps d'au moins 1 heure.

23. Procédé selon la revendication 20 :
dans lequel la quantité de 4-aminopyridine dans le noyau comprimé est dans la plage d'environ 3 % p/p à environ 5 % p/p du noyau comprimé ; et
dans lequel le poly(oxyde d'éthylène) dans le noyau comprimé a un poids moléculaire de 7 000 000, dans lequel la quantité du poly(oxyde d'éthylène) est d'environ 15 % p/p du noyau comprimé ; et
dans lequel le mélange comprenant un poly(acétate de vinyle) et une poly(pyrrolidone de vinyle) est constitué d'environ 80 % de poly(acétate de vinyle), d'environ 19 % de poly(pyrrolidone de vinyle), d'environ 0,8 % de laurylsulfate de sodium, et d'environ 0,2 % de silice, la quantité du mélange étant d'environ 25 % p/p du noyau comprimé ; et
dans lequel la quantité de dihydrate de phosphate de calcium dibasique dans le noyau comprimé est d'environ 55 % p/p du noyau comprimé ; et
dans lequel la quantité de stéarate de magnésium dans le noyau comprimé est d'environ 1 % p/p du noyau comprimé ; et
dans lequel la quantité du revêtement d'éthylcellulose est d'environ 6 % p/p du noyau comprimé ; et
dans lequel l'étape c) du procédé comprend le durcissement du noyau comprimé revêtu en l'exposant à une température dans la plage de 50-60 °C pendant une période de temps d'au moins 1 heure.

24. Procédé selon l'une quelconque des revendications 18 à 23, dans lequel le comprimé à libération prolongée comprend une quantité de 4-aminopyridine qui est thérapeutiquement efficace sur une période de 24 heures.

25. Comprimé à libération prolongée selon l'une quelconque des revendications 1 à 17 destiné à être utilisé dans un procédé de traitement d'un trouble neurologique chez un patient qui en a besoin, ledit procédé comprenant l'administration orale au patient une fois par jour du comprimé à libération prolongée.

26. Comprimé à libération prolongée pour une utilisation selon la revendication 25 :
i) dans lequel le trouble neurologique est la sclérose en plaques ou un accident vasculaire cérébral ; ou
ii) dans lequel le trouble neurologique est la sclérose en plaques ; ou
iii) dans lequel le trouble neurologique est une déficience de la marche associée à la sclérose en plaques ; ou
iv) dans lequel le trouble neurologique est une déficience neurocognitive ou neuropsychiatrique associée à la sclérose en plaques ; ou
v) dans lequel le trouble neurologique est un accident vasculaire cérébral ; ou
vi) dans lequel le trouble neurologique est une déficience sensorimotrice associée à un accident vasculaire cérébral ; ou
vii) dans lequel le trouble neurologique est une déficience de la marche associée à un accident vasculaire cérébral ; et/ou
viii) dans lequel le patient est un patient humain.
